# EUROPEAN PATENT APPLICATION

(11) **EP 3 673 917 A1**
(43) Date of publication of application: **01.07.2020**
(21) Application number: 18248201.8
(22) Date of filing: 28.12.2018
(51) Int. Cl.: A61K 39/12, A61K 39/295, A61P 31/14

(54) **NOROVIRUS VACCINES**

(71) Applicant: Themis Bioscience GmbH, 1190 Vienna (AT)
(72) Inventor: Tauber, Erich, 3426 Muckendorf (AT); Müllner, Matthias, 3441 Pixendorf (AT); Ramsauer, Katrin, 1220 Vienna (AT); Schrauf, Sabrina, 7072 Mörbisch am See (AT); Irmler, Angelika, 1210 Vienna (AT); Csar, Patrick, 3433 Königstetten (AT)
(74) Representative: Eisenführ Speiser

(57) **Abstract**

The present invention provides immunogenic compositions, nucleic acid molecules and VLPs suitable as Norovirus vaccine candidates. Further provided are host cells for producing the biological material as well as methods for producing and/or purifying the immunogenic compositions and VLPs. Further provided is an immunogenic composition for use in methods of preventing/treating a Norovirus infection in a subject.

SEQUENCE LISTING

<110>  Themis Bioscience GmbH

<120>  Norovirus vaccines

<130>  TM 5449-01EP

<140>  not yet known
<141>  2018-12-28

<160>  18

<170>  PatentIn version 3.5

<210>  1
<211>  540
<212>  PRT
<213>  Norovirus

<400>  1

Met Lys Met Ala Ser Ser Asp Ala Asn Pro Ser Asp Gly Ser Ala Ala
1               5                   10                  15

Asn Leu Val Pro Glu Val Asn Asn Glu Val Met Ala Leu Glu Pro Val
            20                  25                  30

Val Gly Ala Ala Ile Ala Ala Pro Val Ala Gly Gln Gln Asn Val Ile
        35                  40                  45

Asp Pro Trp Ile Arg Asn Asn Phe Val Gln Ala Pro Gly Gly Glu Phe
    50                  55                  60

Thr Val Ser Pro Arg Asn Ala Pro Gly Glu Ile Leu Trp Ser Ala Pro
65                  70                  75                  80

Leu Gly Pro Asp Leu Asn Pro Tyr Leu Ser His Leu Ala Arg Met Tyr
                85                  90                  95

Asn Gly Tyr Ala Gly Gly Phe Glu Val Gln Val Ile Leu Ala Gly Asn
            100                 105                 110

Ala Phe Thr Ala Gly Lys Val Ile Phe Ala Ala Val Pro Pro Asn Phe
        115                 120                 125

Pro Thr Glu Gly Leu Ser Pro Ser Gln Val Thr Met Phe Pro His Ile
    130                 135                 140

Val Val Asp Val Arg Gln Leu Glu Pro Val Leu Ile Pro Leu Pro Asp
145                 150                 155                 160

Val Arg Asn Asn Phe Tyr His Tyr Asn Gln Ser Asn Asp Pro Thr Ile

                165                 170                 175

Lys Leu Ile Ala Met Leu Tyr Thr Pro Leu Arg Ala Asn Asn Ala Gly
            180                 185                 190

Asp Asp Val Phe Thr Val Ser Cys Arg Val Leu Thr Arg Pro Ser Pro
        195                 200                 205

Asp Phe Asp Phe Ile Phe Leu Val Pro Pro Thr Val Glu Ser Arg Thr
    210                 215                 220

Lys Pro Phe Ser Val Pro Val Leu Thr Val Glu Glu Met Thr Asn Ser
225                 230                 235                 240

Arg Phe Pro Ile Pro Leu Glu Lys Leu Phe Thr Gly Pro Ser Ser Ala
                245                 250                 255

Phe Val Val Gln Pro Gln Asn Gly Arg Cys Thr Thr Asp Gly Val Leu
            260                 265                 270

Leu Gly Thr Thr Gln Leu Ser Pro Val Asn Ile Cys Thr Phe Arg Gly
        275                 280                 285

Asp Val Thr His Ile Thr Gly Ser Arg Asn Tyr Thr Met Asn Leu Ala
    290                 295                 300

Ser Gln Asn Trp Asn Asp Tyr Asp Pro Thr Glu Glu Ile Pro Ala Pro
305                 310                 315                 320

Leu Gly Thr Pro Asp Phe Val Gly Lys Ile Gln Gly Val Leu Thr Gln
                325                 330                 335

Thr Thr Arg Thr Asp Gly Ser Thr Arg Gly His Lys Ala Thr Val Tyr
            340                 345                 350

Thr Gly Ser Ala Asp Phe Ala Pro Lys Leu Gly Arg Val Gln Phe Glu
        355                 360                 365

Thr Asp Thr Asp Arg Asp Phe Glu Ala Asn Gln Asn Thr Lys Phe Thr
    370                 375                 380

Pro Val Gly Val Ile Gln Asp Gly Gly Thr Thr His Arg Asn Glu Pro
385                 390                 395                 400

Gln Gln Trp Val Leu Pro Ser Tyr Ser Gly Arg Asn Thr His Asn Val
                405                 410                 415

His Leu Ala Pro Ala Val Ala Pro Thr Phe Pro Gly Glu Gln Leu Leu
            420                 425                 430

Phe Phe Arg Ser Thr Met Pro Gly Cys Ser Gly Tyr Pro Asn Met Asp
        435                 440                 445

Leu Asp Cys Leu Leu Pro Gln Glu Trp Val Gln Tyr Phe Tyr Gln Glu
    450                 455                 460

Ala Ala Pro Ala Gln Ser Asp Val Ala Leu Leu Arg Phe Val Asn Pro
465                 470                 475                 480

Asp Thr Gly Arg Val Leu Phe Glu Cys Lys Leu His Lys Ser Gly Tyr
                485                 490                 495

Val Thr Val Ala His Thr Gly Gln His Asp Leu Val Ile Pro Pro Asn
            500                 505                 510

Gly Tyr Phe Arg Phe Asp Ser Trp Val Asn Gln Phe Tyr Thr Leu Ala
        515                 520                 525

Pro Met Gly Asn Gly Thr Gly Arg Arg Arg Ala Val
    530                 535                 540

<210>  2
<211>  268
<212>  PRT
<213>  Norovirus

<400>  2

Met Ala Gly Ala Phe Phe Ala Gly Leu Ala Ser Asp Val Leu Gly Ser
1               5                   10                  15

Gly Leu Gly Ser Leu Ile Asn Ala Gly Ala Gly Ala Ile Asn Gln Lys
            20                  25                  30

Val Glu Phe Glu Asn Asn Arg Lys Leu Gln Gln Ala Ser Phe Gln Phe
        35                  40                  45

Ser Ser Asn Leu Gln Gln Ala Ser Phe Gln His Asp Lys Glu Met Leu
    50                  55                  60

Gln Ala Gln Ile Glu Ala Thr Lys Lys Leu Gln Gln Glu Met Met Lys
65                  70                  75                  80

Val Lys Gln Ala Met Leu Leu Glu Gly Gly Phe Phe Glu Thr Asp Ala
                85                  90                  95

Ala Arg Gly Ala Ile Asn Ala Pro Met Thr Lys Ala Leu Asp Trp Ser
            100                 105                 110

Gly Thr Arg Tyr Trp Ala Pro Asp Ala Arg Thr Thr Thr Tyr Asn Ala
        115                 120                 125

Gly Arg Phe Ser Thr Pro Gln Pro Ser Gly Ala Leu Pro Gly Arg Ala
    130                 135                 140

Asn Leu Arg Asp Ala Val Pro Ala Arg Gly Ser Ser Ser Lys Ser Ser
145                 150                 155                 160

Asn Ser Ser Thr Ala Thr Ser Val Tyr Ser Asn Gln Thr Thr Ser Thr
                165                 170                 175

Arg Leu Gly Ser Thr Ala Val Ser Gly Thr Ser Val Ser Ser Phe Pro
            180                 185                 190

Ser Thr Ala Arg Thr Arg Ser Trp Val Glu Asp Gln Ser Arg Asn Leu
        195                 200                 205

Ser Pro Ser Met Arg Gly Ala His Asn Ile Ser Phe Val Thr Pro Pro
    210                 215                 220

Ser Ser Arg Ser Ser Ser Gln Gly Thr Val Ser Thr Val Pro Lys Glu
225                 230                 235                 240

Val Leu Asp Ser Trp Thr Gly Ala Phe Asn Thr Arg Arg Gln Pro Leu
                245                 250                 255

Phe Ala His Ile Arg Lys Arg Gly Glu Ser Arg Ala
            260                 265

<210>  3
<211>  1623
<212>  DNA
<213>  Norovirus

<400>  3
atgaagatgg cgtcgagtga cgccaaccca tctgatgggt ccgcagccaa cctcgtccca      60

gaggtcaaca atgaggttat ggctctggag cccgttgttg gtgccgccat tgcggcacct     120

gtagcgggcc aacaaaatgt aattgacccc tggattagaa ataattttgt acaagcccct     180

ggtggagagt ttacagtatc ccctagaaac gctccaggtg aaatactatg gagcgcgccc     240

ttgggccctg atctaaatcc ctacctatcc catttggcca gaatgtacaa tggttatgca     300

ggtggttttg aagtgcaggt aattctcgcg gggaacgcgt tcaccgccgg gaaggtcata     360

tttgcagcag tcccaccaaa ttttccaact gaaggcttga gccccagcca ggtcactatg     420

ttcccccata tagtagtaga tgttaggcaa ctagaacctg tgttgattcc cttacccgat 480

gttaggaata atttctatca ttataatcaa tcaaatgacc ctaccattaa gttgatagca 540

atgttgtata caccacttag ggctaataat gctggggatg atgtcttcac agtttcttgc 600

cgagttctca cgagaccatc ccccgatttt gatttcatat ttttagtgcc acccacagtt 660

gagtcaagaa ctaaaccatt ctctgtccca gttttaactg ttgaggagat gaccaattca 720

agattcccca ttcctttgga aaagttgttc acgggtccca gcagtgcctt tgttgtccaa 780

ccacaaaacg gcaggtgcac gactgatggc gtgctcctag gcaccaccca actgtctcct 840

gtcaacatct gcaccttcag aggagatgtc acccacatca caggtagtcg taactacaca 900

atgaatttgg cttctcaaaa ttggaacgat tatgacccaa cagaagaaat cccagcccct 960

ctaggaactc cagattttgt ggggaagatt caaggcgttc tcacccaaac cacaaggaca 1020

gatggctcaa cacgcggcca caaagctaca gtgtacactg ggagcgccga ctttgctcca 1080

aaactgggta gagttcaatt tgaaactgac acagaccgtg attttgaagc taaccaaaac 1140

acaaagttca ccccagtcgg tgtcatccaa gatggtggca ccacccaccg aaatgaaccc 1200

caacagtggg tgctcccaag ctactcaggc agaaatactc ataatgtgca tctggccccc 1260

gctgtagccc ccacttttcc gggtgagcaa cttctcttct tcagatccac catgcccgga 1320

tgcagcgggt accccaacat ggatttggac tgtctgctcc cccaggaatg ggtgcagtac 1380

ttctaccaag aggcagcccc agcacaatct gatgtggctc tgctaagatt tgtgaatcca 1440

gacacaggta gggttttgtt tgagtgtaag cttcataaat caggctatgt tacagtggct 1500

cacactggcc aacatgattt ggttatcccc cccaatggtt attttaggtt tgattcctgg 1560

gtcaaccagt tttacacgct tgcccccatg ggaaatggaa cggggcgtag acgtgcagta 1620

taa 1623

<210> 4
<211> 804
<212> DNA
<213> Norovirus

<400> 4
atggctggag ctttctttgc tggattggca tctgatgtcc ttggctctgg gcttggttcc 60

cttatcaatg ctggggctgg ggccatcaac caaaaagttg agtttgaaaa taacagaaaa 120

ttgcaacaag catccttcca atttagcagc aatctacaac aggcttcctt tcaacatgac 180

aaagagatgc tccaagcaca aattgaggcc accaaaaagc tacaacagga aatgatgaaa 240

gttaagcagg caatgctcct agagggtggg ttctttgaga cagatgcagc ccgcggggca 300

atcaacgccc ccatgacaaa agctttggac tggagcggga caaggtactg ggctcccgat 360

gctaggacta caacatacaa tgcaggccgc ttttccaccc ctcaaccatc gggggcactg 420

ccaggaagag ctaatcttag ggatgctgtc cctgctcggg gttcctccag caaatcttct 480

aactcttcta ctgctacttc tgtgtactca aatcaaacta cttcaacgag acttggttct 540

acagctgttt ctggtaccag tgtctcgagc ttcccgtcaa ctgcaaggac taggagctgg 600

gttgaggatc aaagtaggaa tttgtcacct tccatgaggg gggcccacaa catatcgttt 660

gtcaccccac catctagcag atcctctagc caaggcacag tctcaaccgt gcctaaagag 720

gttttggact cctggactgg cgctttcaac acgcgcaggc agccactctt cgctcacatt 780

cgtaagcgag gggagtcacg ggcg 804

<210> 5
<211> 1623
<212> DNA
<213> Artificial Sequence

<220>
<223> Codon optimized VP1

<400> 5
atgaagatgg cttcctctga cgccaatcct tccgatggaa gcgctgctaa tctggtgccc 60

gaggtcaaca acgaggtaat ggcactggaa cccgtcgtgg gtgcagcgat tgccgcccct 120

gttgctggac agcagaatgt gatcgatccc tggatacgca ataatttcgt gcaggcgcct 180

ggaggcgaat tcacggtgtc tcctagaaat gccccaggcg aaattctgtg gtcagctcct 240

ctggggcccg acttgaaccc atacctcagt catctcgcac gaatgtacaa cgggtatgcc 300

ggaggctttg aagtgcaggt gatccttgcc ggaaacgcat tcaccgccgg gaaagtcatc 360

ttcgccgctg tcccacccaa tttccctacc gaagggcttt caccaagtca ggtaaccatg 420

tttccacaca tcgttgtgga tgtccgccag cttgaacctg tactcatccc tctgccagat 480

gttaggaaca acttctacca ctacaaccag agcaacgatc caaccattaa gctgatcgcc 540

atgttgtata caccactcag agccaataac gcaggcgatg atgtgttcac tgtctcttgt 600

agagtcctga cacgaccctc tcccgatttc gacttcattt tcctcgtgcc acccaccgtt 660

gagagccgga caaaaccgtt tagcgtaccc gtgctgactg tcgaggagat gaccaattct 720

aggttcccca ttcctctgga gaagctgttt actggaccgt ccagcgcctt tgttgtgcag 780

ccacagaacg gccgctgtac tactgatggc gtgctgcttg gtacaaccca actgagccca 840

gtgaatatct gcaccttcag aggggacgtg actcatatca ccggatcccg caactacaca 900

atgaacctgg cttcacagaa ctggaatgac tacgatccaa cggaggaaat accggctcct 960

ctgggtactc cggacttcgt cggcaagatt cagggagtgc tgacccaaac tacccgtact 1020

gatgggagca cccgtggcca caaagctacc gtgtatacag gcagtgctga ctttgcgcct 1080

aaactgggta gggtgcaatt tgagactgac accgatcggg actttgaggc caatcagaat 1140

accaagttta cacccgtcgg cgtcatacag gacggcggga cgacacatcg caacgaaccc 1200

cagcaatggg tattgccctc ctattccggt agaaacacgc acaacgttca cttggcacca 1260

gccgtggccc ctacctttcc tggagagcaa ctgctgttct tccgatctac aatgccgggg 1320

tgtagtggct atccaaacat ggacctcgac tgcctcctgc cccaagagtg ggttcagtac 1380

ttttatcagg aagccgcacc cgcccagagc gacgttgcac tgttgcggtt tgtaaatccc 1440

gatacgggtc gggtgctctt cgagtgcaag ctgcataaat caggctatgt cacagtggca 1500

cacacagggc aacatgacct tgtgattccg cctaacggtt acttccggtt tgacagctgg 1560

gtgaatcagt tttacactct ggctcctatg ggaaatggga caggcaggag aagggcggtt 1620

tga 1623

<210> 6
<211> 804
<212> DNA
<213> Artificial Sequence

<220>
<223> Codon optimized VP2

<400> 6
atggcaggag ccttctttgc aggattggca agcgatgttc tgggctccgg acttggttcc 60

ctgatcaacg caggagctgg cgccatcaat cagaaggtcg aattcgagaa caatcggaag 120

ctgcagcaag cttcctttca gttctctagc aatctgcagc aagcctcatt tcagcatgac 180

aaagagatgc tgcaggctca gatagaggcc acgaagaaac tgcagcaaga gatgatgaaa 240

gtgaagcaag cgatgctcct tgaaggtggc ttctttgaga ctgacgctgc gagaggcgcc 300

attaatgccc cgatgacgaa agccctggac tggtcaggga ccaggtattg ggctcctgat 360

gcccgcacca ctacatacaa cgccggtcgc ttttccactc ctcaaccatc aggggcactc 420

ccaggacggg cgaatctgag ggatgctgtg cccgcaagag gcagcagctc caagagcagt 480

aacagctcaa ccgctacaag cgtgtacagc aaccagacga cctcaacccg gttgggctcc 540

acagccgtat ctggtacctc tgtgagctct ttcccctcta ctgcccgtac tcgaagttgg 600

gtggaagatc agtcccggaa tctctctccc agtatgcgtg gggctcacaa catcagcttc 660

gttaccccac cctctagtcg ctcaagtagc cagggcacag tgtccacagt ccctaaagaa 720

gtcctggact cctggacagg ggcctttaac accagaaggc agcctctctt cgcccacatt 780

aggaagagag gggagagtcg agca 804

<210> 7
<211> 2526
<212> DNA
<213> Artificial Sequence

<220>

<223>  VP1-linker-VP2 construct

<400>  7
cgtacggcca ccatgaagat ggcgtcgagt gacgccaacc catctgatgg gtccgcagcc      60

aacctcgtcc cagaggtcaa caatgaggtt atggctctgg agcccgttgt tggtgccgcc     120

attgcggcac ctgtagcggg ccaacaaaat gtaattgacc cctggattag aaataatttt     180

gtacaagccc ctggtggaga gtttacagta tcccctagaa acgctccagg tgaaatacta     240

tggagcgcgc ccttgggccc tgatctaaat ccctacctat cccatttggc cagaatgtac     300

aatggttatg caggtggttt tgaagtgcag gtaattctcg cggggaacgc gttcaccgcc     360

gggaaggtca tatttgcagc agtcccacca aattttccaa ctgaaggctt gagccccagc     420

caggtcacta tgttccccca tatagtagta gatgttaggc aactagaacc tgtgttgatt     480

cccttacccg atgttaggaa taatttctat cattataatc aatcaaatga ccctaccatt     540

aagttgatag caatgttgta tacaccactt agggctaata atgctgggga tgatgtcttc     600

acagtttctt gccgagttct cacgagacca tcccccgatt ttgatttcat atttttagtg     660

ccacccacag ttgagtcaag aactaaacca ttctctgtcc cagttttaac tgttgaggag     720

atgaccaatt caagattccc cattcctttg gaaaagttgt tcacgggtcc cagcagtgcc     780

tttgttgtcc aaccacaaaa cggcaggtgc acgactgatg gcgtgctcct aggcaccacc     840

caactgtctc ctgtcaacat ctgcaccttc agaggagatg tcacccacat cacaggtagt     900

cgtaactaca caatgaattt ggcttctcaa aattggaacg attatgaccc aacagaagaa     960

atcccagccc ctctaggaac tccagatttt gtggggaaga ttcaaggcgt tctcacccaa    1020

accacaagga cagatggctc aacacgcggc cacaaagcta cagtgtacac tgggagcgcc    1080

gactttgctc caaaactggg tagagttcaa tttgaaactg acacagaccg tgattttgaa    1140

gctaaccaaa acacaaagtt caccccagtc ggtgtcatcc aagatggtgg caccacccac    1200

cgaaatgaac cccaacagtg ggtgctccca agctactcag gcagaaatac tcataatgtg    1260

catctggccc ccgctgtagc ccccactttt ccgggtgagc aacttctctt cttcagatcc    1320

accatgcccg gatgcagcgg gtaccccaac atggatttgg actgtctgct cccccaggaa    1380

tgggtgcagt acttctacca agaggcagcc ccagcacaat ctgatgtggc tctgctaaga    1440

tttgtgaatc cagacacagg tagggttttg tttgagtgta agcttcataa atcaggctat    1500

gttacagtgg ctcacactgg ccaacatgat ttggttatcc cccccaatgg ttattttagg    1560

tttgattcct gggtcaacca gttttacacg cttgccccca tgggaaatgg aacggggcgt    1620

agacgtgcag tataatagcc taccctccat cattgttata aaaaacttag gaaccaggtc    1680

cacacagccg ccagcccatc aacggccacc atggctggag ctttctttgc tggattggca    1740

tctgatgtcc ttggctctgg gcttggttcc cttatcaatg ctggggctgg ggccatcaac    1800

caaaaagttg agtttgaaaa taacagaaaa ttgcaacaag catccttcca atttagcagc 1860

aatctacaac aggcttcctt tcaacatgac aaagagatgc tccaagcaca aattgaggcc 1920

accaaaaagc tacaacagga aatgatgaaa gttaagcagg caatgctcct agagggtggg 1980

ttctttgaga cagatgcagc ccgcggggca atcaacgccc ccatgacaaa agctttggac 2040

tggagcggga caaggtactg ggctcccgat gctaggacta caacatacaa tgcaggccgc 2100

ttttccaccc ctcaaccatc gggggcactg ccaggaagag ctaatcttag ggatgctgtc 2160

cctgctcggg gttcctccag caaatcttct aactcttcta ctgctacttc tgtgtactca 2220

aatcaaacta cttcaacgag acttggttct acagctgttt ctggtaccag tgtctcgagc 2280

ttcccgtcaa ctgcaaggac taggagctgg gttgaggatc aaagtaggaa tttgtcacct 2340

tccatgaggg gggcccacaa catatcgttt gtcaccccac catctagcag atcctctagc 2400

caaggcacag tctcaaccgt gcctaaagag gttttggact cctggactgg cgctttcaac 2460

acgcgcaggc agccactctt cgctcacatt cgtaagcgag gggagtcacg ggcgtaatga 2520

gcgcgc 2526

<210> 8
<211> 2526
<212> DNA
<213> Artificial Sequence

<220>
<223> VP1-linker-VP2 construct codon optimized

<400> 8
   cgtacggcca ccatgaagat ggcttcctct gacgccaatc cttccgatgg aagcgctgct 60
   aatctggtgc ccgaggtcaa caacgaggta atggcactgg aacccgtcgt gggtgcagcg 120
   attgccgccc ctgttgctgg acagcagaat gtgatcgatc cctggatacg caataatttc 180
   gtgcaggcgc ctggaggcga attcacggtg tctcctagaa atgccccagg cgaaattctg 240
   tggtcagctc ctctggggcc cgacttgaac ccatacctca gtcatctcgc acgaatgtac 300
   aacgggtatg ccggaggctt tgaagtgcag gtgatccttg ccggaaacgc attcaccgcc 360
   gggaaagtca tcttcgccgc tgtcccaccc aatttcccta ccgaagggct ttcaccaagt 420
   caggtaacca tgtttccaca catcgttgtg gatgtccgcc agcttgaacc tgtactcatc 480
   cctctgccag atgttaggaa caacttctac cactacaacc agagcaacga tccaaccatt 540
   aagctgatcg ccatgttgta tacaccactc agagccaata acgcaggcga tgatgtgttc 600
   actgtctctt gtagagtcct gacacgaccc tctcccgatt tcgacttcat tttcctcgtg 660
   ccacccaccg ttgagagccg gacaaaaccg tttagcgtac ccgtgctgac tgtcgaggag 720
   atgaccaatt ctaggttccc cattcctctg gagaagctgt ttactggacc gtccagcgcc 780
   tttgttgtgc agccacagaa cggccgctgt actactgatg gcgtgctgct tggtacaacc 840
   caactgagcc cagtgaatat ctgcaccttc agaggggacg tgactcatat caccggatcc 900
   cgcaactaca caatgaacct ggcttcacag aactggaatg actacgatcc aacggaggaa 960
   ataccggctc ctctgggtac tccggacttc gtcggcaaga ttcagggagt gctgacccaa 1020
   actacccgta ctgatgggag cacccgtggc cacaaagcta ccgtgtatac aggcagtgct 1080
   gactttgcgc ctaaactggg tagggtgcaa tttgagactg acaccgatcg ggactttgag 1140
   gccaatcaga ataccaagtt tacacccgtc ggcgtcatac aggacggcgg gacgacacat 1200
   cgcaacgaac cccagcaatg ggtattgccc tcctattccg gtagaaacac gcacaacgtt 1260
   cacttggcac cagccgtggc ccctaccttt cctggagagc aactgctgtt cttccgatct 1320
   acaatgccgg ggtgtagtgg ctatccaaac atggacctcg actgcctcct gccccaagag 1380
   tgggttcagt acttttatca ggaagccgca cccgcccaga gcgacgttgc actgttgcgg 1440
   tttgtaaatc ccgatacggg tcgggtgctc ttcgagtgca agctgcataa atcaggctat 1500
   gtcacagtgg cacacacagg gcaacatgac cttgtgattc cgcctaacgg ttacttccgg 1560
   tttgacagct gggtgaatca gttttacact ctggctccta tgggaaatgg gacaggcagg 1620
   agaagggcgg tttgatagcc taccctccat cattgttata aaaaacttag gaaccaggtc 1680
   cacacagccg ccagcccatc aacggccacc atggcaggag ccttctttgc aggattggca 1740
   agcgatgttc tgggctccgg acttggttcc ctgatcaacg caggagctgg cgccatcaat 1800
   cagaaggtcg aattcgagaa caatcggaag ctgcagcaag cttcctttca gttctctagc 1860
   aatctgcagc aagcctcatt tcagcatgac aaagagatgc tgcaggctca gatagaggcc 1920
   acgaagaaac tgcagcaaga gatgatgaaa gtgaagcaag cgatgctcct tgaaggtggc 1980
   ttctttgaga ctgacgctgc gagaggcgcc attaatgccc cgatgacgaa agccctggac 2040
   tggtcaggga ccaggtattg ggctcctgat gcccgcacca ctacatacaa cgccggtcgc 2100
   ttttccactc ctcaaccatc aggggcactc ccaggacggg cgaatctgag ggatgctgtg 2160
   cccgcaagag gcagcagctc caagagcagt aacagctcaa ccgctacaag cgtgtacagc 2220
   aaccagacga cctcaacccg gttgggctcc acagccgtat ctggtacctc tgtgagctct 2280
   ttcccctcta ctgcccgtac tcgaagttgg gtggaagatc agtcccggaa tctctctccc 2340
   agtatgcgtg gggctcacaa catcagcttc gttaccccac cctctagtcg ctcaagtagc 2400
   cagggcacag tgtccacagt ccctaaagaa gtcctggact cctggacagg ggcctttaac 2460
   accagaaggc agcctctctt cgcccacatt aggaagagag gggagagtcg agcataatga 2520
   gcgcgc 2526

<210> 9
<211> 17634
<212> DNA

<213>  Artificial Sequence

<220>
<223>  MV Noro VP1 optimized cDNA plus strand

<400>  9
accaaacaaa gttgggtaag gatagttcaa tcaatgatca tcttctagtg cacttaggat      60

tcaagatcct attatcaggg acaagagcag gattagggat atccgagatg gccacacttt     120

taaggagctt agcattgttc aaaagaaaca aggacaaacc acccattaca tcaggatccg     180

gtggagccat cagaggaatc aaacacatta ttatagtacc aatccctgga gattcctcaa     240

ttaccactcg atccagactt ctggaccggt tggtgaggtt aattggaaac ccggatgtga     300

gcgggcccaa actaacaggg gcactaatag gtatattatc cttatttgtg gagtctccag     360

gtcaattgat tcagaggatc accgatgacc ctgacgttag cataaggctg ttagaggttg     420

tccagagtga ccagtcacaa tctggcctta ccttcgcatc aagaggtacc aacatggagg     480

atgaggcgga ccaatacttt tcacatgatg atccaattag tagtgatcaa tccaggttcg     540

gatggttcgg gaacaaggaa atctcagata ttgaagtgca agaccctgag ggattcaaca     600

tgattctggg taccatccta gcccaaattt gggtcttgct cgcaaaggcg gttacggccc     660

cagacacggc agctgattcg gagctaagaa ggtggataaa gtacacccaa caaagaaggg     720

tagttggtga atttagattg gagagaaaat ggttggatgt ggtgaggaac aggattgccg     780

aggacctctc cttacgccga ttcatggtcg ctctaatcct ggatatcaag agaacacccg     840

gaaacaaacc caggattgct gaaatgatat gtgacattga tacatatatc gtagaggcag     900

gattagccag ttttatcctg actattaagt ttgggataga aactatgtat cctgctcttg     960

gactgcatga atttgctggt gagttatcca cacttgagtc cttgatgaac ctttaccagc    1020

aaatggggga aactgcaccc tacatggtaa tcctggagaa ctcaattcag aacaagttca    1080

gtgcaggatc ataccctctg ctctggagct atgccatggg agtaggagtg gaacttgaaa    1140

actccatggg aggtttgaac tttggccgat cttactttga tccagcatat tttagattag    1200

ggcaagagat ggtaaggagg tcagctggaa aggtcagttc cacattggca tctgaactcg    1260

gtatcactgc cgaggatgca aggcttgttt cagagattgc aatgcatact actgaggaca    1320

agatcagtag agcggttgga cccagacaag cccaagtatc atttctacac ggtgatcaaa    1380

gtgagaatga gctaccgaga ttggggggca aggaagatag gagggtcaaa cagagtcgag    1440

gagaagccag ggagagctac agagaaaccg ggcccagcag agcaagtgat gcgagagctg    1500

cccatcttcc aaccggcaca cccctagaca ttgacactgc aacggagtcc agccaagatc    1560

cgcaggacag tcgaaggtca gctgacgccc tgcttaggct gcaagccatg gcaggaatct    1620

cggaagaaca aggctcagac acggacaccc ctatagtgta caatgacaga aatcttctag    1680

actaggtgcg agaggccgag ggccagaaca acatccgcct accatccatc attgttataa    1740

aaaacttagg aaccaggtcc acacagccgc cagcccatca accatccact cccacgattg 1800

gagccaatgg cagaagagca ggcacgccat gtcaaaaacg gactggaatg catccgggct 1860

ctcaaggccg agcccatcgg ctcactggcc atcgaggaag ctatggcagc atggtcagaa 1920

atatcagaca acccaggaca ggagcgagcc acctgcaggg aagagaaggc aggcagttcg 1980

ggtctcagca aaccatgcct ctcagcaatt ggatcaactg aaggcggtgc acctcgcatc 2040

cgcggtcagg gacctggaga gagcgatgac gacgctgaaa ctttgggaat ccccccaaga 2100

aatctccagg catcaagcac tgggttacag tgttattacg tttatgatca cagcggtgaa 2160

gcggttaagg gaatccaaga tgctgactct atcatggttc aatcaggcct tgatggtgat 2220

agcaccctct caggaggaga caatgaatct gaaaacagcg atgtggatat tggcgaacct 2280

gataccgagg gatatgctat cactgaccgg ggatctgctc ccatctctat ggggttcagg 2340

gcttctgatg ttgaaactgc agaaggaggg gagatccacg agctcctgag actccaatcc 2400

agaggcaaca actttccgaa gcttgggaaa actctcaatg ttcctccgcc cccggacccc 2460

ggtagggcca gcacttccgg gacacccatt aaaaagggca cagacgcgag attagcctca 2520

tttggaacgg agatcgcgtc tttattgaca ggtggtgcaa cccaatgtgc tcgaaagtca 2580

ccctcggaac catcagggcc aggtgcacct gcggggaatg tccccgagtg tgtgagcaat 2640

gccgcactga tacaggagtg gacacccgaa tctggtacca caatctcccc gagatcccag 2700

aataatgaag aagggggaga ctattatgat gatgagctgt tctctgatgt ccaagatatt 2760

aaaacagcct tggccaaaat acacgaggat aatcagaaga taatctccaa gctagaatca 2820

ctgctgttat tgaagggaga agttgagtca attaagaagc agatcaacag gcaaaatatc 2880

agcatatcca ccctggaagg acacctctca agcatcatga tcgccattcc tggacttggg 2940

aaggatccca acgaccccac tgcagatgtc gaaatcaatc ccgacttgaa acccatcata 3000

ggcagagatt caggccgagc actggccgaa gttctcaaga aacccgttgc cagccgacaa 3060

ctccaaggaa tgacaaatgg acggaccagt tccagaggac agctgctgaa ggaatttcag 3120

ctaaagccga tcgggaaaaa gatgagctca gccgtcgggt ttgttcctga caccggccct 3180

gcatcacgca gtgtaatccg ctccattata aaatccagcc ggctagagga ggatcggaag 3240

cgttacctga tgactctcct tgatgatatc aaaggagcca atgatcttgc caagttccac 3300

cagatgctga tgaagataat aatgaagtag ctacagctca acttacctgc caaccccatg 3360

ccagtcgacc caactagcct accctccatc attgttataa aaaacttagg aaccaggtcc 3420

acacagccgc cagcccatca acgcgtacgg ccaccatgaa gatggcctct tctgacgcca 3480

acccttcaga cggctcagct gccaatttgg tgcctgaggt gaacaacgag gtgatggccc 3540

tggaacccgt ggtcggagcc gctatagccg caccagttgc cggccagcag aatgtcatcg 3600

acccgtggat tcggaataac ttcgtgcaag cgccgggcgg tgagttcacc gtcagcccac 3660

gaaatgcccc aggcgagatc ctgtggagtg ctccattggg gcctgatctt aacccatacc 3720

tgtcacactt ggcgagaatg tacaatgggt atgcaggagg atttgaggtc caggttattc 3780

tggctgggaa tgctttcaca gcagggaagg tgattttcgc cgccgtccct cctaactttc 3840

ccacagaggg tctgagccca agtcaggtca ccatgttccc acacattgtg gtagatgtga 3900

gacagctcga gcctgtcctg attccacttc cagacgttag gaacaacttt taccactaca 3960

atcagagcaa cgatcccacc atcaagctca ttgccatgct gtatacaccc ctgagagcca 4020

acaatgctgg ggatgacgtc tttactgtga gctgtcgcgt tctgacgcgg ccctcccctg 4080

actttgactt catcttcctc gttcctccaa cggtggaatc caggacgaag ccattctcag 4140

tccctgtcct cactgtggag gaaatgacca atagcaggtt tcctatccct ctggagaaac 4200

tgttcactgg cccgagctcc gcatttgtgg tacagcctca gaacgggaga tgtaccacag 4260

atggcgtgct tctcggcaca acccaactct ctccagtgaa tatctgcacg tttcgtggcg 4320

acgtaaccca catcactgga agcaggaatt acaccatgaa tctcgccagt caaaactgga 4380

acgactacga ccccacagaa gaaatacccg cacccctggg gacaccagat tttgtgggta 4440

aaatccaggg agtactgact caaaccaccc gaactgacgg ttctactcgg ggccataaag 4500

ccactgtgta taccgggagc gcggatttcg ctcccaaatt gggacgcgta cagtttgaga 4560

cagatactga tcgggacttc gaagcaaacc agaacaccaa gttcactccc gttggcgtta 4620

ttcaggatgg aggcaccacc catcgaaatg agccgcagca atgggtactg ccctcctaca 4680

gtggacggaa tacgcataac gtgcatctgg ctcctgcagt tgctcctact ttccccggcg 4740

agcagctgct gttcttccgc tctaccatgc ccgggtgtag cgggtatccc aacatggacc 4800

ttgactgctt gcttccccaa gaatgggtgc agtacttcta tcaggaagcc gctcctgcgc 4860

agtctgatgt ggcactcctg cgctttgtga acccggatac aggaagagtg ctgtttgaat 4920

gcaagctgca caaatccgga tacgtgacag tcgctcacac aggtcaacat gatctggtta 4980

taccaccgaa tggctatttc cgctttgact cctgggtgaa tcagttttat accctggcac 5040

ccatgggtaa cggtacaggc cgtaggagag ccgtctaatg agcgcgcagc gcttagacgt 5100

ctcgcgatcg atactagtac aacctaaatc cattataaaa aacttaggag caaagtgatt 5160

gcctcccaag gtccacaatg acagagacct acgacttcga caagtcggca tgggacatca 5220

aagggtcgat cgctccgata caacccacca cctacagtga tggcaggctg gtgccccagg 5280

tcagagtcat agatcctggt ctaggcgaca ggaaggatga atgctttatg tacatgtttc 5340

tgctgggggt tgttgaggac agcgattccc tagggcctcc aatcgggcga gcatttgggt 5400

tcctgccctt aggtgttggc agatccacag caaagcccga aaaactcctc aaagaggcca 5460

ctgagcttga catagttgtt agacgtacag cagggctcaa tgaaaaactg gtgttctaca 5520

acaacacccc actaactctc ctcacacctt ggagaaaggt cctaacaaca gggagtgtct 5580

tcaacgcaaa ccaagtgtgc aatgcggtta atctgatacc gctcgatacc ccgcagaggt 5640

tccgtgttgt ttatatgagc atcacccgtc tttcggataa cgggtattac accgttccta 5700

gaagaatgct ggaattcaga tcggtcaatg cagtggcctt caacctgctg gtgaccctta 5760

ggattgacaa ggcgataggc cctgggaaga tcatcgacaa tacagagcaa cttcctgagg 5820

caacatttat ggtccacatc gggaacttca ggagaaagaa gagtgaagtc tactctgccg 5880

attattgcaa aatgaaaatc gaaaagatgg gcctggtttt tgcacttggt gggatagggg 5940

gcaccagtct tcacattaga agcacaggca aaatgagcaa gactctccat gcacaactcg 6000

ggttcaagaa gaccttatgt tacccgctga tggatatcaa tgaagacctt aatcgattac 6060

tctggaggag cagatgcaag atagtaagaa tccaggcagt tttgcagcca tcagttcctc 6120

aagaattccg catttacgac gacgtgatca taaatgatga ccaaggacta ttcaaagttc 6180

tgtagaccgt agtgcccagc aatgcccgaa aacgaccccc ctcacaatga cagccagaag 6240

gcccggacaa aaaagccccc tccgaaagac tccacggacc aagcgagagg ccagccagca 6300

gccgacggca agcgcgaaca ccaggcggcc ccagcacaga acagccctga cacaaggcca 6360

ccaccagcca ccccaatctg catcctcctc gtgggacccc cgaggaccaa cccccaaggc 6420

tgcccccgat ccaaaccacc aaccgcatcc ccaccacccc cgggaaagaa acccccagca 6480

attggaaggc ccctccccct cttcctcaac acaagaactc cacaaccgaa ccgcacaagc 6540

gaccgaggtg acccaaccgc aggcatccga ctccctagac agatcctctc tccccggcaa 6600

actaaacaaa acttagggcc aaggaacata cacacccaac agaacccaga ccccggccca 6660

cggcgccgcg cccccaaccc ccgacaacca gagggagccc ccaaccaatc ccgccggctc 6720

ccccggtgcc cacaggcagg gacaccaacc cccgaacaga cccagcaccc aaccatcgac 6780

aatccaagac gggggggccc ccccaaaaaa aggcccccag ggccgacagc cagcaccgc 6840

gaggaagccc acccacccca cacacgacca cggcaaccaa accagaaccc agaccaccct 6900

gggccaccag ctcccagact cggccatcac cccgcagaaa ggaaaggcca caacccgcgc 6960

accccagccc cgatccggcg gggagccacc caacccgaac cagcacccaa gagcgatccc 7020

cgaaggaccc ccgaaccgca aaggacatca gtatcccaca gcctctccaa gtcccccggt 7080

ctcctcctct tctcgaaggg accaaaagat caatccacca cacccgacga cactcaactc 7140

cccaccccta aaggagacac cgggaatccc agaatcaaga ctcatccaat gtccatcatg 7200

ggtctcaagg tgaacgtctc tgccatattc atggcagtac tgttaactct ccaaacaccc 7260

accggtcaaa tccattgggg caatctctct aagatagggg tggtaggaat aggaagtgca 7320

agctacaaag ttatgactcg ttccagccat caatcattag tcataaaatt aatgcccaat 7380

ataactctcc tcaataactg cacgagggta gagattgcag aatacaggag actactgaga 7440

acagttttgg aaccaattag agatgcactt aatgcaatga cccagaatat aagaccggtt 7500

cagagtgtag cttcaagtag gagacacaag agatttgcgg gagtagtcct ggcaggtgcg 7560

gccctaggcg ttgccacagc tgctcagata acagccggca ttgcacttca ccagtccatg 7620

ctgaactctc aagccatcga caatctgaga gcgagcctgg aaactactaa tcaggcaatt 7680

gagacaatca gacaagcagg gcaggagatg atattggctg ttcagggtgt ccaagactac 7740

atcaataatg agctgatacc gtctatgaac caactatctt gtgatttaat cggccagaag 7800

ctcgggctca aattgctcag atactataca gaaatcctgt cattatttgg ccccagttta 7860

cgggacccca tatctgcgga gatatctatc caggctttga gctatgcgct tggaggagac 7920

atcaataagg tgttagaaaa gctcggatac agtggaggtg atttactggg catcttagag 7980

agcggaggaa taaaggcccg gataactcac gtcgacacag agtcctactt cattgtcctc 8040

agtatagcct atccgacgct gtccgagatt aagggggtga ttgtccaccg gctagagggg 8100

gtctcgtaca acataggctc tcaagagtgg tataccactg tgcccaagta tgttgcaacc 8160

caagggtacc ttatctcgaa ttttgatgag tcatcgtgta ctttcatgcc agaggggact 8220

gtgtgcagcc aaaatgcctt gtacccgatg agtcctctgc tccaagaatg cctccggggg 8280

tacaccaagt cctgtgctcg tacactcgta tccgggtctt ttgggaaccg gttcatttta 8340

tcacaaggga acctaatagc caattgtgca tcaatccttt gcaagtgtta cacaacagga 8400

acgatcatta atcaagaccc tgacaagatc ctaacataca ttgctgccga tcactgcccg 8460

gtagtcgagg tgaacggcgt gaccatccaa gtcgggagca ggaggtatcc agacgctgtg 8520

tacttgcaca gaattgacct cggtcctccc atatcattgg agaggttgga cgtagggaca 8580

aatctgggga atgcaattgc taagttggag gatgccaagg aattgttgga gtcatcggac 8640

cagatattga ggagtatgaa aggtttatcg agcactagca tagtctacat cctgattgca 8700

gtgtgtcttg gagggttgat agggatcccc gctttaatat gttgctgcag ggggcgttgt 8760

aacaaaaagg gagaacaagt tggtatgtca agaccaggcc taaagcctga tcttacggga 8820

acatcaaaat cctatgtaag gtcgctctga tcctctacaa ctcttgaaac acaaatgtcc 8880

cacaagtctc ctcttcgtca tcaagcaacc accgcaccca gcatcaagcc cacctgaaat 8940

tatctccggc ttccctctgg ccgaacaata tcggtagtta atcaaaactt agggtgcaag 9000

atcatccaca atgtcaccac aacgagaccg gataaatgcc ttctacaaag ataaccccca 9060

tcccaaggga agtaggatag tcattaacag agaacatctt atgattgata gaccttatgt 9120

tttgctggct gttctgtttg tcatgtttct gagcttgatc gggttgctag ccattgcagg 9180

cattagactt catcgggcag ccatctacac cgcagagatc cataaaagcc tcagcaccaa 9240

tctagatgta actaactcaa tcgagcatca ggtcaaggac gtgctgacac cactcttcaa 9300

aatcatcggt gatgaagtgg gcctgaggac acctcagaga ttcactgacc tagtgaaatt 9360

aatctctgac aagattaaat tccttaatcc ggatagggag tacgacttca gagatctcac 9420

ttggtgtatc aacccgccag agagaatcaa attggattat gatcaatact gtgcagatgt 9480

ggctgctgaa gagctcatga atgcattggt gaactcaact ctactggaga ccagaacaac 9540

caatcagttc ctagctgtct caaagggaaa ctgctcaggg cccactacaa tcagaggtca 9600

attctcaaac atgtcgctgt ccctgttaga cttgtattta ggtcgaggtt acaatgtgtc 9660

atctatagtc actatgacat cccagggaat gtatggggga acttacctag tggaaaagcc 9720

taatctgagc agcaaaaggt cagagttgtc acaactgagc atgtaccgag tgtttgaagt 9780

aggtgttatc agaaatccgg gtttgggggc tccggtgttc catatgacaa actatcttga 9840

gcaaccagtc agtaatgatc tcagcaactg tatggtggct ttgggggagc tcaaactcgc 9900

agccctttgt cacggggaag attctatcac aattccctat cagggatcag ggaaaggtgt 9960

cagcttccag ctcgtcaagc taggtgtctg gaaatcccca accgacatgc aatcctgggt 10020

ccccttatca acggatgatc cagtgataga caggctttac ctctcatctc acagaggtgt 10080

tatcgctgac aatcaagcaa aatgggctgt cccgacaaca cgaacagatg acaagttgcg 10140

aatggagaca tgcttccaac aggcgtgtaa gggtaaaatc caagcactct gcgagaatcc 10200

cgagtgggca ccattgaagg ataacaggat tccttcatac ggggtcttgt ctgttgatct 10260

gagtctgaca gttgagctta aaatcaaaat tgcttcggga ttcgggccat tgatcacaca 10320

cggttcaggg atggacctat acaaatccaa ccacaacaat gtgtattggc tgactatccc 10380

gccaatgaag aacctagcct taggtgtaat caacacattg gagtggatac cgagattcaa 10440

ggttagtccc tacctcttca ctgtcccaat taaggaagca ggcgaagact gccatgcccc 10500

aacataccta cctgcggagg tggatggtga tgtcaaactc agttccaatc tggtgattct 10560

acctggtcaa gatctccaat atgttttggc aacctacgat acttccaggg ttgaacatgc 10620

tgtggtttat tacgtttaca gcccaagccg ctcattttct tacttttatc cttttaggtt 10680

gcctataaag ggggtcccca tcgaattaca agtggaatgc ttcacatggg accaaaaact 10740

ctggtgccgt cacttctgtg tgcttgcgga ctcagaatct ggtggacata tcactcactc 10800

tgggatggtg ggcatgggag tcagctgcac agtcacccgg gaagatggaa ccaatcgcag 10860

atagggctgc tagtgaacca atcacatgat gtcacccaga catcaggcat acccactagt 10920

gtgaaataga catcagaatt aagaaaaacg tagggtccaa gtggttcccc gttatggact 10980

cgctatctgt caaccagatc ttataccctg aagttcacct agatagcccg atagttacca 11040

ataagatagt agccatcctg gagtatgctc gagtccctca cgcttacagc ctggaggacc 11100

ctacactgtg tcagaacatc aagcaccgcc taaaaaacgg attttccaac caaatgatta 11160

taaacaatgt ggaagttggg aatgtcatca agtccaagct taggagttat ccggcccact 11220
ctcatattcc atatccaaat tgtaatcagg atttatttaa catagaagac aaagagtcaa 11280
cgaggaagat ccgtgaactc ctcaaaaagg ggaattcgct gtactccaaa gtcagtgata 11340
aggttttcca atgcttaagg gacactaact cacggcttgg cctaggctcc gaattgaggg 11400
aggacatcaa ggagaaagtt attaacttgg gagtttacat gcacagctcc cagtggtttg 11460
agccctttct gttttggttt acagtcaaga ctgagatgag gtcagtgatt aaatcacaaa 11520
cccatacttg ccataggagg agacacacac ctgtattctt cactggtagt tcagttgagt 11580
tgctaatctc tcgtgacctt gttgctataa tcagtaaaga gtctcaacat gtatattacc 11640
tgacatttga actggttttg atgtattgtg atgtcataga ggggaggtta atgacagaga 11700
ccgctatgac tattgatgct aggtatacag agcttctagg aagagtcaga tacatgtgga 11760
aactgataga tggtttcttc cctgcactcg ggaatccaac ttatcaaatt gtagccatgc 11820
tggagcctct ttcacttgct tacctgcagc tgagggatat aacagtagaa ctcagaggtg 11880
ctttccttaa ccactgcttt actgaaatac atgatgttct tgaccaaaac gggttttctg 11940
atgaaggtac ttatcatgag ttaactgaag ctctagatta cattttcata actgatgaca 12000
tacatctgac aggggagatt ttctcatttt tcagaagttt cggccacccc agacttgaag 12060
cagtaacggc tgctgaaaat gttaggaaat acatgaatca gcctaaagtc attgtgtatg 12120
agactctgat gaaaggtcat gccatatttt gtggaatcat aatcaacggc tatcgtgaca 12180
ggcacggagg cagttggcca ccgctgaccc tccccctgca tgctgcagac acaatccgga 12240
atgctcaagc ttcaggtgaa gggttaacac atgagcagtg cgttgataac tggaaatctt 12300
ttgctggagt gaaatttggc tgctttatgc ctcttagcct ggatagtgat ctgacaatgt 12360
acctaaagga caaggcactt gctgctctcc aaagggaatg ggattcagtt tacccgaaag 12420
agttcctgcg ttacgaccct cccaagggaa ccgggtcacg gaggcttgta gatgttttcc 12480
ttaatgattc gagctttgac ccatatgatg tgataatgta tgttgtaagt ggagcttacc 12540
tccatgaccc tgagttcaac ctgtcttaca gcctgaaaga aaaggagatc aaggaaacag 12600
gtagactttt tgctaaaatg acttacaaaa tgagggcatg ccaagtgatt gctgaaaatc 12660
taatctcaaa cgggattggc aaatatttta aggacaatgg gatggccaag gatgagcacg 12720
atttgactaa ggcactccac actctagctg tctcaggagt ccccaaagat ctcaaagaaa 12780
gtcacagggg ggggccagtc ttaaaaacct actcccgaag cccagtccac acaagtacca 12840
ggaacgtgag agcagcaaaa gggtttatag ggttccctca agtaattcgg caggaccaag 12900
acactgatca tccggagaat atggaagctt acgagacagt cagtgcattt atcacgactg 12960
atctcaagaa gtactgcctt aattggagat atgagaccat cagcttgttt gcacagaggc 13020
taaatgagat ttacggattg ccctcatttt tccagtggct gcataagagg cttgagacct 13080

ctgtcctgta tgtaagtgac cctcattgcc cccccgacct tgacgcccat atcccgttat 13140

ataaagtccc caatgatcaa atcttcatta agtaccctat gggaggtata gaagggtatt 13200

gtcagaagct gtggaccatc agcaccattc cctatctata cctggctgct tatgagagcg 13260

gagtaaggat tgcttcgtta gtgcaagggg acaatcagac catagccgta acaaaaaggg 13320

tacccagcac atggccctac aaccttaaga aacgggaagc tgctagagta actagagatt 13380

actttgtaat tcttaggcaa aggctacatg atattggcca tcacctcaag gcaaatgaga 13440

caattgtttc atcacatttt tttgtctatt caaaaggaat atattatgat gggctacttg 13500

tgtcccaatc actcaagagc atcgcaagat gtgtattctg gtcagagact atagttgatg 13560

aaacaagggc agcatgcagt aatattgcta caacaatggc taaaagcatc gagagaggtt 13620

atgaccgtta ccttgcatat tccctgaacg tcctaaaagt gatacagcaa attctgatct 13680

ctcttggctt cacaatcaat tcaaccatga cccgggatgt agtcataccc ctcctcacaa 13740

acaacgacct cttaataagg atggcactgt tgcccgctcc tattgggggg atgaattatc 13800

tgaatatgag caggctgttt gtcagaaaca tcggtgatcc agtaacatca tcaattgctg 13860

atctcaagag aatgattctc gcctcactaa tgcctgaaga gaccctccat caagtaatga 13920

cacaacaacc ggggactctt tcattcctag actgggctag cgacccttac tcagcaaatc 13980

ttgtatgtgt ccagagcatc actagactcc tcaagaacat aactgcaagg tttgtcctga 14040

tccatagtcc aaacccaatg ttaaaaggat tattccatga tgacagtaaa gaagaggacg 14100

agggactggc ggcattcctc atggacaggc atattatagt acctagggca gctcatgaaa 14160

tcctggatca tagtgtcaca ggggcaagag agtctattgc aggcatgctg gataccacaa 14220

aaggcttgat tcgagccagc atgaggaagg gggggttaac ctctcgagtg ataaccagat 14280

tgtccaatta tgactatgaa caattcagag cagggatggt gctattgaca ggaagaaaga 14340

gaaatgtcct cattgacaaa gagtcatgtt cagtgcagct ggcgagagct ctaagaagcc 14400

atatgtgggc gaggctagct cgaggacggc ctatttacgg ccttgaggtc cctgatgtac 14460

tagaatctat gcgaggccac cttattcggc gtcatgagac atgtgtcatc tgcgagtgtg 14520

gatcagtcaa ctacggatgg ttttttgtcc cctcgggttg ccaactggat gatattgaca 14580

aggaaacatc atccttgaga gtcccatata ttggttctac cactgatgag agaacagaca 14640

tgaagcttgc cttcgtaaga gccccaagtc gatccttgcg atctgctgtt agaatagcaa 14700

cagtgtactc atgggcttac ggtgatgatg atagctcttg gaacgaagcc tggttgttgg 14760

ctaggcaaag ggccaatgtg agcctggagg agctaagggt gatcactccc atctcaactt 14820

cgactaattt agcgcatagg ttgagggatc gtagcactca agtgaaatac tcaggtacat 14880

cccttgtccg agtggcgagg tataccacaa tctccaacga caatctctca tttgtcatat 14940

cagataagaa ggttgatact aactttatat accaacaagg aatgcttcta gggttgggtg 15000

ttttagaaac attgtttcga ctcgagaaag ataccggatc atctaacacg gtattacatc 15060

ttcacgtcga aacagattgt tgcgtgatcc cgatgataga tcatcccagg atacccagct 15120

cccgcaagct agagctgagg gcagagctat gtaccaaccc attgatatat gataatgcac 15180

ctttaattga cagagatgca acaaggctat acacccagag ccataggagg caccttgtgg 15240

aatttgttac atggtccaca ccccaactat atcacatttt agctaagtcc acagcactat 15300

ctatgattga cctggtaaca aaatttgaga aggaccatat gaatgaaatt tcagctctca 15360

taggggatga cgatatcaat agtttcataa ctgagtttct gctcatagag ccaagattat 15420

tcactatcta cttgggccag tgtgcggcca tcaattgggc atttgatgta cattatcata 15480

gaccatcagg gaaatatcag atgggtgagc tgttgtcatc gttcctttct agaatgagca 15540

aaggagtgtt taaggtgctt gtcaatgctc taagccaccc aaagatctac aagaaattct 15600

ggcattgtgg tattatagag cctatccatg gtccttcact tgatgctcaa aacttgcaca 15660

caactgtgtg caacatggtt tacacatgct atatgaccta cctcgacctg ttgttgaatg 15720

aagagttaga agagttcaca tttctcttgt gtgaaagcga cgaggatgta gtaccggaca 15780

gattcgacaa catccaggca aaacacttat gtgttctggc agatttgtac tgtcaaccag 15840

ggacctgccc accaattcga ggtctaagac cggtagagaa atgtgcagtt ctaaccgacc 15900

atatcaaggc agaggctatg ttatctccag caggatcttc gtggaacata aatccaatta 15960

ttgtagacca ttactcatgc tctctgactt atctccggcg aggatcgatc aaacagataa 16020

gattgagagt tgatccagga ttcattttcg acgccctcgc tgaggtaaat gtcagtcagc 16080

caaagatcgg cagcaacaac atctcaaata tgagcatcaa ggctttcaga cccccacacg 16140

atgatgttgc aaaattgctc aaagatatca acacaagcaa gcacaatctt cccatttcag 16200

ggggcaatct cgccaattat gaaatccatg ctttccgcag aatcgggttg aactcatctg 16260

cttgctacaa agctgttgag atatcaacat taattaggag atgccttgag ccaggggagg 16320

acggcttgtt cttgggtgag ggatcgggtt ctatgttgat cacttataaa gagatactta 16380

aactaaacaa gtgcttctat aatagtgggg tttccgccaa ttctagatct ggtcaaaggg 16440

aattagcacc ctatccctcc gaagttggcc ttgtcgaaca cagaatggga gtaggtaata 16500

ttgtcaaagt gctctttaac gggaggcccg aagtcacgtg ggtaggcagt gtagattgct 16560

tcaatttcat agttagtaat atccctacct ctagtgtggg gtttatccat tcagatatag 16620

agaccttgcc tgacaaagat actatagaga agctagagga attggcagcc atcttatcga 16680

tggctctgct cctgggcaaa ataggatcaa tactggtgat taagcttatg cctttcagcg 16740

gggattttgt tcagggattt ataagttatg tagggtctca ttatagagaa gtgaaccttg 16800

tataccctag atacagcaac ttcatctcta ctgaatctta tttggttatg acagatctca 16860

aggctaaccg gctaatgaat cctgaaaaga ttaagcagca gataattgaa tcatctgtga 16920

ggacttcacc tggacttata ggtcacatcc tatccattaa gcaactaagc tgcatacaag 16980

caattgtggg agacgcagtt agtagaggtg atatcaatcc tactctgaaa aaacttacac 17040

ctatagagca ggtgctgatc aattgcgggt tggcaattaa cggacctaag ctgtgcaaag 17100

aattgatcca ccatgatgtt gcctcagggc aagatggatt gcttaattct atactcatcc 17160

tctacaggga gttggcaaga ttcaaagaca accaaagaag tcaacaaggg atgttccacg 17220

cttaccccgt attggtaagt agcaggcaac gagaacttat atctaggatc acccgcaaat 17280

tctgggggca cattcttctt tactccggga acaaaaagtt gataaataag tttatccaga 17340

atctcaagtc cggctatctg atactagact tacaccagaa tatcttcgtt aagaatctat 17400

ccaagtcaga gaaacagatt attatgacgg ggggtttgaa acgtgagtgg gtttttaagg 17460

taacagtcaa ggagaccaaa gaatggtata agttagtcgg atacagtgcc ctgattaagg 17520

actaattggt tgaactccgg aaccctaatc ctgccctagg tggttaggca ttatttgcaa 17580

tatattaaag aaaactttga aaatacgaag tttctattcc cagctttgtc tggt 17634

<210> 10
<211> 18516
<212> DNA
<213> Artificial Sequence

<220>
<223> MV Noro VP1 ATU VP2 optimized cDNA plus strand

<400> 10
accaaacaaa gttgggtaag gatagttcaa tcaatgatca tcttctagtg cacttaggat 60

tcaagatcct attatcaggg acaagagcag gattagggat atccgagatg gccacacttt 120

taaggagctt agcattgttc aaaagaaaca aggacaaacc acccattaca tcaggatccg 180

gtggagccat cagaggaatc aaacacatta ttatagtacc aatccctgga gattcctcaa 240

ttaccactcg atccagactt ctggaccggt tggtgaggtt aattggaaac ccggatgtga 300

gcgggcccaa actaacaggg gcactaatag gtatattatc cttatttgtg gagtctccag 360

gtcaattgat tcagaggatc accgatgacc ctgacgttag cataaggctg ttagaggttg 420

tccagagtga ccagtcacaa tctggcctta ccttcgcatc aagaggtacc aacatggagg 480

atgaggcgga ccaatacttt tcacatgatg atccaattag tagtgatcaa tccaggttcg 540

gatggttcgg gaacaaggaa atctcagata ttgaagtgca agaccctgag ggattcaaca 600

tgattctggg taccatccta gcccaaattt gggtcttgct cgcaaaggcg gttacggccc 660

cagacacggc agctgattcg gagctaagaa ggtggataaa gtacacccaa caaagaaggg 720

tagttggtga atttagattg gagagaaaat ggttggatgt ggtgaggaac aggattgccg 780

aggacctctc cttacgccga ttcatggtcg ctctaatcct ggatatcaag agaacacccg 840
gaaacaaacc caggattgct gaaatgatat gtgacattga tacatatatc gtagaggcag 900
gattagccag ttttatcctg actattaagt ttgggataga aactatgtat cctgctcttg 960
gactgcatga atttgctggt gagttatcca cacttgagtc cttgatgaac ctttaccagc 1020
aaatggggga aactgcaccc tacatggtaa tcctggagaa ctcaattcag aacaagttca 1080
gtgcaggatc ataccctctg ctctggagct atgccatggg agtaggagtg gaacttgaaa 1140
actccatggg aggtttgaac tttggccgat cttactttga tccagcatat tttagattag 1200
ggcaagagat ggtaaggagg tcagctggaa aggtcagttc cacattggca tctgaactcg 1260
gtatcactgc cgaggatgca aggcttgttt cagagattgc aatgcatact actgaggaca 1320
agatcagtag agcggttgga cccagacaag cccaagtatc atttctacac ggtgatcaaa 1380
gtgagaatga gctaccgaga ttggggggca aggaagatag gagggtcaaa cagagtcgag 1440
gagaagccag ggagagctac agagaaaccg ggcccagcag agcaagtgat gcgagagctg 1500
cccatcttcc aaccggcaca cccctagaca ttgacactgc aacggagtcc agccaagatc 1560
cgcaggacag tcgaaggtca gctgacgccc tgcttaggct gcaagccatg gcaggaatct 1620
cggaagaaca aggctcagac acggacaccc ctatagtgta caatgacaga aatcttctag 1680
actaggtgcg agaggccgag ggccagaaca acatccgcct accatccatc attgttataa 1740
aaaacttagg aaccaggtcc acacagccgc cagcccatca accatccact cccacgattg 1800
gagccaatgg cagaagagca ggcacgccat gtcaaaaacg gactggaatg catccgggct 1860
ctcaaggccg agcccatcgg ctcactggcc atcgaggaag ctatggcagc atggtcagaa 1920
atatcagaca acccaggaca ggagcgagcc acctgcaggg aagagaaggc aggcagttcg 1980
ggtctcagca aaccatgcct ctcagcaatt ggatcaactg aaggcggtgc acctcgcatc 2040
cgcggtcagg gacctggaga gagcgatgac gacgctgaaa ctttgggaat ccccccaaga 2100
aatctccagg catcaagcac tgggttacag tgttattacg tttatgatca cagcggtgaa 2160
gcggttaagg gaatccaaga tgctgactct atcatggttc aatcaggcct tgatggtgat 2220
agcaccctct caggaggaga caatgaatct gaaaacagcg atgtggatat tggcgaacct 2280
gataccgagg gatatgctat cactgaccgg ggatctgctc ccatctctat ggggttcagg 2340
gcttctgatg ttgaaactgc agaaggaggg gagatccacg agctcctgag actccaatcc 2400
agaggcaaca actttccgaa gcttgggaaa actctcaatg ttcctccgcc cccggacccc 2460
ggtagggcca gcacttccgg gacacccatt aaaaagggca cagacgcgag attagcctca 2520
tttggaacgg agatcgcgtc tttattgaca ggtggtgcaa cccaatgtgc tcgaaagtca 2580
ccctcggaac catcagggcc aggtgcacct gcggggaatg tccccgagtg tgtgagcaat 2640
gccgcactga tacaggagtg gacacccgaa tctggtacca caatctcccc gagatcccag 2700

aataatgaag aagggggaga ctattatgat gatgagctgt tctctgatgt ccaagatatt 2760

aaaacagcct tggccaaaat acacgaggat aatcagaaga taatctccaa gctagaatca 2820

ctgctgttat tgaagggaga agttgagtca attaagaagc agatcaacag gcaaaatatc 2880

agcatatcca ccctggaagg acacctctca agcatcatga tcgccattcc tggacttggg 2940

aaggatccca acgaccccac tgcagatgtc gaaatcaatc ccgacttgaa acccatcata 3000

ggcagagatt caggccgagc actggccgaa gttctcaaga aacccgttgc cagccgacaa 3060

ctccaaggaa tgacaaatgg acggaccagt tccagaggac agctgctgaa ggaatttcag 3120

ctaaagccga tcgggaaaaa gatgagctca gccgtcgggt ttgttcctga caccggccct 3180

gcatcacgca gtgtaatccg ctccattata aaatccagcc ggctagagga ggatcggaag 3240

cgttacctga tgactctcct tgatgatatc aaaggagcca atgatcttgc caagttccac 3300

cagatgctga tgaagataat aatgaagtag ctacagctca acttacctgc caaccccatg 3360

ccagtcgacc caactagcct accctccatc attgttataa aaaacttagg aaccaggtcc 3420

acacagccgc cagcccatca acgcgtacgg ccaccatgaa gatggcttcc tctgacgcca 3480

atccttccga tggaagcgct gctaatctgg tgcccgaggt caacaacgag gtaatggcac 3540

tggaacccgt cgtgggtgca gcgattgccg cccctgttgc tggacagcag aatgtgatcg 3600

atccctggat acgcaataat ttcgtgcagg cgcctggagg cgaattcacg gtgtctccta 3660

gaaatgcccc aggcgaaatt ctgtggtcag ctcctctggg gcccgacttg aacccatacc 3720

tcagtcatct cgcacgaatg tacaacgggt atgccggagg ctttgaagtg caggtgatcc 3780

ttgccggaaa cgcattcacc gccgggaaag tcatcttcgc cgctgtccca cccaatttcc 3840

ctaccgaagg gctttcacca agtcaggtaa ccatgtttcc acacatcgtt gtggatgtcc 3900

gccagcttga acctgtactc atccctctgc cagatgttag gaacaacttc taccactaca 3960

accagagcaa cgatccaacc attaagctga tcgccatgtt gtatacacca ctcagagcca 4020

ataacgcagg cgatgatgtg ttcactgtct cttgtagagt cctgacacga ccctctcccg 4080

atttcgactt cattttcctc gtgccaccca ccgttgagag ccggacaaaa ccgtttagcg 4140

tacccgtgct gactgtcgag gagatgacca attctaggtt ccccattcct ctggagaagc 4200

tgtttactgg accgtccagc gcctttgttg tgcagccaca gaacggccgc tgtactactg 4260

atggcgtgct gcttggtaca acccaactga gcccagtgaa tatctgcacc ttcagagggg 4320

acgtgactca tatcaccgga tcccgcaact acacaatgaa cctggcttca cagaactgga 4380

atgactacga tccaacggag gaaataccgg ctcctctggg tactccggac ttcgtcggca 4440

agattcaggg agtgctgacc caaactaccc gtactgatgg gagcacccgt ggccacaaag 4500

ctaccgtgta tacaggcagt gctgactttg cgcctaaact gggtagggtg caatttgaga 4560

ctgacaccga tcgggacttt gaggccaatc agaataccaa gtttacaccc gtcggcgtca 4620

tacaggacgg cgggacgaca catcgcaacg aaccccagca atgggtattg ccctcctatt 4680

ccggtagaaa cacgcacaac gttcacttgg caccagccgt ggcccctacc tttcctggag 4740

agcaactgct gttcttccga tctacaatgc cggggtgtag tggctatcca aacatggacc 4800

tcgactgcct cctgccccaa gagtgggttc agtactttta tcaggaagcc gcacccgccc 4860

agagcgacgt tgcactgttg cggtttgtaa atcccgatac gggtcgggtg ctcttcgagt 4920

gcaagctgca taaatcaggc tatgtcacag tggcacacac agggcaacat gaccttgtga 4980

ttccgcctaa cggttacttc cggtttgaca gctgggtgaa tcagttttac actctggctc 5040

ctatgggaaa tgggacaggc aggagaaggg cggtttgata gcctaccctc catcattgtt 5100

ataaaaaact taggaaccag gtccacacag ccgccagccc atcaacggcc accatggcag 5160

gagccttctt tgcaggattg gcaagcgatg ttctgggctc cggacttggt tccctgatca 5220

acgcaggagc tggcgccatc aatcagaagg tcgaattcga gaacaatcgg aagctgcagc 5280

aagcttcctt tcagttctct agcaatctgc agcaagcctc atttcagcat gacaaagaga 5340

tgctgcaggc tcagatagag gccacgaaga aactgcagca agagatgatg aaagtgaagc 5400

aagcgatgct ccttgaaggt ggcttctttg agactgacgc tgcgagaggc gccattaatg 5460

ccccgatgac gaaagccctg gactggtcag ggaccaggta ttgggctcct gatgcccgca 5520

ccactacata caacgccggt cgcttttcca ctcctcaacc atcaggggca ctcccaggac 5580

gggcgaatct gagggatgct gtgcccgcaa gaggcagcag ctccaagagc agtaacagct 5640

caaccgctac aagcgtgtac agcaaccaga cgacctcaac ccggttgggc tccacagccg 5700

tatctggtac ctctgtgagc tctttcccct ctactgcccg tactcgaagt tgggtggaag 5760

atcagtcccg gaatctctct cccagtatgc gtggggctca caacatcagc ttcgttaccc 5820

caccctctag tcgctcaagt agccagggca cagtgtccac agtccctaaa gaagtcctgg 5880

actcctggac aggggccttt aacaccagaa ggcagcctct cttcgcccac attaggaaga 5940

gaggggagag tcgagcataa tgagcgcgca gcgcttagac gtctcgcgat cgatactagt 6000

acaacctaaa tccattataa aaaacttagg agcaaagtga ttgcctccca aggtccacaa 6060

tgacagagac ctacgacttc gacaagtcgg catgggacat caaagggtcg atcgctccga 6120

tacaacccac cacctacagt gatggcaggc tggtgcccca ggtcagagtc atagatcctg 6180

gtctaggcga caggaaggat gaatgcttta tgtacatgtt tctgctgggg gttgttgagg 6240

acagcgattc cctagggcct ccaatcgggc gagcatttgg gttcctgccc ttaggtgttg 6300

gcagatccac agcaaagccc gaaaaactcc tcaaagaggc cactgagctt gacatagttg 6360

ttagacgtac agcagggctc aatgaaaaac tggtgttcta caacaacacc ccactaactc 6420

tcctcacacc ttggagaaag gtcctaacaa cagggagtgt cttcaacgca aaccaagtgt 6480

gcaatgcggt taatctgata ccgctcgata ccccgcagag gttccgtgtt gtttatatga 6540

gcatcacccg tctttcggat aacgggtatt acaccgttcc tagaagaatg ctggaattca 6600

gatcggtcaa tgcagtggcc ttcaacctgc tggtgaccct taggattgac aaggcgatag 6660

gccctgggaa gatcatcgac aatacagagc aacttcctga ggcaacattt atggtccaca 6720

tcgggaactt caggagaaag aagagtgaag tctactctgc cgattattgc aaaatgaaaa 6780

tcgaaaagat gggcctggtt tttgcacttg gtgggatagg gggcaccagt cttcacatta 6840

gaagcacagg caaaatgagc aagactctcc atgcacaact cgggttcaag aagaccttat 6900

gttacccgct gatggatatc aatgaagacc ttaatcgatt actctggagg agcagatgca 6960

agatagtaag aatccaggca gttttgcagc catcagttcc tcaagaattc cgcatttacg 7020

acgacgtgat cataaatgat gaccaaggac tattcaaagt tctgtagacc gtagtgccca 7080

gcaatgcccg aaaacgaccc ccctcacaat gacagccaga aggcccggac aaaaaagccc 7140

cctccgaaag actccacgga ccaagcgaga ggccagccag cagccgacgg caagcgcgaa 7200

caccaggcgg ccccagcaca gaacagccct gacacaaggc caccaccagc caccccaatc 7260

tgcatcctcc tcgtgggacc cccgaggacc aacccccaag gctgcccccg atccaaacca 7320

ccaaccgcat ccccaccacc cccgggaaag aaacccccag caattggaag gcccctcccc 7380

ctcttcctca acacaagaac tccacaaccg aaccgcacaa gcgaccgagg tgacccaacc 7440

gcaggcatcc gactccctag acagatcctc tctccccggc aaactaaaca aaacttaggg 7500

ccaaggaaca tacacaccca acagaaccca gaccccggcc cacggcgccg cgcccccaac 7560

ccccgacaac cagagggagc ccccaaccaa tcccgccggc tcccccggtg cccacaggca 7620

gggacaccaa cccccgaaca gacccagcac ccaaccatcg acaatccaag acgggggggc 7680

ccccccaaaa aaaggccccc aggggccgac agccagcacc gcgaggaagc ccacccaccc 7740

cacacacgac cacggcaacc aaaccagaac ccagaccacc ctgggccacc agctcccaga 7800

ctcggccatc accccgcaga aaggaaaggc cacaacccgc gcaccccagc cccgatccgg 7860

cggggagcca cccaacccga accagcaccc aagagcgatc cccgaaggac ccccgaaccg 7920

caaaggacat cagtatccca cagcctctcc aagtcccccg gtctcctcct cttctcgaag 7980

ggaccaaaag atcaatccac cacacccgac gacactcaac tccccacccc taaaggagac 8040

accgggaatc ccagaatcaa gactcatcca atgtccatca tgggtctcaa ggtgaacgtc 8100

tctgccatat tcatggcagt actgttaact ctccaaacac ccaccggtca aatccattgg 8160

ggcaatctct ctaagatagg ggtggtagga ataggaagtg caagctacaa agttatgact 8220

cgttccagcc atcaatcatt agtcataaaa ttaatgccca atataactct cctcaataac 8280

tgcacgaggg tagagattgc agaatacagg agactactga gaacagtttt ggaaccaatt 8340

agagatgcac ttaatgcaat gacccagaat ataagaccgg ttcagagtgt agcttcaagt 8400
aggagacaca agagatttgc gggagtagtc ctggcaggtg cggccctagg cgttgccaca 8460
gctgctcaga taacagccgg cattgcactt caccagtcca tgctgaactc tcaagccatc 8520
gacaatctga gagcgagcct ggaaactact aatcaggcaa ttgagacaat cagacaagca 8580
gggcaggaga tgatattggc tgttcagggt gtccaagact acatcaataa tgagctgata 8640
ccgtctatga accaactatc ttgtgattta atcggccaga agctcgggct caaattgctc 8700
agatactata cagaaatcct gtcattattt ggccccagtt tacgggaccc catatctgcg 8760
gagatatcta tccaggcttt gagctatgcg cttggaggag acatcaataa ggtgttagaa 8820
aagctcggat acagtggagg tgatttactg ggcatcttag agagcggagg aataaaggcc 8880
cggataactc acgtcgacac agagtcctac ttcattgtcc tcagtatagc ctatccgacg 8940
ctgtccgaga ttaagggggt gattgtccac cggctagagg gggtctcgta caacataggc 9000
tctcaagagt ggtataccac tgtgcccaag tatgttgcaa cccaagggta ccttatctcg 9060
aattttgatg agtcatcgtg tactttcatg ccagagggga ctgtgtgcag ccaaaatgcc 9120
ttgtacccga tgagtcctct gctccaagaa tgcctccggg ggtacaccaa gtcctgtgct 9180
cgtacactcg tatccgggtc ttttgggaac cggttcattt tatcacaagg gaacctaata 9240
gccaattgtg catcaatcct ttgcaagtgt tacacaacag gaacgatcat taatcaagac 9300
cctgacaaga tcctaacata cattgctgcc gatcactgcc cggtagtcga ggtgaacggc 9360
gtgaccatcc aagtcgggag caggaggtat ccagacgctg tgtacttgca cagaattgac 9420
ctcggtcctc ccatatcatt ggagaggttg gacgtaggga caaatctggg gaatgcaatt 9480
gctaagttgg aggatgccaa ggaattgttg gagtcatcgg accagatatt gaggagtatg 9540
aaaggtttat cgagcactag catagtctac atcctgattg cagtgtgtct tggagggttg 9600
atagggatcc ccgctttaat atgttgctgc agggggcgtt gtaacaaaaa gggagaacaa 9660
gttggtatgt caagaccagg cctaaagcct gatcttacgg gaacatcaaa atcctatgta 9720
aggtcgctct gatcctctac aactcttgaa acacaaatgt cccacaagtc tcctcttcgt 9780
catcaagcaa ccaccgcacc cagcatcaag cccacctgaa attatctccg gcttccctct 9840
ggccgaacaa tatcggtagt taatcaaaac ttagggtgca agatcatcca caatgtcacc 9900
acaacgagac cggataaatg ccttctacaa agataacccc catcccaagg gaagtaggat 9960
agtcattaac agagaacatc ttatgattga tagaccttat gttttgctgg ctgttctgtt 10020
tgtcatgttt ctgagcttga tcgggttgct agccattgca ggcattagac ttcatcgggc 10080
agccatctac accgcagaga tccataaaag cctcagcacc aatctagatg taactaactc 10140
aatcgagcat caggtcaagg acgtgctgac accactcttc aaaatcatcg gtgatgaagt 10200
gggcctgagg acacctcaga gattcactga cctagtgaaa ttaatctctg acaagattaa 10260

attccttaat ccggataggg agtacgactt cagagatctc acttggtgta tcaacccgcc 10320

agagagaatc aaattggatt atgatcaata ctgtgcagat gtggctgctg aagagctcat 10380

gaatgcattg gtgaactcaa ctctactgga gaccagaaca accaatcagt tcctagctgt 10440

ctcaaaggga aactgctcag ggcccactac aatcagaggt caattctcaa acatgtcgct 10500

gtccctgtta gacttgtatt taggtcgagg ttacaatgtg tcatctatag tcactatgac 10560

atcccaggga atgtatgggg gaacttacct agtggaaaag cctaatctga gcagcaaaag 10620

gtcagagttg tcacaactga gcatgtaccg agtgtttgaa gtaggtgtta tcagaaatcc 10680

gggtttgggg gctccggtgt tccatatgac aaactatctt gagcaaccag tcagtaatga 10740

tctcagcaac tgtatggtgg ctttggggga gctcaaactc gcagcccttt gtcacgggga 10800

agattctatc acaattccct atcagggatc agggaaaggt gtcagcttcc agctcgtcaa 10860

gctaggtgtc tggaaatccc caaccgacat gcaatcctgg gtccccttat caacggatga 10920

tccagtgata gacaggcttt acctctcatc tcacagaggt gttatcgctg acaatcaagc 10980

aaaatgggct gtcccgacaa cacgaacaga tgacaagttg cgaatggaga catgcttcca 11040

acaggcgtgt aagggtaaaa tccaagcact ctgcgagaat cccgagtggg caccattgaa 11100

ggataacagg attccttcat acggggtctt gtctgttgat ctgagtctga cagttgagct 11160

taaaatcaaa attgcttcgg gattcgggcc attgatcaca cacggttcag ggatggacct 11220

atacaaatcc aaccacaaca atgtgtattg gctgactatc ccgccaatga agaacctagc 11280

cttaggtgta atcaacacat tggagtggat accgagattc aaggttagtc cctacctctt 11340

cactgtccca attaaggaag caggcgaaga ctgccatgcc ccaacatacc tacctgcgga 11400

ggtggatggt gatgtcaaac tcagttccaa tctggtgatt ctacctggtc aagatctcca 11460

atatgttttg gcaacctacg atacttccag ggttgaacat gctgtggttt attacgttta 11520

cagcccaagc cgctcatttt cttactttta tccttttagg ttgcctataa aggggtcccc 11580

catcgaatta caagtggaat gcttcacatg ggaccaaaaa ctctggtgcc gtcacttctg 11640

tgtgcttgcg gactcagaat ctggtggaca tatcactcac tctgggatgg tgggcatggg 11700

agtcagctgc acagtcaccc gggaagatgg aaccaatcgc agatagggct gctagtgaac 11760

caatcacatg atgtcaccca gacatcaggc atacccacta gtgtgaaata gacatcagaa 11820

ttaagaaaaa cgtagggtcc aagtggttcc ccgttatgga ctcgctatct gtcaaccaga 11880

tcttataccc tgaagttcac ctagatagcc cgatagttac caataagata gtagccatcc 11940

tggagtatgc tcgagtccct cacgcttaca gcctggagga ccctacactg tgtcagaaca 12000

tcaagcaccg cctaaaaaac ggattttcca accaaatgat tataaacaat gtggaagttg 12060

ggaatgtcat caagtccaag cttaggagtt atccggccca ctctcatatt ccatatccaa 12120

attgtaatca ggatttattt aacatagaag acaaagagtc aacgaggaag atccgtgaac 12180
tcctcaaaaa ggggaattcg ctgtactcca aagtcagtga taaggttttc caatgcttaa 12240
gggacactaa ctcacggctt ggcctaggct ccgaattgag ggaggacatc aaggagaaag 12300
ttattaactt gggagtttac atgcacagct cccagtggtt tgagcccttt ctgttttggt 12360
ttacagtcaa gactgagatg aggtcagtga ttaaatcaca aacccatact tgccatagga 12420
ggagacacac acctgtattc ttcactggta gttcagttga gttgctaatc tctcgtgacc 12480
ttgttgctat aatcagtaaa gagtctcaac atgtatatta cctgacattt gaactggttt 12540
tgatgtattg tgatgtcata gaggggaggt taatgacaga gaccgctatg actattgatg 12600
ctaggtatac agagcttcta ggaagagtca gatacatgtg gaaactgata gatggtttct 12660
tccctgcact cgggaatcca acttatcaaa ttgtagccat gctggagcct ctttcacttg 12720
cttacctgca gctgagggat ataacagtag aactcagagg tgctttcctt aaccactgct 12780
ttactgaaat acatgatgtt cttgaccaaa acgggttttc tgatgaaggt acttatcatg 12840
agttaactga agctctagat tacattttca taactgatga catacatctg acaggggaga 12900
ttttctcatt tttcagaagt ttcggccacc ccagacttga agcagtaacg gctgctgaaa 12960
atgttaggaa atacatgaat cagcctaaag tcattgtgta tgagactctg atgaaaggtc 13020
atgccatatt ttgtggaatc ataatcaacg gctatcgtga caggcacgga ggcagttggc 13080
caccgctgac cctccccctg catgctgcag acacaatccg gaatgctcaa gcttcaggtg 13140
aagggttaac acatgagcag tgcgttgata actggaaatc ttttgctgga gtgaaatttg 13200
gctgctttat gcctcttagc ctggatagtg atctgacaat gtacctaaag gacaaggcac 13260
ttgctgctct ccaaagggaa tgggattcag tttacccgaa agagttcctg cgttacgacc 13320
ctcccaaggg aaccgggtca cggaggcttg tagatgtttt ccttaatgat tcgagctttg 13380
acccatatga tgtgataatg tatgttgtaa gtggagctta cctccatgac cctgagttca 13440
acctgtctta cagcctgaaa gaaaaggaga tcaaggaaac aggtagactt tttgctaaaa 13500
tgacttacaa aatgagggca tgccaagtga ttgctgaaaa tctaatctca aacgggattg 13560
gcaaatattt taaggacaat gggatggcca aggatgagca cgatttgact aaggcactcc 13620
acactctagc tgtctcagga gtccccaaag atctcaaaga aagtcacagg ggggggccag 13680
tcttaaaaac ctactcccga agcccagtcc acacaagtac caggaacgtg agagcagcaa 13740
aagggtttat agggttccct caagtaattc ggcaggacca agacactgat catccggaga 13800
atatggaagc ttacgagaca gtcagtgcat ttatcacgac tgatctcaag aagtactgcc 13860
ttaattggag atatgagacc atcagcttgt ttgcacagag gctaaatgag atttacggat 13920
tgccctcatt tttccagtgg ctgcataaga ggcttgagac ctctgtcctg tatgtaagtg 13980
accctcattg cccccccgac cttgacgccc atatcccgtt atataaagtc cccaatgatc 14040

aaatcttcat taagtaccct atgggaggta tagaagggta ttgtcagaag ctgtggacca 14100

tcagcaccat tccctatcta tacctggctg cttatgagag cggagtaagg attgcttcgt 14160

tagtgcaagg ggacaatcag accatagccg taacaaaaag ggtacccagc acatggccct 14220

acaaccttaa gaaacgggaa gctgctagag taactagaga ttactttgta attcttaggc 14280

aaaggctaca tgatattggc catcacctca aggcaaatga gacaattgtt tcatcacatt 14340

tttttgtcta ttcaaaagga atatattatg atgggctact tgtgtcccaa tcactcaaga 14400

gcatcgcaag atgtgtattc tggtcagaga ctatagttga tgaaacaagg gcagcatgca 14460

gtaatattgc tacaacaatg gctaaaagca tcgagagagg ttatgaccgt taccttgcat 14520

attccctgaa cgtcctaaaa gtgatacagc aaattctgat ctctcttggc ttcacaatca 14580

attcaaccat gacccgggat gtagtcatac ccctcctcac aaacaacgac ctcttaataa 14640

ggatggcact gttgcccgct cctattgggg ggatgaatta tctgaatatg agcaggctgt 14700

ttgtcagaaa catcggtgat ccagtaacat catcaattgc tgatctcaag agaatgattc 14760

tcgcctcact aatgcctgaa gagaccctcc atcaagtaat gacacaacaa ccgggggact 14820

cttcattcct agactgggct agcgaccctt actcagcaaa tcttgtatgt gtccagagca 14880

tcactagact cctcaagaac ataactgcaa ggtttgtcct gatccatagt ccaaacccaa 14940

tgttaaaagg attattccat gatgacagta aagaagagga cgagggactg gcggcattcc 15000

tcatggacag gcatattata gtacctaggg cagctcatga aatcctggat catagtgtca 15060

caggggcaag agagtctatt gcaggcatgc tggataccac aaaaggcttg attcgagcca 15120

gcatgaggaa gggggggtta acctctcgag tgataaccag attgtccaat tatgactatg 15180

aacaattcag agcagggatg gtgctattga caggaagaaa gagaaatgtc ctcattgaca 15240

aagagtcatg ttcagtgcag ctggcgagag ctctaagaag ccatatgtgg gcgaggctag 15300

ctcgaggacg gcctatttac ggccttgagg tccctgatgt actagaatct atgcgaggcc 15360

accttattcg gcgtcatgag acatgtgtca tctgcgagtg tggatcagtc aactacggat 15420

ggttttttgt cccctcgggt tgccaactgg atgatattga caaggaaaca tcatccttga 15480

gagtcccata tattggttct accactgatg agagaacaga catgaagctt gccttcgtaa 15540

gagccccaag tcgatccttg cgatctgctg ttagaatagc aacagtgtac tcatgggctt 15600

acggtgatga tgatagctct tggaacgaag cctggttgtt ggctaggcaa agggccaatg 15660

tgagcctgga ggagctaagg gtgatcactc ccatctcaac ttcgactaat ttagcgcata 15720

ggttgaggga tcgtagcact caagtgaaat actcaggtac atcccttgtc cgagtggcga 15780

ggtataccac aatctccaac gacaatctct catttgtcat atcagataag aaggttgata 15840

ctaactttat ataccaacaa ggaatgcttc tagggttggg tgttttagaa acattgtttc 15900

gactcgagaa agataccgga tcatctaaca cggtattaca tcttcacgtc gaaacagatt 15960

gttgcgtgat cccgatgata gatcatccca ggatacccag ctcccgcaag ctagagctga 16020

gggcagagct atgtaccaac ccattgatat atgataatgc acctttaatt gacagagatg 16080

caacaaggct atacacccag agccatagga ggcaccttgt ggaatttgtt acatggtcca 16140

caccccaact atatcacatt ttagctaagt ccacagcact atctatgatt gacctggtaa 16200

caaaatttga gaaggaccat atgaatgaaa tttcagctct cataggggat gacgatatca 16260

atagtttcat aactgagttt ctgctcatag agccaagatt attcactatc tacttgggcc 16320

agtgtgcggc catcaattgg gcatttgatg tacattatca tagaccatca gggaaatatc 16380

agatgggtga gctgttgtca tcgttccttt ctagaatgag caaaggagtg tttaaggtgc 16440

ttgtcaatgc tctaagccac ccaaagatct acaagaaatt ctggcattgt ggtattatag 16500

agcctatcca tggtccttca cttgatgctc aaaacttgca cacaactgtg tgcaacatgg 16560

tttacacatg ctatatgacc tacctcgacc tgttgttgaa tgaagagtta gaagagttca 16620

catttctctt gtgtgaaagc gacgaggatg tagtaccgga cagattcgac aacatccagg 16680

caaaacactt atgtgttctg gcagatttgt actgtcaacc agggacctgc ccaccaattc 16740

gaggtctaag accggtagag aaatgtgcag ttctaaccga ccatatcaag gcagaggcta 16800

tgttatctcc agcaggatct tcgtggaaca taaatccaat tattgtagac cattactcat 16860

gctctctgac ttatctccgg cgaggatcga tcaaacagat aagattgaga gttgatccag 16920

gattcatttt cgacgccctc gctgaggtaa atgtcagtca gccaaagatc ggcagcaaca 16980

acatctcaaa tatgagcatc aaggctttca gacccccaca cgatgatgtt gcaaaattgc 17040

tcaaagatat caacacaagc aagcacaatc ttcccatttc agggggcaat ctcgccaatt 17100

atgaaatcca tgctttccgc agaatcgggt tgaactcatc tgcttgctac aaagctgttg 17160

agatatcaac attaattagg agatgccttg agccagggga ggacggcttg ttcttgggtg 17220

agggatcggg ttctatgttg atcacttata aagagatact taaactaaac aagtgcttct 17280

ataatagtgg ggtttccgcc aattctagat ctggtcaaag ggaattagca ccctatccct 17340

ccgaagttgg ccttgtcgaa cacagaatgg gagtaggtaa tattgtcaaa gtgctcttta 17400

acgggaggcc cgaagtcacg tgggtaggca gtgtagattg cttcaatttc atagttagta 17460

atatccctac ctctagtgtg gggtttatcc attcagatat agagaccttg cctgacaaag 17520

atactataga gaagctagag gaattggcag ccatcttatc gatggctctg ctcctgggca 17580

aaataggatc aatactggtg attaagctta tgcctttcag cggggatttt gttcagggat 17640

ttataagtta tgtagggtct cattatagag aagtgaacct tgtataccct agatacagca 17700

acttcatctc tactgaatct tatttggtta tgacagatct caaggctaac cggctaatga 17760

atcctgaaaa gattaagcag cagataattg aatcatctgt gaggacttca cctggactta 17820

taggtcacat cctatccatt aagcaactaa gctgcataca agcaattgtg ggagacgcag 17880

ttagtagagg tgatatcaat cctactctga aaaaacttac acctatagag caggtgctga 17940

tcaattgcgg gttggcaatt aacggaccta agctgtgcaa agaattgatc caccatgatg 18000

ttgcctcagg gcaagatgga ttgcttaatt ctatactcat cctctacagg gagttggcaa 18060

gattcaaaga caaccaaaga agtcaacaag ggatgttcca cgcttacccc gtattggtaa 18120

gtagcaggca acgagaactt atatctagga tcacccgcaa attctggggg cacattcttc 18180

tttactccgg gaacaaaaag ttgataaata agtttatcca gaatctcaag tccggctatc 18240

tgatactaga cttacaccag aatatcttcg ttaagaatct atccaagtca gagaaacaga 18300

ttattatgac gggggggttg aaacgtgagt gggtttttaa ggtaacagtc aaggagacca 18360

aagaatggta taagttagtc ggatacagtg ccctgattaa ggactaattg gttgaactcc 18420

ggaaccctaa tcctgcccta ggtggttagg cattatttgc aatatattaa agaaaacttt 18480

gaaaatacga agtttctatt cccagctttg tctggt 18516

<210> 11
<211> 540
<212> PRT
<213> Norovirus

<400> 11

Met Lys Met Ala Ser Asn Asp Ala Asn Pro Ser Asp Gly Ser Ala Ala
1               5                   10                  15

Asn Leu Val Pro Glu Val Asn Asn Glu Val Met Ala Leu Glu Pro Val
            20                  25                  30

Val Gly Ala Ala Ile Ala Ala Pro Val Ala Gly Gln Gln Asn Val Ile
        35                  40                  45

Asp Pro Trp Ile Arg Asn Asn Phe Val Gln Ala Pro Gly Gly Glu Phe
    50                  55                  60

Thr Val Ser Pro Arg Asn Ala Pro Gly Glu Ile Leu Trp Ser Ala Pro
65                  70                  75                  80

Leu Gly Pro Asp Leu Asn Pro Tyr Leu Ser His Leu Ala Arg Met Tyr
                85                  90                  95

Asn Gly Tyr Ala Gly Gly Phe Glu Val Gln Val Ile Leu Ala Gly Asn
            100                 105                 110

Ala Phe Thr Ala Gly Lys Ile Ile Phe Ala Ala Val Pro Pro Asn Phe
        115                 120                 125

Pro Thr Glu Gly Leu Ser Pro Ser Gln Val Thr Met Phe Pro His Ile
    130                 135                 140

Ile Val Asp Val Arg Gln Leu Glu Pro Val Leu Ile Pro Leu Pro Asp
145                 150                 155                 160

Val Arg Asn Asn Phe Tyr His Tyr Asn Gln Ser Asn Asp Ser Thr Ile
                165                 170                 175

Lys Leu Ile Ala Met Leu Tyr Thr Pro Leu Arg Ala Asn Asn Ala Gly
            180                 185                 190

Asp Asp Val Phe Thr Val Ser Cys Arg Val Leu Thr Arg Pro Ser Pro
        195                 200                 205

Asp Phe Asp Phe Ile Phe Leu Val Pro Pro Thr Val Glu Ser Arg Thr
    210                 215                 220

Lys Pro Phe Ser Val Pro Ile Leu Thr Val Glu Glu Met Thr Asn Ser
225                 230                 235                 240

Arg Phe Pro Ile Pro Leu Glu Lys Leu Phe Thr Gly Pro Ser Ser Thr
                245                 250                 255

Phe Val Val Gln Pro Gln Asn Gly Arg Cys Thr Thr Asp Gly Val Leu
            260                 265                 270

Leu Gly Thr Thr Gln Leu Ser Pro Val Asn Ile Cys Thr Phe Arg Gly
        275                 280                 285

Asp Val Thr His Ile Thr Gly Ser Arg Asn Tyr Thr Met Asn Leu Ala
    290                 295                 300

Ser Gln Asn Trp Asn Ser Tyr Asp Pro Thr Glu Glu Ile Pro Ala Pro
305                 310                 315                 320

Leu Gly Thr Pro Asp Phe Val Gly Lys Ile Gln Gly Val Leu Thr Gln
                325                 330                 335

Thr Thr Arg Thr Asp Gly Ser Thr Arg Gly His Lys Ala Thr Val Tyr
            340                 345                 350

Thr Gly Ser Ala Asp Phe Ser Pro Lys Leu Gly Arg Val Gln Phe Ala
        355                 360                 365

Thr Asp Thr Asp Asn Asp Phe Asp Thr Asn Gln Asn Thr Lys Phe Thr

370                 375                 380

Pro Val Gly Val Ile Gln Asp Gly Gly Thr Thr His Arg Asn Glu Pro
385                 390                 395                 400

Gln Gln Trp Val Leu Pro Ser Tyr Ser Gly Arg Asn Thr His Asn Val
                405                 410                 415

His Leu Ala Pro Ala Val Ala Pro Thr Phe Pro Gly Glu Gln Leu Leu
            420                 425                 430

Phe Phe Arg Ser Thr Met Pro Gly Ser Ser Gly Tyr Pro Asn Met Asp
        435                 440                 445

Leu Asp Cys Leu Leu Pro Gln Glu Trp Val Gln Tyr Phe Tyr Gln Glu
    450                 455                 460

Ala Ala Pro Ala Gln Ser Asp Val Ala Leu Leu Arg Phe Val Asn Pro
465                 470                 475                 480

Asp Thr Gly Arg Val Leu Phe Glu Cys Lys Leu His Lys Ser Gly Tyr
                485                 490                 495

Val Thr Val Ala His Thr Gly Gln His Asp Leu Val Ile Pro Pro Asn
            500                 505                 510

Gly Tyr Phe Arg Phe Asp Ser Trp Val Asn Gln Phe Tyr Thr Leu Ala
        515                 520                 525

Pro Met Gly Asn Gly Thr Gly Arg Arg Arg Ala Val
    530                 535                 540

<210>  12
<211>  268
<212>  PRT
<213>  Norovirus

<400>  12

Met Ala Gly Ala Phe Phe Ala Gly Leu Ala Ser Asp Val Leu Ser Ser
1               5                   10                  15

Gly Leu Gly Ser Leu Ile Asn Ala Gly Ala Gly Ala Ile Asn Gln Thr
            20                  25                  30

Val Glu Phe Glu Asn Asn Arg Lys Leu Gln Gln Ala Ser Phe Gln Phe
        35                  40                  45

Ser Ser Asn Leu Gln Gln Ala Ser Phe Gln His Asp Lys Glu Met Leu

50                  55                  60

Gln Ser Gln Ile Asp Ala Thr Lys Lys Leu Gln Gln Glu Met Met Arg
65                  70                  75                  80

Val Lys Gln Ala Met Leu Leu Glu Gly Gly Phe Ser Glu Thr Asp Ala
                85                  90                  95

Ala Arg Gly Ala Ile Asn Ala Pro Met Thr Lys Val Leu Asp Trp Ser
            100                 105                 110

Gly Thr Arg Tyr Trp Ala Pro Asp Ala Arg Thr Thr Thr Tyr Asn Ala
        115                 120                 125

Gly Arg Phe Ser Thr Pro Gln Ser Ser Gly Ala Pro Pro Gly Arg Ala
    130                 135                 140

Asn Leu Arg Ala Ala Val Pro Ala Gln Gly Ser Ser Ser Thr Ser Ser
145                 150                 155                 160

Asn Ser Ser Ile Ala Thr Ser Met Tyr Ser Asn Gln Thr Thr Ser Thr
                165                 170                 175

Arg Leu Gly Ser Thr Ala Gly Ser Gly Thr Ser Val Ser Ser Leu Pro
            180                 185                 190

Ser Ser Ala Arg Thr Arg Ser Trp Val Glu Asp Gln Asn Arg Asn Leu
        195                 200                 205

Ser Pro Phe Met Arg Gly Ala His Asn Ile Ser Phe Val Thr Pro Pro
    210                 215                 220

Ser Ser Arg Ser Ser Ser Gln Gly Thr Val Ser Thr Val Pro Lys Glu
225                 230                 235                 240

Val Leu Asp Ser Trp Thr Gly Ala Phe Asn Thr Arg Arg Gln Pro Leu
                245                 250                 255

Phe Ala His Ile Arg Arg Arg Gly Glu Ser Arg Val
            260                 265

<210>  13
<211>  540
<212>  PRT
<213>  Norovirus

<400>  13

Met Lys Met Ala Ser Ser Asp Ala Asn Pro Ser Asp Gly Ser Ala Ala

1               5                   10                  15

Asn Leu Val Pro Glu Val Asn Asn Glu Val Met Ala Leu Glu Pro Val
            20                  25                  30

Val Gly Ala Ala Ile Ala Ala Pro Val Ala Gly Gln Gln Asn Val Ile
        35                  40                  45

Asp Pro Trp Ile Arg Asn Asn Phe Val Gln Ala Pro Gly Gly Glu Phe
    50                  55                  60

Thr Val Ser Pro Arg Asn Ala Pro Gly Glu Ile Leu Trp Ser Ala Pro
65                  70                  75                  80

Leu Gly Pro Asp Leu Asn Pro Tyr Leu Ser His Leu Ala Arg Met Tyr
                85                  90                  95

Asn Gly Tyr Ala Gly Gly Phe Glu Val Gln Val Ile Leu Ala Gly Asn
            100                 105                 110

Ala Phe Thr Ala Gly Lys Val Ile Phe Ala Ala Val Pro Pro Asn Phe
        115                 120                 125

Pro Thr Glu Gly Leu Ser Pro Ser Gln Val Thr Met Phe Pro His Ile
    130                 135                 140

Val Val Asp Val Arg Gln Leu Glu Pro Val Leu Ile Pro Leu Pro Asp
145                 150                 155                 160

Val Arg Asn Asn Phe Tyr His Tyr Asn Gln Ser Asn Asp Pro Thr Ile
                165                 170                 175

Lys Leu Ile Ala Met Leu Tyr Thr Pro Leu Arg Ala Asn Asn Ala Gly
            180                 185                 190

Asp Asp Val Phe Thr Val Ser Cys Arg Val Leu Thr Arg Pro Ser Pro
        195                 200                 205

Asp Phe Asp Phe Ile Phe Leu Val Pro Pro Thr Val Glu Ser Arg Thr
    210                 215                 220

Lys Pro Phe Ser Val Pro Val Leu Thr Val Glu Glu Met Thr Asn Ser
225                 230                 235                 240

Arg Phe Pro Ile Pro Leu Glu Lys Leu Phe Thr Gly Pro Ser Ser Ala
                245                 250                 255

Phe Val Val Gln Pro Gln Asn Gly Arg Cys Thr Thr Asp Gly Val Leu
            260                 265                 270

Leu Gly Thr Thr Gln Leu Ser Pro Val Asn Ile Cys Thr Phe Arg Gly
        275                 280                 285

Asp Val Thr His Ile Thr Gly Ser Arg Asn Tyr Thr Met Asn Leu Ala
    290                 295                 300

Ser Gln Asn Trp Ser Asn Tyr Asp Pro Thr Glu Glu Thr Pro Ala Pro
305                 310                 315                 320

Leu Gly Thr Pro Asp Phe Val Gly Lys Ile Gln Gly Val Leu Thr Gln
                325                 330                 335

Thr Thr Arg Thr Asp Gly Ser Thr Arg Gly His Lys Ala Thr Val Tyr
            340                 345                 350

Thr Gly Ser Ala Asp Phe Ala Pro Lys Leu Gly Arg Val Gln Phe Glu
        355                 360                 365

Thr Asp Thr Asp Arg Asp Phe Glu Ala Asn Gln Asn Thr Lys Phe Thr
    370                 375                 380

Pro Val Gly Val Ile Gln Asp Gly Ser Thr Thr His Arg Asn Glu Pro
385                 390                 395                 400

Gln Gln Trp Val Leu Pro Ser Tyr Ser Gly Arg Asn Thr Pro Asn Val
                405                 410                 415

His Leu Ala Pro Ala Val Ala Pro Thr Phe Pro Gly Glu Gln Leu Leu
            420                 425                 430

Phe Phe Arg Ser Thr Met Pro Gly Cys Ser Gly Tyr Pro Asn Met Asp
        435                 440                 445

Leu Asp Cys Leu Leu Pro Gln Glu Trp Val Gln Tyr Phe Tyr Gln Glu
    450                 455                 460

Ala Ala Pro Ala Gln Ser Asp Val Ala Leu Leu Arg Phe Val Asn Pro
465                 470                 475                 480

Asp Thr Gly Arg Val Leu Phe Glu Cys Lys Leu His Lys Ser Gly Tyr
                485                 490                 495

Val Thr Val Ala His Thr Gly Gln His Asp Leu Val Ile Pro Pro Asn
            500                 505                 510

Gly Tyr Phe Arg Phe Asp Ser Trp Val Asn Gln Phe Tyr Thr Leu Ala
        515                 520                 525

Pro Met Gly Asn Gly Thr Gly Arg Arg Arg Ala Leu
    530                 535                 540

<210>  14
<211>  268
<212>  PRT
<213>  Norovirus

<400>  14

Met Ala Gly Ala Phe Phe Ala Gly Leu Ala Ser Asp Val Leu Gly Ser
1               5                   10                  15

Gly Leu Gly Ser Leu Ile Asn Ala Gly Ala Gly Ala Ile Asn Gln Lys
            20                  25                  30

Val Glu Phe Glu Asn Asn Arg Lys Leu Gln Gln Ala Ser Phe Gln Phe
        35                  40                  45

Ser Ser Asn Leu Gln Gln Ala Ser Phe Gln His Asp Lys Glu Met Leu
    50                  55                  60

Gln Ala Gln Ile Glu Ala Thr Lys Arg Leu Gln Gln Glu Met Met Lys
65                  70                  75                  80

Val Lys Gln Ala Met Leu Leu Glu Gly Gly Phe Ser Glu Thr Asp Ala
                85                  90                  95

Ala Arg Gly Ala Ile Asn Ala Pro Met Thr Lys Ala Leu Asp Trp Ser
            100                 105                 110

Gly Thr Arg Tyr Trp Ala Pro Asp Ala Arg Thr Thr Thr Tyr Asn Ala
        115                 120                 125

Gly Arg Phe Ser Thr Pro Gln Pro Ser Gly Ala Leu Pro Gly Arg Ala
    130                 135                 140

Asn Leu Arg Asp Ala Val Pro Ala Arg Gly Ser Ser Ser Lys Ser Ser
145                 150                 155                 160

Asn Ser Ser Thr Ala Thr Ser Val Tyr Ser Asn Gln Thr Thr Ser Thr
                165                 170                 175

Arg Leu Gly Ser Thr Ala Gly Ser Gly Thr Ser Val Ser Ser Phe Pro
            180                 185                 190

Ser Thr Ala Arg Thr Arg Ser Trp Val Glu Asp Gln Ser Arg Asn Leu
        195                 200                 205

Ser Pro Phe Met Arg Gly Ala His Asn Ile Ser Phe Val Thr Pro Pro
    210                 215                 220

Ser Ser Arg Ser Ser Ser Gln Gly Thr Val Ser Thr Val Pro Lys Glu
225                 230                 235                 240

Ile Leu Asp Ser Trp Thr Gly Ala Phe Asn Thr Arg Arg Gln Pro Leu
                245                 250                 255

Phe Ala His Ile Arg Lys Arg Gly Glu Ser Arg Ala
            260                 265

<210>  15
<211>  540
<212>  PRT
<213>  Norovirus

<400>  15

Met Lys Met Ala Ser Ser Asp Ala Asn Pro Ser Asp Gly Ser Ala Ala
1               5                   10                  15

Asn Leu Val Pro Glu Val Asn Asn Glu Val Met Ala Leu Glu Pro Val
            20                  25                  30

Val Gly Ala Ala Ile Ala Ala Pro Val Ala Gly Gln Gln Asn Val Ile
        35                  40                  45

Asp Pro Trp Ile Arg Ser Asn Phe Val Gln Ala Pro Gly Gly Glu Phe
    50                  55                  60

Thr Val Ser Pro Arg Asn Ala Pro Gly Glu Ile Leu Trp Ser Ala Pro
65                  70                  75                  80

Leu Gly Pro Asp Leu Asn Pro Tyr Leu Ser His Leu Ala Arg Met Tyr
                85                  90                  95

Asn Gly Tyr Ala Gly Gly Phe Glu Val Gln Val Ile Leu Ala Gly Asn
            100                 105                 110

Ala Phe Thr Ala Gly Lys Val Ile Phe Ala Ala Val Pro Pro Asn Phe
        115                 120                 125

Pro Thr Glu Gly Leu Ser Pro Ser Gln Val Thr Met Phe Pro His Ile
    130                 135                 140

Val Val Asp Val Arg Gln Leu Glu Pro Val Leu Ile Pro Leu Pro Asp
145                 150                 155                 160

Val Arg Asn Asn Phe Tyr His Tyr Asn Gln Ser Asn Asp Pro Thr Ile
                165                 170                 175

Lys Leu Ile Ala Met Leu Tyr Thr Pro Leu Arg Ala Asn Asn Ala Gly
            180                 185                 190

Asp Asp Val Phe Thr Val Ser Cys Arg Val Leu Thr Arg Pro Ser Pro
        195                 200                 205

Asp Phe Asp Phe Ile Phe Leu Val Pro Pro Thr Val Glu Ser Arg Thr
    210                 215                 220

Lys Pro Phe Ser Val Pro Val Leu Thr Val Glu Glu Met Thr Asn Ser
225                 230                 235                 240

Arg Phe Pro Ile Pro Leu Glu Lys Leu Phe Thr Gly Pro Ser Ser Ala
                245                 250                 255

Phe Val Val Gln Pro Gln Asn Gly Arg Cys Thr Thr Asp Gly Val Leu
            260                 265                 270

Leu Gly Thr Thr Gln Leu Ser Pro Val Asn Ile Cys Thr Phe Arg Gly
        275                 280                 285

Asp Val Thr His Ile Thr Gly Ser Arg Asn Tyr Thr Met Asn Leu Ala
    290                 295                 300

Ser Gln Asn Trp Ser Asn Tyr Asp Pro Thr Glu Glu Ile Pro Ala Pro
305                 310                 315                 320

Leu Gly Thr Pro Asp Phe Val Gly Lys Ile Gln Gly Val Leu Thr Gln
                325                 330                 335

Thr Thr Arg Thr Asp Gly Ser Thr Arg Gly His Lys Ala Thr Val Tyr
            340                 345                 350

Thr Gly Ser Ala Asn Phe Ala Pro Lys Leu Gly Arg Val Gln Phe Glu
        355                 360                 365

Thr Asp Thr Asp His Asp Phe Glu Ala Asn Gln Asn Thr Lys Phe Thr
    370                 375                 380

Pro Val Gly Val Ile Gln Asp Gly Ser Thr Thr His Arg Asn Glu Pro
385                 390                 395                 400

Gln Gln Trp Val Leu Pro Ser Tyr Ser Gly Arg Asn Thr Pro Asn Val
                405                 410                 415

His Leu Ala Pro Ala Val Ala Pro Thr Phe Pro Gly Glu Gln Leu Leu
            420                 425                 430

Phe Phe Arg Ser Thr Met Pro Gly Cys Ser Gly Tyr Pro Asn Met Asp
        435                 440                 445

Leu Asp Cys Leu Leu Pro Gln Glu Trp Val Gln Tyr Phe Tyr Gln Glu
    450                 455                 460

Ala Ala Pro Ala Gln Ser Asp Val Ala Leu Leu Arg Phe Val Asn Pro
465                 470                 475                 480

Asp Thr Gly Arg Val Leu Phe Glu Cys Lys Leu His Lys Ser Gly Tyr
                485                 490                 495

Val Thr Val Ala His Thr Gly Gln His Asp Leu Val Ile Pro Pro Asn
            500                 505                 510

Gly Tyr Phe Arg Phe Asp Ser Trp Val Asn Gln Phe Tyr Thr Leu Ala
        515                 520                 525

Pro Met Gly Asn Gly Thr Gly Arg Arg Arg Ala Leu
    530                 535                 540

<210>  16
<211>  268
<212>  PRT
<213>  Norovirus

<400>  16

Met Ala Gly Ala Phe Phe Ala Gly Leu Ala Ser Asp Val Leu Gly Ser
1               5                   10                  15

Gly Leu Gly Ser Leu Ile Asn Ala Gly Ala Gly Ala Ile Asn Gln Lys
            20                  25                  30

Val Glu Phe Glu Asn Asn Arg Lys Leu Gln Gln Ala Ser Phe Gln Phe
        35                  40                  45

Ser Ser Asn Leu Gln Gln Ala Ser Phe Gln His Asp Lys Glu Met Leu
    50                  55                  60

Gln Ala Gln Ile Glu Ala Thr Lys Arg Leu Gln Gln Glu Met Met Lys
65                  70                  75                  80

Val Lys Gln Ala Met Leu Leu Glu Gly Gly Phe Ser Glu Thr Asp Ala
                85                  90                  95

Ala Arg Gly Ala Ile Asn Ala Pro Met Thr Lys Ala Leu Asp Trp Ser
            100                 105                 110

Gly Thr Arg Tyr Trp Ala Pro Asp Ala Arg Thr Thr Thr Tyr Asn Ala
        115                 120                 125

Gly Arg Phe Ser Thr Pro Gln Pro Ser Gly Ala Leu Leu Gly Arg Ala
    130                 135                 140

Asn Leu Arg Asp Ala Val Pro Ala Arg Gly Ser Ser Ser Lys Ser Thr
145                 150                 155                 160

Asn Ser Ser Thr Ala Thr Ser Val Tyr Ser Asn Gln Thr Thr Ser Thr
                165                 170                 175

Arg Leu Gly Ser Thr Ala Gly Ser Gly Thr Ser Val Ser Ser Phe Pro
            180                 185                 190

Ser Thr Ala Arg Thr Arg Ser Trp Val Glu Asp Gln Ser Arg Asn Leu
        195                 200                 205

Ser Pro Phe Met Arg Gly Ala His Asn Ile Ser Phe Val Thr Pro Pro
    210                 215                 220

Ser Ser Arg Ser Ser Ser Gln Gly Thr Val Ser Thr Val Pro Lys Glu
225                 230                 235                 240

Ile Leu Asp Ser Trp Thr Gly Ala Phe Asn Thr Arg Arg Gln Pro Leu
                245                 250                 255

Phe Ala His Ile Arg Lys Arg Gly Glu Ser Arg Ala
            260                 265

<210>  17
<211>  68
<212>  DNA
<213>  Artificial Sequence

<220>
<223>  ATU

<400>  17
tagcctaccc tccatcattg ttataaaaaa cttaggaacc aggtccacac agccgccagc      60

ccatcaac                                                                68

<210>  18
<211>  25
<212>  DNA
<213>  Artificial Sequence

<220>
<223>  ATU

<400>  18
agcgcttaga cgtctcgcga tcgat                                          25

## Description

### Technical Field

The present invention provides immunogenic compositions, nucleic acid molecules and VLPs suitable as Norovirus vaccine candidates. In particular, suitable antigens originating from relevant Norovirus genotypes, suitable vaccine architectures and methods of efficiently producing said antigens in a suitable vector backbone are provided. Further provided are host cells for producing the biological material as well as methods for producing and/or purifying the immunogenic compositions and VLPs. Further provided is an immunogenic composition for use in a method of preventing/treating a Norovirus infection in a subject.

### Background of Invention

Human Noroviruses are the leading cause of acute gastroenteritis worldwide and are replacing rotavirus as predominant gastrointestinal pathogen in the pediatric population (Ramani et al., Curr. Opin. Gastroenterol., 2014). The virus causes acute debilitating illness characterized by vomiting and diarrhea as typical symptoms of a Norovirus infection. The infections can occur in all age groups and result in significant morbidity and mortality, particularly in the very old and the very young. Approximately 200,000 children per year die from complications of Norovirus infections worldwide according to an estimation. The transmission rate is very high, requiring a very low infectious dose of only less than 10-100 virions. The disease is mostly self-limiting within a few days. Nevertheless, the virus can cause also severe outcomes mainly in elderly, young children and immunosuppressed groups. Beyond supportive care no treatment is currently available.

Norovirus is divided into five genogroups (I-V), with GI, GII and GIV causing human infections. Each genogroup is further subdivided into genotypes based on analysis of the amino acid sequence of its major viral capsid protein VP1. The Norwalk virus, the prototype human Norovirus is classified as a GI virus.

The epidemiology of the virus is very complex with over 30 genotypes in three genogroups (GI, GII and GIV) causing infections in humans. The division into genotypes is based on the analysis of the amino acid sequences of its major viral capsid protein VP1. Most confirmed infections are associated with genotype GII.4, i.e. over 80% (WHO, Status of Vaccine Research and Development for Norovirus, prepared for WHO PD-VAC, 2014). A subdivision into serotypes as commonly done for virus is impossible due to the fact that the virus cannot be cultured *in vitro.* Therefore, it remains to be elucidated if this classification is of biological relevance. Further, the inability to culture Norovirus hampers research on pathogenesis, vaccine development, and diagnostics. Finally, epidemiology of Norovirus on a global level is highly dynamic and seasonality and predominance of certain Norovirus strains varies, also depending on the prevailing climate conditions.

Structurally, noroviruses are -38 nm icosahedral viruses with an -7.5-kb single-stranded, positive-sense RNA genome that encodes three large open reading frames (ORFs). ORF1 encodes the replicase polyprotein, while ORFs 2 and 3 encode the major and minor capsid proteins VP1 and VP2, respectively. Expression of the major capsid protein VP1 (ORF2) in baculovirus and Venezuelan equine encephalitis (VEE) virus was shown to result in formation of virus-like particles (VLPs) composed of 180 copies of the monomeric protein. The monomer is structurally divided into the shell domain (S) that forms the core of the particle and the protruding domain (P) that extends away from the core. The P domain is further subdivided into the P1 subdomain (residues 226 to 278 and 406 to 520) and the P2 subdomain (residues 279 to 405) (53). P2 represents the most exposed surface of the viral particle and determines interaction with both potential neutralizing antibody and histo-blood group antigens (HBGAs) (Lindesmith et al., Norovirus GII.4 Strain Antigenic Variation, Journal of Virology, vol. 85, no. 1, 2011).

Another fact complicating vaccine development is that the virus rapidly undergoes mutations, antigenic drift and recombination events. Antigenic drift is a typical feature of noroviruses belonging to the GII.4 genotype. In the early 2000s, the association of host genetics and the risk of Norovirus infection was identified. Pathogenesis of this virus is mainly driven by its binding capacity to human histo-blood group antigens (HBGA). These glycans are cell attachment factors for Noroviruses and are present on epithelial cell surfaces (mainly the gut and respiratory tract). Different variants of HBGAs exist. Human challenge studies have demonstrated that only individuals expressing certain HBGAs are susceptible to infection with the prototype virus Norwalk virus belonging to GI. Interestingly, Norovirus GII.4 binds a variety of HBGAs, possibly explaining its predominance in causing Norovirus infections and outbreaks.

Noroviruses are non-enveloped single-stranded, positive-sense RNA viruses and belong to the Family of *Caliciviridae.* The capsid is formed by two proteins, the major capsid protein VP1 and the minor capsid protein VP2. The outer capsid shell consists of the major capsid protein VP1. This protein has the ability to self-assemble into virus-like particles (VLPs), which morphologically and antigenically resemble the native virus. Therefore, for the specific case of Norovirus, VLPs can be highly immunogenic and seem to represent a promising candidate for vaccine development provided that a technique can be established allowing that functional recombinant VLPs can be produced and further provided that said VLPs will present the correct antigens able to elicit a desired immune response. No additional protein is needed. Nevertheless, the minor capsid protein if expressed together with VP1 can also be incorporated into the virus-like particles. It stays within the capsid and is thus not target of the immune response. Despite evidence that at least GI Norwalk virus minor structural protein VP2 is present in infectious virions, the available crystallographic and electron cryomicroscopy structures of NV have not revealed the location of VP2. Later, it was determined that VP1 associates with VP2 at the interior surface of the capsid, specifically with the shell (S) domain of VP1. Studies have shown that VP2 may increase the stability of the VLPs, doubling the half-life, at least *in vitro,* which was demonstrated for VP2 of the GI Norwalk Norovirus in an analytical setting (Vongpunsawad et al., Norwalk Virus Minor Capsid Protein VP2 Associates within the VP1 Shell Domain, J.Virol., 2013, 87:94818-4825). Notably, VP2 is not essential for Norovirus VLP formation, which may be a reason for the fact that so far no efforts have been made to design a vaccine based on the additional protein VP2.

Currently, there are no licensed vaccines available. Given the high mortality and morbidity of the virus, the need for a prophylactic Norovirus vaccine is very high. The facts that the virus cannot be grown in cell culture as well as that no appropriate preclinical model system exist to explore pathogenesis and vaccine efficacy hamper vaccine development. A few vaccine candidates are currently in development, mainly based on virus-like particles. Virus-like particles represent the best way to move forward in the vaccine development as they are highly immunogenic and easy to produce due to the self-assemble function of the VP1 protein. Most of these vaccine candidates are in pre-clinical development, except one. Vaccine feasibility was recently demonstrated with this candidate by showing protection particularly against severe disease in two human challenge studies. This most advanced vaccine candidate is a bivalent virus-like particle vaccine against genotypes GI.1/GII.4 based on a consensus sequence for GII.4. These particles are formed solely by the expression of the major capsid protein VP1. In all reported activities in Norovirus vaccine development, however, no VP2 was expressed.

The most advanced vaccine candidate for Norovirus currently in development was developed by Takeda Vaccines and is a recombinant VLP vaccine achieved by the expression of the major capsid protein VP1 solely. The vaccine is based on the VP1 sequence of GI.1 Norwalk virus and a VP1 consensus sequence of three GII.4 viruses. A bivalent vaccine candidate. The VLPs are expressed in the baculovirus system. Purified VLPs resemble the vaccine candidate. Thus, the vaccine can be classified as a recombinant protein vaccine. In order to make such protein vaccines potent in human beings, the protein has to be formulated with additional adjuvants like Alum and MPL, increasing also the reactogenicity of the vaccine candidate. An alternative VLP candidate is being developed by Arizona State University using the same construct as the Takeda VLP, but it is produced in a plant-based vector. A Norovirus VLP-rotavirus protein combination vaccine candidate is currently in preclinical development, and clinical trials may begin in the near term (see WHO, 2014, *supra*)*.* The vaccine is being co-developed by the University of Tampere (Finland) and UMN Pharma (Japan). Finally, the University of Cincinnati has conducted preclinical immunological studies on a Norovirus P particle construct. This vaccine candidate is derived from the protruding (P) domain of the Norovirus VP1 capsid protein (see WHO, 2014, *supra*)*.*

Regarding Norovirus structure, most of the work done has been established in Norwalk virus, in particular using VP1. Depending on whether full-length or successively truncated recombinant forms of VP1 were used, structurally different Norovirus complexes were observed, i.e. (A) Virus-like particles (180-mer: ∼37 nm); (B) S particles (180-mer: ∼27 nm); (C) P particles (24-mer: ∼20 nm), i.e. those particles only containing the protruding domains of the capsid; (D) small P particles (12-mer: ∼14 nm); (E) P dimers (∼6 nm), or (F) linear structures of norovirus VP1 comprising truncated S (1-217) and P (226-530) domains that are linked by a short hinge. Numbers are based on Norwalk virus VP1 (Tan et al., Terminal modifications of norovirus P domain resulted in a new type of subviral particles, the small P particles, Virology. 2011 Feb 20; 410(2):345-52).

From a lesser-developed country perspective, there are unique issues to consider for vaccine development and feasibility. Dose number and schedule are important, as well as possibly different circulating strains compared to those found in developed countries. Furthermore, the current vaccine approach for an indication in adults is based on an intramuscular injection of the vaccine with an effective immune response that is thought in part to rely on the boosting of memory from previous natural infection. As a Norovirus vaccine would likely be targeted to younger children, the effectiveness of the vaccine in a naïve infant may be less, and an alternative mucosal priming-parenteral boost hypothetically may be necessary, but also complicates the development of a vaccine for use in a resource-limited setting (see WHO, 2014, *supra*)*.*

Taking the present status quo of Norovirus vaccine development into account, it was thus an object of the present invention to establish a Norovirus vaccine candidate based on the expression of two proteins, VP1 and VP2, to confer broad protection against the most common and severe Norovirus infection genotypes. It should be figured out whether co-expression of both proteins in a suitable vector system could induce a better immunogenicity in a target population.

In particular, it was an object to establish the potential of VP2-based vaccines so far not being studied in detail as protective Norovirus antigenic sequences. Based on this co-expression strategy, a platform to rapidly develop vaccine candidates should be established regarding the implications of antigenic diversity and drift in the context of Norovirus to be able to provide efficacious vaccine compositions readily adaptable to the norovirus antigenic variation. Furthermore, stable and immunogenic VP1-based VLP vaccines should be created. This strategy should also address the high alternating predominance of different Norovirus GII.4 and also non-GII.4 genotypes in view of the fact that Norovirus variations on a global level demands the design of different vaccines following the same basic construction principle to obtain a preventive measure at short notice. To this end, the inventors also aimed and identifying consensus sequences of VP1 and VP2 Norovirus sequences to establish sequences with a high or low degree of variation over several Norovirus genogroups and genotypes to establish a vaccine with broad coverage against different Noroviruses. Thus, a vaccine candidate based on the co-expression of VP1 and VP2 should be established to provide more stable VLPs, in turn increasing the immunogenicity of the vaccine candidate. The strategy of co-expression was also designed to be used for the rapid development of multivalent and seasonal Norovirus vaccine candidates.

Furthermore, it was an object to provide a safe and efficient manufacturing technique avoiding the need of adjuvants and further allowing the provision of a vaccine which can be administered to children. Finally, a reliable expression vector system should be provided that allows high yield vaccine production, and thus could represent a manufacturing advantage through relatively low cost of goods whilst simultaneously being safe and efficient in antigen presentation. In addition, the proteins expressed by a self-replicating and therefore self-adjuvanting vector system itself should be used to increase the potency of the vaccine candidate.

### Summary of the Invention

The above objects have been achieved by providing, in a first aspect, an immunogenic composition comprising at least one recombinant Norovirus antigen, and/or a sequence encoding the same, wherein the at least one recombinant Norovirus antigen is encoded by at least one nucleic acid sequence encoding at least one Norovirus VP1 sequence and at least one Norovirus VP2 sequence, wherein the at least one nucleic acid sequence is operably linked to a vector backbone, wherein the immunogenic composition optionally comprises at least one pharmaceutically acceptable carrier and/or excipient, optionally wherein the Norovirus VP1 sequence and/or the Norovirus VP2 sequence is independently derived from a Norovirus of the GII genogroup, preferably from a Norovirus having a GII.4 genotype, preferably wherein the Norovirus VP1 sequence and the Norovirus VP2 sequence are encoded by the same nucleic acid sequence and are optionally separated by at least one linker sequence.

In one embodiment, the vector backbone is selected from the group consisting of a measles virus backbone, a lentivirus backbone, a Vesicular stomatitis virus (VSV) backbone, or a vaccinia Ankara backbone, preferably wherein the vector backbone is from a recombinant live-attenuated measles virus strain, the measles virus strain preferably being selected from the group consisting of a Schwarz strain, a Zagreb strain, an AIK-C strain and a Moraten strain, and/or the VP1 and/or the VP2 sequence are independently selected from a Norovirus having a GII.4 genotype, preferably from a sequence from a Norovirus Hu/GII.4/Sydney/NSW0514/2012/AU, a Norovirus Hu/GII.4/Orange/NSW001P/2008/AU, a Norovirus Hu/GII.4/NIHIC17.5/2012/USA, a Norovirus Hu/GII.4/GII4-NG1242/2011/JP, or a Norovirus Hu/GII.4/Hiroshima4/2012/JP, wherein the VP1 and the VP2 sequence are derived from the same Norovirus, or wherein the VP1 and the VP2 sequence are derived from a different Norovirus, optionally wherein the VP1 and the VP2 sequence are modified in comparison to the reference sequence.

In another embodiment, the at least one VP1 and/or the at least one VP2 sequence represent(s) a conserved consensus sequence, and/or the immunogenic composition is a bivalent or a multivalent immunogenic composition, wherein the at least one nucleic acid sequence encoding the at least one Norovirus VP1 sequence and/or the at least one nucleic acid sequence encoding at least one Norovirus VP2 sequence are selected from at least two different Norovirus genogroups, preferably a GII and a GI genogroups, and/or wherein the at least one nucleic acid sequence encoding the at least one Norovirus VP1 sequence and/or the at least one nucleic acid sequence encoding at least one Norovirus VP2 sequence are selected from different Norovirus GII genotypes, preferably a GII.4 and a GII.17 or GII.2 genotype.

In a second aspect, there is provided a nucleic acid molecule comprising at least a first and at least a second nucleic acid sequence, wherein the first nucleic acid sequence encodes at least one VP1 protein of a Norovirus or a functional fragment thereof, and wherein the second nucleic acid sequence encodes at least one VP2 protein of a Norovirus or a functional fragment thereof, optionally wherein the at least one first and the at least one second nucleic acid sequence is/are separated by at least one linker, optionally wherein the nucleic acid molecule comprises a sequence selected from any one of SEQ ID NOs:3 to 8, or a sequence having at least 80%, 81%, 82%, 83%, 84%, 85%, 86%, 87%, 88%, 89%, 90%, 91%, 92%, 93%, 94%, 95%, 96%, 97%, 98% or 99% sequence identity thereto, or wherein the first nucleic acid sequence and/or the second nucleic acid sequence represent(s) a conserved consensus sequence.

In a third aspect, there is provided a recombinant virus-like particle (VLP) comprising at least one recombinant Norovirus VP1 antigen and at least one recombinant Norovirus VP2 antigen.

In yet another aspect, there is provided a host cell comprising at least one nucleic acid sequence encoding at least one Norovirus VP1 sequence and at least one Norovirus VP2 sequence, or comprising at least one nucleic acid molecule according to the second aspect, or comprising at least one nucleic acid sequence encoding a recombinant virus-like particle (VLP) according the above third aspect, preferably wherein the at least one host cell is selected from the group consisting of Vero cells, chicken embryo fibroblast cells, HEK293 cells, HeLa cells, fetal rhesus lung cells or MRC5 cells.

In a further aspect, there is provided a method for producing an immunogenic composition according to the first aspect, or for producing a recombinant virus-like particle (VLP) according to the third aspect, the method comprising the following steps: (i) inserting at least one nucleic acid sequence as defined according to the first aspect, or inserting at least one nucleic acid molecule as defined according to the second aspect above into a vector backbone as defined in the turn of the first aspect to operably link the nucleic acid sequence or molecule and the vector backbone to obtain a recombinant chimeric virus sequence; (ii) infecting at least one host cell with at least one recombinant chimeric virus sequence obtained in step (i) to obtain a virus sample; (iii) optionally: clarifying the virus sample of step (ii); (iv) optionally: purifying the clarified virus sample of step (iii); (v) optionally: formulating the at least one recombinant chimeric virus with at least one pharmaceutically and/or veterinary acceptable carrier and/or excipient; (vi) obtaining an immunogenic composition comprising infectious virus particles and virus-like particles (VLPs); (vii) optionally: further purifying the immunogenic composition and thus obtaining, separated from each other, recombinant virus-like particles (VLPs) and/or infectious virus particles.

In a further aspect, there is provided an immunogenic composition according to the first aspect, or a recombinant virus-like particle (VLP) according to the third aspect for use in a method of preventing or treating a Norovirus virus disease in a subject.

The present invention will now be described in detail based on the detailed description, including non-limiting Examples, the attached drawings and the sequences as provided with the sequence listing.

### Brief Description of Drawings

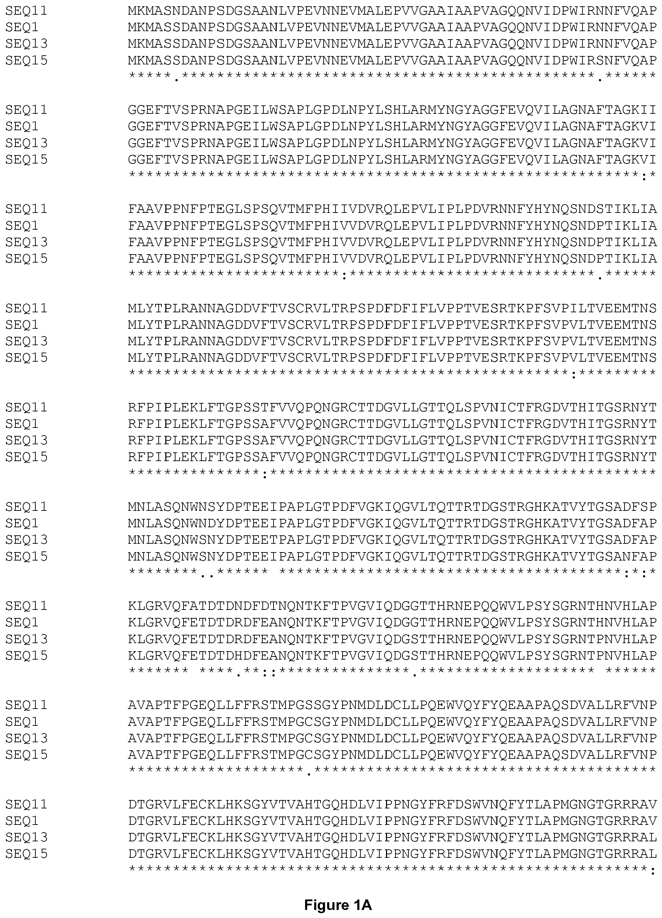

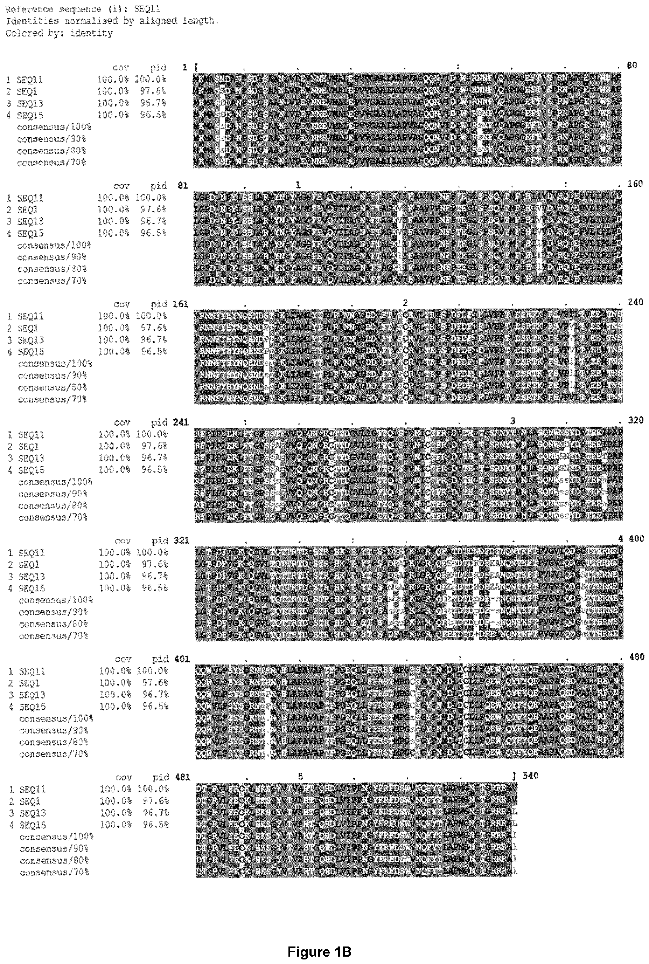

**Figure 1** (**Figures 1A** to **B**) shows in **Figure 1A** an alignment of VP1 amino acid sequences of SEQ ID Nos:1, 11, 13 and 15 of their entire length. The alignment was created using the publicly available tool MUSCLE (**MU**ltiple **S**equence **C**omparison by **L**og-**E**xpectation; https://www.ebi.ac.uk/Tools/msa/muscle/) using default parameters. Asterisks (*) indicate positions which have a single, fully conserved residue. Colons (:) indicate conservation between groups of strongly similar properties and thus highly conserved residues. Periods (.) indicate conservation between groups of weakly similar properties. The raw data were then immediately submitted to the Tool MView (version 1.63), which was also used according to default parameters (https://www.ebi.ac.uk/Tools/msa/mview/; Brown, N.P., Leroy C., Sander C. (1998). MView: A Web compatible database search or multiple alignment viewer. Bioinformatics. 14 (4):380-381). The result is shown in **Figure 1B****.** The upper four rows represent the sequence of the corresponding SEQ ID NOs. consensus 100%, 90%, 80% and 70% then show the default calculated consensus mechanism displaying consensus lines calculated at four levels of identity (100%, 90%, 80%, 70%). The default protein alignment is defined by physicochemical (positive, aromatic etc.) and sterical (small, tiny etc.) properties of the respective amino acid sequences (symbols: classical amino acid one-letter codes, further symbols are: alcohol => o { S, T }; aliphatic => I {I, L, V}; aromatic => a { F, H, W, Y}; charged => c { D, E, H, K, R }; hydrophobic => h {A, C, F, G, H, I, K, L, M, R, T, V, W, Y }; negative => - { D, E }; polar => p { C, D, E, H, K, N, Q, R, S, T}; positive => + { H, K, R }; small => s {A, C, D, G, N, P, S, T, V }; tiny => u {A, G, S }; turnlike => t {A, C, D, E, G, H, K, N, Q, R, S, T}; stop => * {*}.

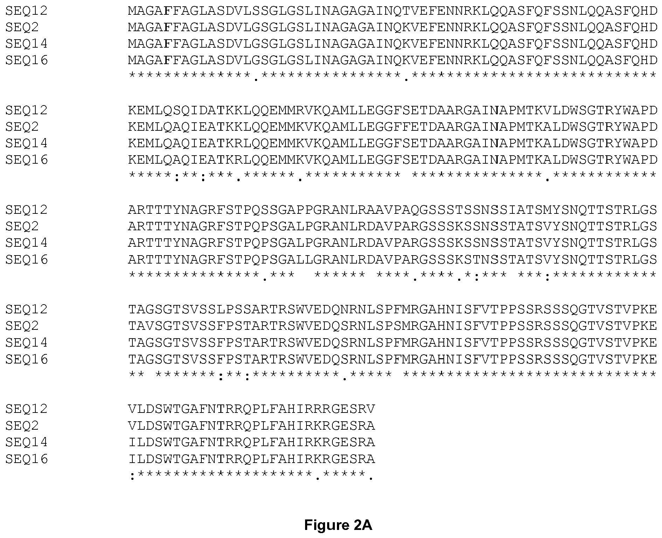

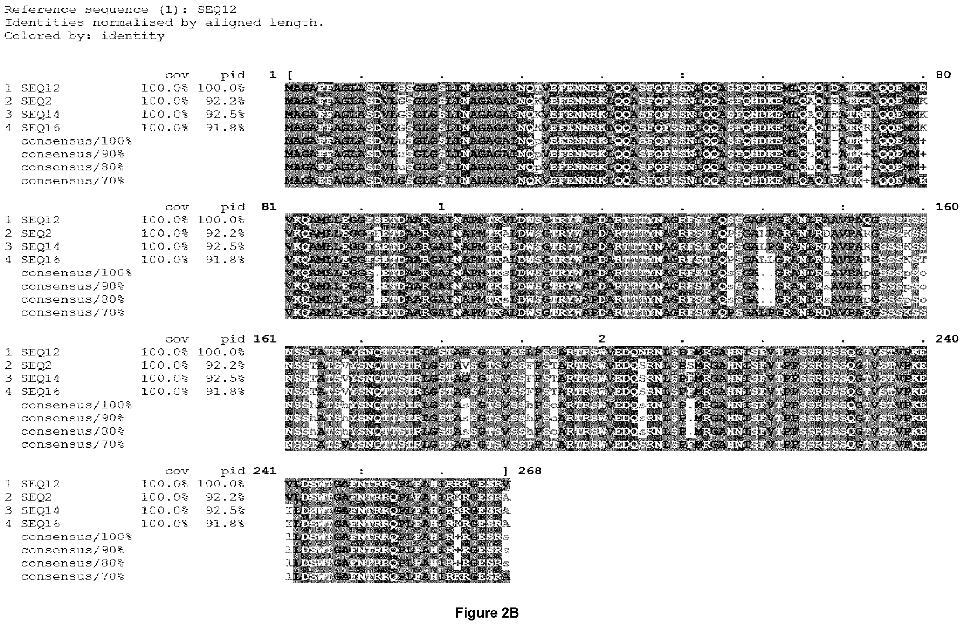

**Figure 2** (**Figures 2A** and **B**) shows in **Figure 2A** an alignment of VP2 amino acid sequences of SEQ ID Nos:2, 12, 14 and 16 of their entire length. The alignment was created using the publicly available tool MUSCLE using default parameters. **Figure 2B** shows a MView 1.63 consensus pattern definition (default parameters used) for the given VP2 sequences as detailed for Figure 1 above. Based on at least two, preferably at least three given input sequences (DNA or protein) the skilled person can thus easily identify a consensus pattern as described in and disclosed with Figures **1** and **2****.**

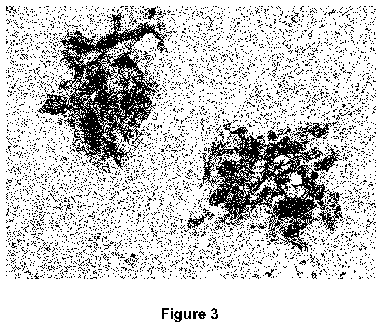

**Figure 3** shows Norovirus VLP expression in MV-Noro infected cells. Cells were infected with a MOI of 0.01 and three days post infection, the supernatant was removed, the cells were washed and fixed. Subsequently, the fixed cells were incubated with a Norovirus GII.4 antibody recognizing Norovirus virus-like particle (VLP) from GII.4 VLP 1987-2009 and 2012. Bound antibodies were visualized by incubation with a second peroxidase labelled antibody followed by a substrate reaction. Pictures were taken with a Leica DMIL LED using a 10x objective at a 100x magnification. See also **Example 6.**

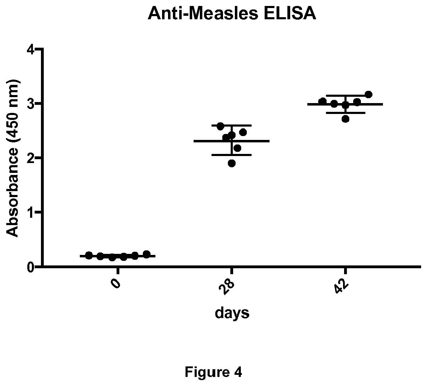

**Figure 4** shows the ELISA measurement of measles-specific antibodies in cotton rats developed upon immunization with a dose of 1x10e5 TCID50 of MV-Norovirus (see SEQ ID NO:10) as further detailed in **Example 7.**

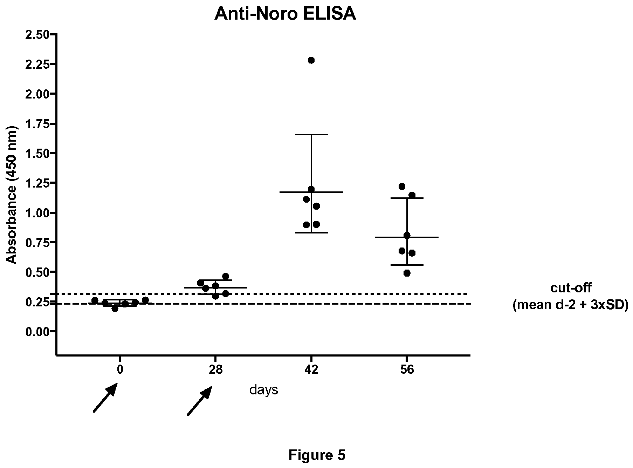

**Figure 5** shows the ELISA measurement of Norovirus-specific antibodies in cotton rats developed upon immunization with a dose of 1x10e5 TCID50 of MV-Norovirus (see SEQ ID NO:10). as further detailed in **Example 7.**

### Definitions

An "adjuvant" in the field of immunology and as used herein refers to a substance or composition enhancing antigenicity of another substance. This is achieved by specifically stimulating and balancing mainly the Th1, Th2, Th17 and regulatory T (Treg) subsets of T-cells and thus their effector molecules. Usually, immunogenic compositions based on live-attenuated or killed viruses are highly immunogenic *per se* and thus there might not be the need for an additional adjuvant, whereas an additional adjuvant might still be favourable to balance the provoked immune response. Th1 cells secrete IFN-y, which activates macrophages and thus induces the production of opsonizing antibodies by B cells. The Th1 response therefore leads mainly to a cell-mediated immunity. Th2 cells mainly secrete cytokines, including IL-4, which induces B cells to make neutralizing antibodies. Th2 cells generally induce a humoral (antibody) response critical in the defense against extracellular pathogens (helminths, extracellular microbes and toxins). Treg cells secrete IL-10 and transforming growth factor (TGF)-β, which modulate helper T-cell activity and suppress some of their functions, inducing tolerance to antigens. Th17 cells as a unique CD4⁺ T-cell subset produce IL-17, IL-17F, IL-6, IL-22 and TNF-α, which, in turn, act on fibroblasts, macrophages, and endothelial and epithelial cells to elicit inflammatory mediator and chemokine release responses.

The term "antigen" as used herein and as commonly used in the field of immunology refers to an "antibody generating" molecule, i.e. a substance, which can elicit an adaptive immune response. An antigen is thus a molecule binding to an antigen-specific receptor, either a T-cell or a B-cell receptor. An antigen is usually a (poly)peptide, but it can also be a polysaccharide or a lipid, possibly combined with a protein or polysaccharide carrier molecule. For the purpose of the various aspects and embodiments of the present invention, the antigen is a (poly)peptide, i.e. an amino acid sequence. In the case of binding to a T-cell receptor, the antigen is presented to the respective T-cell receptor via an antigen-presenting cell as an antigenic peptide bound to a histocompatibility molecule on the surface of the antigen presenting cell, wherein the antigenic peptide has been processed in advance by the antigen presenting cell. Antigen presentation by professional antigen-presenting cells (APC) is the first step towards the initiation of an adaptive immune response carried out by naïve T lymphocytes. Thus, an "antigen" as used herein refers to a molecule, such as a protein or a polypeptide, comprising one or more epitopes that will stimulate a host's immune system to make a humoral and/or cellular antigen-specific response. As such, an antigen can either be located on the surface exposed portion of a recombinant infectious virus or an VLP. An antigen can also be made accessible to the immune system of a subject after processing of a recombinant infectious virus or a VLP or, for certain viruses, another recombinant subviral particle (RSP) and can then be loaded onto a MHC molecule to be presented to a T cell to elicit an immune response, for example.

The terms "attenuation" or "attenuated" as used herein in connection with a virus strain or a material derived therefrom refers to a virus weakened under laboratory conditions which is less vigorous than the respective wild-type virus. An attenuated virus may be used to make a vaccine that is capable of stimulating an immune response and creating immunity, but not of causing illness associated with the wild-type strain.

The term "cDNA" stands for a complementary DNA and refers to a nucleic acid sequence/molecule obtained by reverse transcription from an RNA molecule. As it is a standard method for the person skilled in the art to obtain cDNAs from a given sequence and to further use this cDNA or to clone said cDNA into a vector, preferably a plasmid vector, of interest.

The terms "derived", "derivative", "derived from", "descendant" or "progenitor" as used herein in the context of either a host cell, a cell population or an infectious virus particle according to the present application relates to the descendants of such a host cell or infectious virus particle which results from natural reproductive propagation including sexual and asexual propagation and, in the context of virus nucleic acids, the propagation of the virus genetic material by the host cell machinery. It is well known to the person having skill in the art that said propagation can lead to the introduction of mutations into the genome of an organism resulting from natural phenomena which results in a descendant or progeny, which is genomically different to the parental host cell, however, still belongs to the same genus/species and possesses the same characteristics as the parental host cell. In the context of a nucleic acid sequence or any other biomolecule being "derived from" a certain sequence this term implies that the respective nucleic acid sequence is based on the reference sequence as template, i.e. originating from said sequence, whereas the reference sequence can comprise more sequences, e.g. the whole genome or a full polyprotein encoding sequence, of a virus, whereas the sequence "derived from" the native sequence may only comprise one isolated gene or function fragment thereof, or a coherent fragment thereof. The skilled person can thus easily define a sequence "derived from" a reference sequence in case the reference sequence is known, defined or available, which will, by sequence alignment on DNA or amino acid level, have a high identity to the respective reference sequence and which will have coherent stretches of DNA/amino acids in common with the respective reference sequence (>75% query identity provided that the derived sequence is the query and the reference sequence represents the subject during a sequence alignment). The skilled person can thus clone the respective sequences based on the disclosure provided herein by means of polymerase chain reactions and the like into a suitable vector system of interest, or use a sequence as vector scaffold. The term "derived" is thus not to be understood to define an arbitrary sequence, but a sequence corresponding to a reference sequence it is derived from, whereas certain differences, e.g. certain mutations naturally occurring during replication of a recombinant construct within a host cell, cannot be excluded and are thus comprised by the term "derived from". Notably, in certain instances, e.g. related to an "antigen" according to the present invention, the term "derived (from)" specifies the nucleic acid sequence the antigen (the antigen being an amino acid sequence) originates from. The skilled person is well aware of the fact that this nucleic acid sequence according to the present invention will then be transcribed and translated *in vivo* and will be either presented to an immune cell, or it will be further digested and/or processed within a host cell. Therefore, it is more precise to define an antigen in a technical sense based on the corresponding nucleic acid sequence encoding said antigen. In the context of a virus, derivative or descendant or progenitor can thus naturally possess one or more mutations. Such derivatives or descendants resulting from natural phenomena during reproduction or propagation are thus comprised by the term host cell or cell population according to the present disclosure and the skilled person can easily define, by means of molecular biology, microscopy or the like, that the derivative or descendant is indeed derived from a parental host cell of the same genus. These terms, therefore, do not refer to any arbitrary derivative, descendant or progenitor, but rather to a derivative, or descendant or progenitor phylogenetically associated with, i.e. based on, a parent cell or virus or a molecule thereof, whereas this relationship between the derivative, descendant or progenitor and the parent or reference sequence is clearly inferable by a person skilled in the art.

An "epitope" as used herein can be a conformational epitope or a linear epitope. A conformational epitope is a sequence of sub-units, usually amino acids, composing an antigen that comes in direct contact with a receptor of the immune system. An antigen is any substance that the immune system can recognize as foreign. Antigens are usually proteins that are too large to bind as a whole to any receptor so only specific segments, that form the antigen, bind with a specific receptor, such segments usually being called "epitopes". Likewise, it is only paratope of the respective recognition receptor that comes in contact with the epitope. Whenever a receptor interacts with an undigested antigen, the surface amino acids that come in contact may not be continuous with each other if the protein is unwound. Such discontinuous amino acids that come together in three-dimensional conformation and interact with the receptor's paratope are called conformational epitopes. A linear epitope linear or a sequential epitope, in contrast, is an epitope that is recognized by the corresponding antigen receptor by its linear sequence of amino acids, or primary structure.

The terms "fragment" or "functional fragment" as used herein in the context of amino acid/protein, or nucleic acid sequences, including sequences of viruses and vectors and scaffold based on said viruses, refers to a at least one coherent fragment, which is comprised by a whole, the reference sequence. The terms thus mean a portion or fragment of a whole capable of performing, in whole or in part, a function of the whole. For example, a biologically functional portion of a molecule means a portion of the molecule that performs a biological function of the whole or intact molecule, i.e. a nucleic acid sequence or an amino acid sequence, preferably an antigen sequence, according to the present invention. For example, a functional portion of a nucleic acid sequence or the encoded protein is a fragment or portion of the specified nucleic acid sequence or the encoded protein that comprises at least one coherent part of the native or wild-type sequence. Functional fragments are thus suitable for vaccination purposes to focus on a sub-fragment of a whole protein to specifically elect those parts of immunogenic interest. As such, a "functional fragment" *per se* may have 100% sequence identity to the respective stretch of the sequence it is derived from (the reference sequence) over a given length of alignment. Still, a single nucleotide or amino acid may not be unambiguously attributable to a parent structure. In terms of absolute sequence identity, a "functional fragment" according to the present invention will thus not only be 100% identical to a given length over an alignment with the parent structure, but the "functional fragment" will have at least 15, at least 20, at least 25, at least 30, at least 35 or at least 40 nucleic acid positions in common with the respective parent structure (on DNA and/or amino acid level) it is derived from. In case that the parent sequence will comprise at least one mutation, the "functional fragment" can also comprise said mutation(s), or the "functional fragment" can comprise at least one additional mutation to optimize its properties (e.g., expression rate, folding characteristics, presentation to immune cells, immunogenicity *in vitro* and *in vivo*) desirable according to the present disclosure. The fragment will, however, usually be shorter than the sequence it is derived from.

The term "host cell" or "host cell population" as used herein refers to at least one but preferably more than one host cell(s). The term host cell comprises non-recombinant cells, i.e. cells that were not immortalized or transformed or manipulated in a purposive manner. Including also primary cells. To be suitable for certain aspects of the present invention, the host cell(s) of the cell population must be able to support the measles virus replication cycle, i.e. the cell(s) must be susceptible to measles virus or recombinant measles virus infection and the cell(s) must suitable for the subsequent propagation or replication cycle, including replication, translation encapsidation of the RNA of the virus and budding from the host cell to be released as virus particle. Several eukaryotic host cells are fulfilling this purpose are cited herein or known to the skilled person.

An "immunogenic composition" as used herein refers to a composition which is able to induce any immune response in a subject or a cellular system *in vitro.* A vaccine or vaccine composition perse is thus also comprised by the term immunogenic composition. However, it is well known to the skilled person that for being suitable to administration to an animal, an immunogenic composition can additionally comprise suitable pharmaceutically and/or veterinary acceptable carriers. The term "vaccine composition" as used herein refers to composition, preferably based on a virus particle and/or a VLP, which is able to induce a protective immune response in a subject.

As used herein, the term "immune response" refers to an innate or adaptive immune response, e.g. the T cell response or to the increased serum levels of antibodies as resulting from exposure to an antigen due to a B-cell response, or presence of neutralizing antibodies to an antigen, or it refers to an innate immune response, e.g. mediated by the complement system and cells of the innate immune system like macrophages or dendritic cells. It also refers to an allergic response, *inter alia* including mast-cell, eosinophil or NK-cell action, T-cells, B-cell secreted antibodies. The term immune response is to be understood as including a humoral response and/or a cellular response and/or an inflammatory response.

The term "measles virus" (MV) or "measles virus particle" as used herein, is to be understood as referring to any particle comprising or derived from MV including native (wild-type) or recombinant MV, optionally further comprising artificial elements or mutations, preferably purposive mutations, either in live, attenuated, inactivated or killed form. Also covered by said term is a virion and a virus-like particle derived from, and thus comprising structural elements of, MV. The term "measles virus backbone" is used interchangeably with the term "measles virus scaffold" herein and (MV being a negative-sense single-stranded RNA virus of the Family *Paramyxoviridae*) refers to the antigenomic (+) RNA strand of a measles virus, usually provided as cDNA, which may encode an infectious MV, but which may also be used as expression vector or expression construct for sequences encoding non-MV antigens or sequences.

The term "modified" in the context of a nucleic acid or amino acid sequence comprises any recombinant, chemical or enzymatic modification of the protein, peptide, polypeptide and enzyme or of the nucleic acid sequence encoding the same.

A "monovalent" vaccine is meant to refer to a vaccine designed to immunize against a single antigen(s) or single microorganism (including viruses), whereas "polyvalent" or "multivalent" vaccines aim to immunize against several strains (or genotypes) of the same microorganism (including viruses), or against several microorganisms (including viruses). A "bivalent" as used herein refers to a vaccine aiming at immunizing against two different strains (or genotypes) of the same microorganism (including viruses), or against two different microorganisms (including viruses).

As used herein, the term "pharmaceutically acceptable" refers to those compounds, materials, compositions, and/or dosage forms which are, within the scope of medical judgment or within the definition of any regulatory medical authority, suitable for contact with the cells, tissues, or components of a subject, i.e. human beings and animals, including contact with malignant cells or tissues of a subject, without excessive toxicity, irritation, allergic response, or other complications or side-effects commensurate with a reasonable benefit/risk ratio for a subject/patient.

The term "protection" or "protective immunity" or "protective immune response" as used herein refers to the ability of a substance to induce the production of serum antibodies (adaptive humoral response) and/or a cellular response during immunization to protect (partially or totally) a subject having been treated with the substance against a virus of interest during a subsequent infection or vaccination. Thus, an animal showing a protective immune response will show limited growth and spread if infected with the respective naturally occurring virus afterwards. Also an induced innate immune response mediated by immune cells of the innate immune system like macrophages or dendritic cells, can contribute to the protective effect of a substance, in case the substance stimulates said innate immune response, which can in turn contribute to enhance an adaptive immune response.

The term "reactogenicity" refers to the property of a vaccine of being able to produce common, "expected" adverse reactions, especially excessive immunological responses and associated signs and symptoms like fever, sore arm at injection site, etc.

The terms "recombinant", "genetically modified" or "genetical engineering"/"genetically engineered" as used herein refer to any nucleic acid molecule and/or an amino acid molecule or a host cell implying a targeted and purposive manipulation and/or modification achieved by means of molecular biology or protein engineering, e.g. by introducing a heterologous sequence into another host cell, by modifying a naturally occurring nucleic acid sequences and the like. Further modifications include, but are not limited to, one or more point mutation(s), one or more point mutation(s), e.g. for targeted protein engineering or for codon optimization, deletion(s), and one or more insertion(s) of at least one nucleic acid or amino acid molecule, modification of an amino acid sequence, or a combination thereof. The terms can also imply a sequence, which *per se* occurs in nature, but has been purposively treated by means of molecular biology isolated from its natural environment *in vitro* so that the sequence represents an artificial sequence.

The term "regulatory sequence" as used herein refers to a nucleic acid sequence which can directly influence, or which can indirectly be influenced (e.g. by binding to a transcription factor), to effect the transcription and/or translation of a target nucleic acid sequence of interest. The term thus refers to promoter and terminator sequences or to polyadenylation signals, a Kozak sequence and the like.

"Subject", as used herein, may mean either a human or non-human animal. The term includes, but is not limited to, mammals (e.g., humans, other primates, pigs, rodents (e.g., mice and rats or hamsters), rabbits, guinea pigs, cows, horses, cats, dogs, sheep, and goats). In an embodiment, the subject is a human being.

"Treat", "treating" and "treatment", as used herein, mean the treatment of a disease in a mammal, e.g. in a human, including (a) inhibiting the disease, i.e. arresting its development; (b) relieving the disease, i.e. causing regression of the disease state; and (c) curing the disease. The terms "prevent" or "preventing" imply that a prophylactic treatment is provided before the onset of the disease or before the onset of the symptoms associated with a disease to be prevented.

The term "vector", "vector backbone", "(vector) scaffold" or "plasmid vector"or "expression vector" as used herein defines a system comprising at least one vector suitable for transformation, transfection or transduction of a host cell. The vector is a suitable cargo for cloning to insert sequences of interests, e.g. antigenic sequences, which have to be positioned at the appropriate cloning sites. A vector *per se* thus denotes a cargo for the delivery of a biomolecule into a host cell of interest, wherein the biomolecule includes a nucleic acid molecule, including DNA, RNA and cDNA, or, in the case of a transfection system as vector, an amino acid molecule, or a combination thereof. A preferred vector according to the present invention is a plasmid or expression vector, which can be produced in a host cell. An expression vector can comprise one vector encoding at least one target molecule, preferably a nucleic acid molecule, to be introduced into a host cell. A vector of the vector system can also comprise more than one target molecules to be introduced. Alternatively, the vector system can be built from several individual vectors carrying at least one target molecule to be introduced. An expression vector additionally comprises all elements necessary for driving transcription and/or translation of a sequence of interest in a host cell, the expression vector is designed for. These elements comprise, *inter alia,* regulatory elements, which are involved in the regulation of transcription, including promoters and the like functional in the host cell of interest. Furthermore, an expression vector comprises an origin of replication and optionally depending on the type of vector and the intended use a selectable marker gene, a multiple cloning site, a tag to be attached to a sequence of interest, a chromosomal integration cassette and the like. The choice and possible modification of a suitable expression vector for use with a respective host cell and sequence of interest to be inserted into the expression vector is well within the capabilities of the person skilled in the art.

A "viral particle" or "virus particle" or "recombinant and/or infectious virus particle" as used herein refers to a single particle derived from a viral nucleic acid, which is located outside a cell. The viral particle thus represents the mature and infectious form of a virus. As the viral particle contains genetic information, it is able to replicate and/or it can be propagated in a susceptible host cell. Depending on the complexity of a virus, the viral particle comprises nucleic acid and polypeptide sequences and, optionally lipids, preferably in the form of a lipid membrane derived from the host cell. The virus particle may encode a sequence which can form a virus-like particle.

A "virion" as used herein refers to a single particle derived from a nucleic acid encoding a virus or a viral particle, which is located outside a cell containing nucleic acids and thus being able to replicate or to be transcribed in a suitable host cell.

A "virus-like particle" or "VLP" as used herein refers to at least one virus particle, which does not contain any nucleic acid. VLPs or VLPs usually comprise at least one viral structural protein from a virus they are derived from and optionally lipid constituents. As such, they can be used to for vaccination or for inducing an immunogenic reaction in a subject, due to the absence of nucleic acids they will, however, not be able to replicate in a host cell and are thus non-replicative. VLPs are thus understood to represent particles lacking genetic information and are thus non-replicative. VLPs *per se* are thus non-infectious in the sense that they cannot replicate in a cell to give rise to new viral particles and thus to spread to further cells after a replicative cycle. Still, VLPs, after their assembly and based on the molecules exposed on their surface, can interact with a host cell and/or host cell molecules, e.g. surface receptors, or, after uptake and/or processing by an immune cell, e.g. an antigen-representing cell, epitopes or antigens comprised by a VLP can be presented or cross-presented by the immune cell to effector cells. By means of this interaction, VLPs can induce an immune response in an organism. This ability makes VLPs suitable structures for the provision of immunogenic or vaccine compositions.

A "virus sample", "virus material" or the like refers to a material comprising at least one of a (recombinant infectious) virus particle and/or a virion and/or a VLP.

Whenever the present disclosure relates to the percentage of the homology or identity of nucleic acid or amino acid sequences to each other, these values define those as obtained by using the EMBOSS Water Pairwise Sequence Alignments (nucleotide) programme (http://www.ebi.ac.uk/Tools/psa/emboss_water/nucleotide.html) nucleic acids or the EMBOSS Water Pairwise Sequence Alignments (protein) programme (http://www.ebi.ac.uk/Tools/psa/emboss_water/) for amino acid sequences. Those tools provided by the European Molecular Biology Laboratory (EMBL) European Bioinformatics Institute (EBI) for local sequence alignments use a modified Smith-Waterman algorithm (see http://www.ebi.ac.uk/Tools/psa/ and Smith, T.F. & Waterman, M.S. "Identification of common molecular subsequences" Journal of Molecular Biology, 1981 147 (1):195-197). When conducting an alignment, the default parameters defined by the EMBL-EBI are used. Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5.

### Detailed Description

Human Noroviruses are the leading cause of foodborne illnesses across all age groups and meanwhile account for nearly one fifth of all acute gastroenteritis infections on a global level. Human Noroviruses are presently superseding rotavirus as the most common cause of acute gastroenteritis amongst children, in particular in regions where rotavirus vaccination has been widely and successfully deployed.

As detailed above, there is presently no approved Norovirus vaccine despite the emerging global relevance of Norovirus infections, in particular in children or in the elderly or immunosuppressed population. The inventors thus established a vaccine candidate with high immunogenicity by providing an expression construct allowing the generation of stable and immunogenic VLPs. In addition, the relevant Norovirus antigens were expressed by a self-replicating and therefore self-adjuvanting vector system which may be used to increase the potency of the vaccine candidate. The strategy of co-expression disclosed herein was reported for mono and bi-/multivalent vaccine candidates and can thus also be used for the development of new (mono- or multivalent) Norovirus vaccine candidates in a rapid manner to address the urgent need of promptly generating new and potent vaccine candidates facing the rapid emergence of new and epidemiologically important Norovirus genogroups and genotypes.

In a first aspect, there may this be provided an immunogenic composition comprising at least one recombinant Norovirus antigen, and/or a sequence encoding the same, wherein the at least one recombinant Norovirus antigen is encoded by at least one nucleic acid sequence encoding at least one Norovirus VP1 sequence and at least one Norovirus VP2 sequence, wherein the at least one nucleic acid sequence is operably linked to a vector backbone, wherein the immunogenic composition optionally comprises at least one pharmaceutically acceptable carrier and/or excipient.

In one embodiment, the Norovirus VP1 sequence and/or the Norovirus VP2 sequence may be independently derived from a Norovirus of the GII genogroup, preferably from a Norovirus having a GII.4 genotype, preferably wherein the Norovirus VP1 sequence and the Norovirus VP2 sequence may be encoded by the same nucleic acid sequence and optionally may be separated by at least one linker sequence.

The term "linker" as used herein refers to any coding or non-coding nucleic acid sequence. In one embodiment, the linker sequence may be a regulatory sequence, for example, comprising at least one terminator and/or promoter sequence allowing the separation of a sequence encoding at least one VP1 and/or VP2 sequence from the same or a different genotype/genogroups. Relying on a linker sequence allows the creation of monovalent and multivalent vaccines carrying more than one VP1 and/or VP2 sequence to obtain a vaccine candidate with maximum coverage of prevailing Norovirus antigens. Representative linker sequences are comprised in SEQ ID NOs:9 and 10 and are shown in SEQ ID NOs:17 and 18 (ATUs used as linker regions to insert VP1 and/or VP2 sequences in a targeted way to allow both measles virus replication as well as expression of VP1 and/or VP2 in a functional manner so that VLPs can be formed from the Norovirus sequences inserted, measles regulatory terminator and/or promoter regions).

In another embodiment, the linker sequence may encode a protein sequence which serves as flexible linker connecting VP1 and/or VP2 antigen sequences as long as the linker enhances VLP formation, e.g. the linker may encode a short loop or helical segment further enhancing VLP formation. In certain embodiments, the linker may be a 2A self-cleaving peptide (cf. Liu et al., Sci. Rep., 2017; 7:2193).

The linker sequence may be an artificial sequence. In certain embodiments, the linker sequence may be derived from a sequence of a virus belonging to the family *Paramyxoviridae,* for example, from measles virus, respiratory syncytical virus (RSV), or Vesicular stomatitis virus (VSV), the latter from the family *Rhabdoviridae.* The linker sequence does not need to be endogenous to the genome of a virus used as vector backbone in modified form.

The linker may thus fulfil the function of a spacer either to separate two sequences encoding antigens from each other, or the linker may have regulatory functions, i.e. by encoding a regulatory sequence acting in cis or in trans as regulators. The linker may further include a marker sequence (DNA or encoding a marker protein) to visualize expression, or the linker may comprise elements to allow proper function of the backbone used as vector.

The above immunogenic composition allows both, the provision of an infectious replicative particle which is self-adjuvanting and can be used to propagate itself and thus an immune response in a subject, as well as the provision of non-enveloped VLPs as derived from Norovirus capsid proteins. If desired, the different populations can be further separated from each other as further detailed below.

In one embodiment according to the various aspects disclosed herein, wherein the VP1 and/or the VP2 sequence are independently selected from a Norovirus having a GII.4 genotype, preferably from a sequence from a Norovirus Hu/GII.4/Sydney/NSW0514/2012/AU, a Norovirus Hu/GII.4/Orange/NSW001P/2008/AU, a Norovirus Hu/GII.4/NIHIC17.5/2012/USA, a Norovirus Hu/GII.4/GII4-NG1242/2011/JP, or a Norovirus Hu/GII.4/Hiroshima4/2012/JP, wherein the VP1 and the VP2 sequence are derived from the same Norovirus, or wherein the VP1 and the VP2 sequence are derived from a different Norovirus, optionally wherein the VP1 and the VP2 sequence are modified in comparison to the reference sequence. The term "modified" or "modification" in this context is to be understood as at least one targeted mutation of a (c)DNA or RNA sequence to optimize a reference sequence as retrieved from a Norovirus to obtain, for example, more stable sequences better forming VLPs, codon-optimized sequences showing a better transcription, translation and assembly, or for obtaining a consensus sequence showing a broader scope of protection against various Norovirus genogroups/genotypes and the like.

Notably, Norovirus sequences are readily retrievable from public databases and can be chemically synthesized, optionally including codon optimization, for cloning purposes and vaccine design. Under clinical settings, a Norovirus GI and GII duplex RT-PCR can be performed targeting the 5' end of capsid gene for norovirus genotyping along with a norovirus GI or GII RT-PCR targeting the ORF1/ORF2 overlap for the identification of potential recombinants in patients.

Hu/GII.4/Sydney/NSW0514/2012/AU (*) (GenBank Entry: JX459908) first identified in Sydney in 2012 was chosen as one of the candidates studied in view of the fact that this GII.4 genotype of Norovirus caused severe and pandemic Norovirus infections and was dominating over other genotypes. For the past two decades, Norovirus pandemic variants have emerged around every 3-5 years, and have dominated until they are replaced by alternate strains. However, this scenario changed in 2016 with the co-circulation of six prevalent viruses, three of which possessed the pandemic GII.4 Sydney 2012 capsid for which reason this Norovirus was selected as source for a vaccine candidate. In 2016, there was a decline of the pandemic Sydney 2012 variant, concomitant with the emergence of two novel GII.4 recombinant viruses, both of which retained the Sydney 2012 capsid but acquired new non-structural regions (GII.P4 New Orleans 2009/GII.4 Sydney 2012 and GII.P16/GII.4 Sydney 2012; see Lun et al., Emerging recombinant noroviruses identified by clinical and waste water screening. Emerg. Microbes Infect. 2018;7:50). Therefore, it was followed that the VP1 and VP2 sequences of the original virus seem to be rather conserved. It was thus also speculated that the Hu/GII.4/Sydney/NSW0514/2012/AU sequences could serve as one of at least two sequences to obtain a multifunctional consensus sequence to obtain a vaccine providing broad and more than seasonal protection against Norovirus infection. Based on the prototypic expression construct, further mono-and multivalent Norovirus vaccine candidates were cloned to show that the strategy disclosed herein may be rapidly adapted to new emerging Norovirus strains, as well as known genogroups and genotypes (e.g. Norwalk virus belonging to the GI genogroup) and can be used to provide broad protection against different genogroups/genotypes, which becomes of increasing importance in a global society where different Norovirus strains are dominant in certain regions due to climatic and other reasons.

Other Norovirus strains can be selected from, but are not restricted to Norovirus Hu/GII.4/NIHIC17.5/2012/USA (GenBank Entry: KF429790), Norovirus Hu/GII.4/Orange/NSW001P/2008/AU (*) (GenBank Entry: GQ845367), Norovirus Hu/GII.4/GII4-NG1242/2011/JP (GenBank Entry: AB972502.1), or a Norovirus Hu/GII.4/Hiroshima4/2012/JP (GenBank Entry: AB972494.1). Further GII.4 variants can be selected from CHDC5191/1974/US (GenBank Entry: FJ537134), Lorsdale/1993/GB (GenBank Entry: X86557), Camberwell/1994/AU (GenBank Entry: AF145896), Hu/NLV/Dresden174/pUS-Norll/1997/GE (*) (GenBank Entry: AY741811), Hu/Houston/TCH186/2002/US (GenBank Entry: EU310927), Hu/GII.4/Kaiso/030556/2003/JP (GenBank Entry: AB294779), Hu/NoV/Farmington Hills/2002/USA (*) (GenBank Entry: AY502023.1), Norovirus Hu/NLV/Dresden174/pUS-Norll/1997/GE (GenBank Entry: AY741811.1), Norovirus Hu/GII.4/Hunter504D/040/AU (*) (GenBank Entry: DQ078814.2), Norovirus Hu/GII.4/Shellharbour/NSW696T/2006/AUS (*) (GenBank Entry: EF684915.2), Norovirus Hu/GII.4/Osaka1/2007/JP (GenBank Entry: AB541319.1), Norovirus Hu/GII.4/Sutherland/NSW505G/2007/AUS (GenBank Entry: (GenBank Entry: GQ845368.2), or a Norovirus Hu/GII.4/JB-15/KOR/2008 (GenBank Entry: HQ009513.1). GII.4 variants marked with an asterisk (*) were associated with a pandemic of acute gastroenteritis and demonstrated a global distribution (Eden et al., Recombination within the pandemic norovirus GII.4 lineage, J. Virol. 2013 Jun;87(11):6270-82. doi: 10.1128/JVI.03464-12).

Further suitable Norovirus sequences, including non-GII.4 sequences recently of interest in Asia, are disclosed in Chi-Wai et al. (Bimodal Seasonality and Alternating Predominance of Norovirus GII.4 and Non-GII.4, Hong Kong, China, 2014-2017, CDC EID Journal, vol. 24, 2018). Another Norovirus, prototypic, but not limiting for a genogroup GI strain, is Norwalk virus (Hu/GII.6/CHDC4073/1984/USA, GenBank entry: JX846927.1).

Selecting a Norovirus genotype having a pandemic potential may be preferable in certain embodiments. In view of the rapid antigenic drift and shift of Norovirus and differences in global Norovirus genotype distribution, it may be preferable to provide an immunogenic composition comprising at least to different antigens (e.g. VP1 and VP2) being able to form a functional, i.e. a self-assembling and immunogenic antigen presenting, VLP. In another embodiment, it may be preferable to combine different VP1 sequences from different genogroups/genotypes VP1 being able to self-assemble into a VLP.

In certain embodiments, the at least one sequence encoding at least one VP1 and/or VP2 antigen may be modified in comparison to the wild-type sequence as retrievable from a wild-type isolate as sequenced from material obtained from a patient.

In other embodiments, it may be preferable to design a bi- or multivalent vaccine comprising at least one VP1 or VP2 sequence from a first genogroups or genotype and further comprising another at least one VP1 or VP2 sequence from another genogroups or genotype.

In certain embodiments according to the various aspects disclosed herein, an immunogenic composition may comprise more than one distinct Norovirus VLP, for example by creating a tandem construct wherein the vector backbone comprises a sequence encoding a first VP1 and optionally a second VP2 sequence building one first VLP, and wherein the vector backbone further comprises a third and optionally a fourth sequence encoding a different VP1 and optionally a different VP2 sequence. These sequences can be separated by adequate linker sequences as disclosed herein and they can be cloned into the same, or into a different ATU sequence in the vector backbone. The immunogenic composition thus created will thus comprise antigenic diverse VLPs derived from different Norovirus isolates. This can provide a multiplication of the immunogenic function and in particular a wider scope of protection against different Norovirus genotypes so that an individual can be protected against infection with different Norovirus genogroups or genotypes emerging from different regions worldwide.

In certain embodiments according to the various aspects of the present invention it may be preferable to use a VP1 sequence together with a VP2 sequence to obtain more stable VLPs showing a better antigen presentation to the immune system.

Depending on the at least one VP1 antigen chosen, and in turn the sequences encoding the same, there may also be provided an immunogenic composition comprising at least one recombinant Norovirus antigen, wherein the antigen is encoded by at least one nucleic acid sequence encoding at least one Norovirus VP1 sequence, wherein the at least one nucleic acid sequence is operably linked to a vector backbone, wherein the immunogenic composition optionally comprises at least one pharmaceutically acceptable carrier and/or excipient. In this embodiment, the use of a suitable linker allows the provision of at least two, at least three, at least four, at least five, at least six, at least seven, at least eight, at least 9 or even at least ten different VP1 sequences all provided in the same vector backbone so that an immunogenic composition can be provided that comprises at least two different VP1 Norovirus antigens, preferably obtained from different Norovirus genogroups or genotypes so that a bi- or multivalent vaccine can be provided that simultaneously provides a subject protection against two or even several different Noroviruses.

In certain embodiments, the at least one VP1 and/or the at least one VP2 sequence represent(s) a conserved consensus sequence, preferably a consensus sequence within the same genotype, i.e. GII.4, GII.17 and the like. As detailed above, Norovirus strains show a regional distribution and related, but not identical, Norviruses may be dominating in certain regions. Therefore, it may be suitable to identify a consensus sequence as detailed in **Figure 1** and **Figure 2** to obtain an optimum protection of a vaccine candidate comprising antigenic sequences covering different genotypes. The skilled person can easily define a suitable consensus sequence based on at least two, preferably at least three sequences compared as depicted for the disclosure provided with **Figure 1** and **Figure 2****.** Highly conserved amino acid positions will always be present in a consensus sequence. In case sequence comparison reveals that certain amino acid positions are less conserved over the same genotype, more than one VP1 and/or VP2 sequence encoding the respective capsid proteins and thus antigens from a Norovirus may be selected. The consensus sequence is determined over the whole length of a sequence encoding a Norovirus VP1 and VP2 sequence. The consensus sequence may comprise mutations and/or the consensus sequence may be truncated. Preferably, the consensus sequence has at least 90%, 91%, 92%, 93%, 94%, 95%, 96%, 97%, 98% or 99% amino acid sequence identity to each reference sequence used for creating the consensus sequence over the whole length of the respective reference amino acid sequences.

In certain embodiments, the at least one recombinant Norovirus VP1 antigen may be selected from an amino acid sequence of SEQ ID NOs:1, 11, 13 or 15 or a nucleic acid sequence encoding the same (e.g., SEQ ID NOs:3, 5) or an amino acid sequence having at least 90%, 91%, 92%, 93%, 94%, 95%, 96%, 97%, 98% or 99% sequence identity thereto, and wherein the at least one recombinant Norovirus VP2 antigen is selected from an amino acid sequence of SEQ ID NOs:2, 12, 14 or 16 or a nucleic acid sequence encoding the same (e.g., SEQ ID NOs:4, 6), or an amino acid sequence having at least 90%, 91%, 92%, 93%, 94%, 95%, 96%, 97%, 98% or 99% sequence identity thereto. Any other Norovirus VP1 and/or VP2 sequence, in particular a seasonal or recent sequence emerging and having been identified and thus being publicly available, may be used. SEQ ID NO:1 represents the VP1 amino acid sequence from Hu/GII.4/Sydney/NSW0514/2012/AU and SEQ ID NO:3 represents the corresponding nucleic acid sequence. SEQ ID NO:2 represents the VP2 amino acid sequence from Hu/GII.4/Sydney/NSW0514/2012/AU and SEQ ID NO:4 represents the corresponding nucleic acid sequence. SEQ ID NOs:5 and 6 represent codon-optimized nucleic acid sequences of VP1 and VP2 of Hu/GII.4/Sydney/NSW0514/2012/AU, respectively. SEQ ID NO:7 then shows an artificial VP1-linker-VP2 constructs derived from of Hu/GII.4/Sydney/NSW0514/2012/AU and SEQ ID NO:8 shows a codon-optimized VP12-linker-VP2 construct as derived from of Hu/GII.4/Sydney/NSW0514/2012/AU sequences as reference sequences for VP1 and VP2. SEQ ID NOs:11 and 12 show the amino acid sequence of VP1 and VP2 of Norovirus Hu/GII.4/NIHIC17.5/2012/USA, respectively. SEQ ID NOs:13 and 14 show the amino acid sequence of VP1 and VP2 of Norovirus Hu/GII.4/GII4-NG1242/2011/JP, respectively. SEQ ID NOs:15 and 16 show the amino acid sequence of VP1 and VP2 of Norovirus Hu/GII.4/Hiroshima4/2012/JP, respectively. These sequences are represented here in an exemplary manner to provide some more recent Norovirus GII.4 sequences. As detailed above, the skilled person can easily retrieve further Norovirus sequences of different genogroups and genotypes from publicly available databases.

According to the various embodiments of the present invention, the vector backbone serving as platform to introduce at least one, preferably at least one VP1 and at least one VP2 Norovirus antigen according to the present invention may be selected from the group consisting of a measles virus backbone, a lentivirus backbone, a Vesicular stomatitis virus (VSV) backbone, or a vaccinia Ankara backbone, preferably wherein the vector backbone may be from a recombinant live-attenuated measles virus strain, the measles virus strain preferably being selected from the group consisting of a Schwarz strain, a Zagreb strain, an AIK-C strain and a Moraten strain. Preferably, a vector backbone is a modified recombinant backbone to fulfill the needs of a safe vaccine as well as the needs of a suitable multipurpose cloning vector. Preferably, the backbone should be recombinantly optimized to fulfill the needs for a viral vaccine vector.

A preferred vector backbone according to the present disclosure is a measles virus (MV) backbone. For example, EP 1 939 214 B1 discloses that live attenuated RNA viruses especially the measles vaccine, has been used in hundreds of millions of children and has been proven to be effective and safe. This vaccine induces life-long immunity after one or two injections. It is easily produced on a large scale at low cost in most countries. These advantages make measles virus, especially attenuated vaccine strains, a good candidate vector to immunize children. Measles virus (MV) belongs to the genus Morbillivirus in the family *Paramyxoviridae.* It is an enveloped virus with a non-segmented RNA genome of negative polarity (15,894 bp). Measles can only be contracted once as the immune system mounts a strong specific response and establishes life-long memory protecting against re-infection. Such protection is based on both the production of antibodies and memory cytotoxic CD8+ T lymphocytes (CTL). Pathogenic strains strongly disrupt haematopoiesis (Arneborn et al., 1983; Kim et al., 2002; Okada et al., 2000) thus resulting in transitory immunosuppression responsible for most deaths due to measles infection in developing countries. In contrast to primary strains, attenuated strains do not induce immunosuppression (Okada et al., 2001). Transcription and replication of measles virus does not involve the nucleus of the infected cells but rather take place in the cytoplasm of said infected cells. The genome of the measles virus comprises genes encoding six major structural proteins from the six genes (designated N, P, M, F, H and L) and two additional non-structural proteins from the P gene. The gene order is the following: 3', N, P (including C and V), M, F, H, and L large polymerase protein at the 5' end. The genome further comprises non coding regions in the intergenic region M/F; this non-coding region contains approximately 1000 nucleotides of untranslated RNA. The cited genes respectively encode the leader peptide (I gene), the proteins of the nucleocapsid of the virus, i.e., the nucleoprotein (N), the phosphoprotein (P), and the large protein (L) which assemble around the genome RNA to provide the nucleocapsid. The other genes encode the proteins of viral envelope including the hemagglutinin (H), the fusion (F) and the matrix (M) proteins. The Edmonston strain of measles virus was already isolated in 1954 by culture in primary human cells (Enders et al., 1954). Adaptation to chicken embryonic fibroblasts produced vaccine seeds that were furthermore attenuated by subsequent passages in chicken embryonic fibroblasts (Schwarz et al., 1962). The Schwarz and Moraten strains that possess identical nucleotide sequences (Parks et al., 2001a; Parks et al., 2001 b) constitute the most frequently used measles vaccine. Vaccination with one or two injections induces life-long immunity (Griffin et al., 2001; Hilleman et al., 2002). The inventors of EP 1 939 214 B1 have developed a vector using the Schwarz MV, the most commonly used measles vaccine in the world (Combredet et al., 2003). This vector can stably express a variety of genes or combination of large genes for more than 12 passages.

Recombinant MV vectors containing 4,000-5,000 additional nucleotides were produced, representing an additional 30% of genome. These viruses were produced in cell culture at titers comparable to standard MV. To optimize the output of the reverse genetics system, the antigenomic viral cDNA was placed under the control of the T7 phage RNA polymerase promoter with an additional GGG motif required for optimal efficacy. To allow exact cleavage of the viral RNA, a hammerhead ribozyme was inserted between the GGG motif and the first viral nucleotide, and the ribozyme from hepatitis delta virus was placed downstream of the last viral nucleotide. The resulting pTM-MVSchw plasmid enabled the production of the corresponding virus using a previously described reverse genetics system based on the transfection of human helper cells (Radecke et al., 1995). Furthermore, EP 1 939 214 B1 discloses that pTM-MVSchw plasmid was modified for the expression of foreign genes by the introduction of additional transcriptional units (ATU) at different positions of the genome. These ATUs are multi-cloning site cassettes inserted in a copy of the intergenic N-P region of the viral genome (containing the cis acting sequences required for transcription). With the help of the ATUs, and following precise cloning rule, chimeric viruses can thus be created using MV as backbone for the expression of a non-MV sequence.

To be suitable as general purpose expression vector, MV thus has to be attenuated and has to fulfil certain characteristics of a classical cloning vector, i.e. suitable unique cloning sites have to be present. Furthermore, the so-called "rule of six" has to be fulfilled if working with a sequence encoding an infectious replicative MV. This rule implies the total number of nucleotides present in the (antigenomic) cDNA encoding MV amounts to a multiple of six, which allows sufficient replication of genome RNA of the measles virus.

In certain embodiments, a suitable Kozak sequence may be included before the start codon for a sequence encoding a VP1 and/or a VP2 protein according to the present disclosure. A Kozak sequence is a sequence which occurs on eukaryotic mRNA and usually has the consensus (gcc)gccRccAUGG. The Kozak consensus sequence plays a major role in the initiation of the translation process and may vary from species to species. The skilled person can easily define a suitable Kozak sequence. Examples in the context of the present invention can be derived from SEQ ID NOs:9 and 10, where a Kozak sequence precedes the coding sequence of the VP1 and/or the VP2 sequence.

Furthermore, unique cloning sites have to be present to allow the insertion of a heterologous sequence of interest to generate a chimeric MV including an insert of interest. Suitable, but not limiting, restriction enzyme cutting sites can be selected from a *BsiW*I (5' cloning site for heterologous gene) and a *BssH*II (3' cloning site for heterologous gene) site as also present in SEQ ID NOs:9 and 10.

In a preferred embodiment according to the various aspects of the present disclosure, a MV backbone may comprise at least one ATU. An ATU, or several ATUs, may, for example, be inserted between the regions encoding MV P and M proteins, or between the regions encoding H and L proteins. Suitable ATUs according to the present disclosure are shown in SEQ ID Nos:17 and 18, which represent the 5'and 3' ATU as present in SEQ ID NO:10 and represent measles terminator/promotor regions for heterologous gene expression, i.e. for the expression of a sequence encoding VP1 (SEQ ID NO:9) or VP1 and VP2 (SEQ ID NO:10). As detailed above, additional ATUs can be inserted between coding regions of MV genes provided that the insertion does not to disrupt the biological activity of MV (transcription, expression and self-assembly in a host cell).

This basis recombinant and infectious measles vector thus allows the design of combined vaccines based on a live attenuated vaccine strain so that the recombinant measles virus vector may be used as a versatile backbone or scaffold for the introduction, production and purification of infectious virus particles or as cargo for the expression of VLPs consisting essentially of viral structural proteins or epitopes other than those derived from a measles virus strain.

In particular, in the context of combating viruses having a high antigenic drift and/or shift tendency, as it is the case for Norovirus or Influenza, the recombinant and specifically modified MV backbone can thus be used as platform to rapidly establish seasonal vaccines rapidly to fulfil the need of providing a new vaccine generation at short notice.

In certain embodiments, the vector backbone may comprise a nucleotide sequence of the full-length, infectious antigenomic (+) RNA strand of a measles virus, preferably wherein the immunogenic composition comprises a sequence of SEQ ID NO:10, or a sequence having at least 80%, 81%, 82%, 83%, 84%, 85%, 86%, 87%, 88%, 89%, 90%, 91%, 92%, 93%, 94%, 95%, 96%, 97%, 98% or 99% sequence identity thereto.

In another embodiment, the full-length, infectious antigenomic (+) RNA strand of a measles virus may be further modified to comprise suitable mutations or truncations, for example a full or partial knock-out of a structural or non-structural gene, or a mutation having a further attenuating effect, or a targeted mutation optimizing the replication rate and the like.

In yet a further embodiment, the immunogenic composition may be a bivalent or a multivalent immunogenic composition, wherein the at least one nucleic acid sequence encoding the at least one Norovirus VP1 sequence and/or the at least one nucleic acid sequence encoding at least one Norovirus VP2 sequence may be selected from at least two different Norovirus genogroups, preferably a GII and a GI genogroups, and/or wherein the at least one nucleic acid sequence encoding the at least one Norovirus VP1 sequence and/or the at least one nucleic acid sequence encoding at least one Norovirus VP2 sequence may be selected from different Norovirus GII genotypes, preferably a GII.4 and a GII.17 or GII.2 genotype.

The combination of different GII genotypes, or the inclusion of a GI genogroup antigen together with a GII genogroup antigen can further enhance the therapeutic spectrum and may be of increasing importance facing the global spread of different Noroviruses to regions other than the region the virus was initially identified.

In a second aspect, there is provided a nucleic acid molecule, wherein the nucleic acid molecule may comprise at least a first and at least a second nucleic acid sequence, wherein the first nucleic acid sequence may encode at least one VP1 protein of a Norovirus or a functional fragment thereof, and wherein the second nucleic acid sequence may encode at least one VP2 protein of a Norovirus or a functional fragment thereof, optionally wherein the at least one first and the at least one second nucleic acid sequence is/are separated by at least one linker.

Based on the construction principle detailed above for the first aspect of the present invention, Norovirus constructs suitable to form a Norovirus VLP and thus suitable as antigen presenting entities for vaccination purposes may be designed rapidly in view of the rapid adaption and change of the Norovirus.

The co-expression of at least one VP1 and at least one VP2 encoding sequence can enhance the stability of the VLPs formed from the capsid proteins after recombinant expression or after immunization so that a reliable immune response can be triggered by the antigens such presented.

In one embodiment, the at least one first and/or the at least one second nucleic acid sequence may be independently selected from a Norovirus having a GII.4 genotype, preferably from a sequence from a Norovirus Hu/GII.4/Sydney/NSW0514/2012/AU, a Norovirus Hu/GII.4/Orange/NSW001P/2008/AU, a Norovirus Hu/GII.4/NIHIC17.5/2012/USA, a Norovirus Hu/GII.4/GII4-NG1242/2011/JP, or a Norovirus Hu/GII.4/Hiroshima4/2012/JP, wherein the at least one nucleic acid sequence encoding the at least one Norovirus VP1 and the at least one nucleic acid sequence encoding the at least one Norovirus VP2 may be derived from the same Norovirus, and/or wherein the at least one nucleic acid sequence encoding the at least one Norovirus VP1 and the at least one nucleic acid sequence encoding the at least one Norovirus VP2 may be derived from a different Norovirus, preferably selected from a Norovirus of a different genogroups, including genogroups GI, GII and GIV, and/or wherein the at least one nucleic acid sequence encoding the at least one Norovirus VP1 and/or the at least one nucleic acid sequence encoding at least one Norovirus VP2 are selected from different Norovirus GII genotypes, preferably a GII.4 and a GII.17 or GII.2 genotype. As already detailed above for the first aspect, relying on the basic cloning and construction principles disclosed herein allowing the provision of an immunogenic composition comprising both infectious virus particles and Norovirus VLPs as self-assembled in a functional form, other Norovirus VP1 and/or VP2 sequences can be defined.

In one specific embodiment, the nucleic acid molecule may comprise a sequence selected from any one of SEQ ID NOs:3 to 8, or a sequence having at least 80%, 81%, 82%, 83%, 84%, 85%, 86%, 87%, 88%, 89%, 90%, 91%, 92%, 93%, 94%, 95%, 96%, 97%, 98% or 99% sequence identity thereto, or wherein the first nucleic acid sequence and/or the second nucleic acid sequence represent(s) a conserved consensus sequence.

There is no specific order in which the at least one VP1 and/or the at least one VP2 sequence have to be provided in a vector backbone as long as a functional full-length sequence can be expressed, which can then alone (VP1) or together with another partner (VP1 and VP2) assemble into a VLP presenting the relevant antigens.

In a third aspect according to the present invention, there is provided a recombinant virus-like particle (VLP), wherein the VLP may comprise at least one recombinant Norovirus VP1 antigen and at least one recombinant Norovirus VP2 antigen as defined according to the first aspect detailed above.

One advantage of the immunogenic compositions according to the present invention is the fact that the vector backbone itself provide immune-stimulation so that no additional adjuvant has to be provided in the immunogenic composition. For certain applications, however, it may be favorable to administer a composition which is not replicative, i.e. a VLP originating from a Norovirus. The present invention provides methods to produce both infectious virus particles as well as VLPs as well as methods to separate the two populations from each other to provide an immunogenic composition which comprises both populations, or to provide an immunogenic composition which comprises either infectious virus particles or VLPs. More than one VLP species may be present in certain embodiments, e.g. VLPs from a VP1 and VP2 protein, VLPs only comprising VP1 proteins, or compositions comprising several VLPs of different constitution, e.g. when using several different VP1 and/or VP2 sequences, for example, in a monovalent vaccine. Immunogenic compositions exclusively comprising VLPs can be further provided together with at least one pharmaceutically acceptable adjuvant.

In certain embodiments, the ratio between infectious virus particles (IVPs) and the at least one VLP, which may have the same or a different composition of VP1 and/or VP2 Norovirus antigens, may be specifically balanced. I.e., the ratio of IVPs to VLPs may be 1:1; 1:5; 1:10; 1:20; 1:50; 1:100; 1:500; 1:1,000; 1:5,000; 1:10,000 and the like. A surplus of VLPs in the composition may be suitable in certain patient/subject groups. Still, the presence of IVPs, at least to a certain extent, can be favorable in view of the self-adjuvanting effect thereof. Based on the methods disclosed herein, immunogenic compositions comprising a specific ratio of IVPs and VLPs can be produced in a reliable manner. In certain embodiments, where a strong immune response, in particular a string initial innate immune response is required, the amount of IVPs may be greater (in terms of ration) than the number of VLPs. In certain embodiments, only IVPs or only VLPs may be provided.

In another aspect, there is provided a host cell, wherein the host cell may comprise at least one nucleic acid sequence encoding at least one Norovirus VP1 sequence and at least one Norovirus VP2 sequence according to the first aspect above, or wherein the host cell may comprise at least one nucleic acid molecule according to the second aspect disclosed above, or wherein the host cell may comprise at least one nucleic acid sequence encoding a recombinant virus-like particle (VLP) according to the third aspect, preferably wherein the at least one host cell is selected from the group consisting of Vero cells, chicken embryo fibroblast cells, HEK293 cells, HeLa cells, fetal rhesus lung cells or MRC5 cells. Other suitable host cells are available to the skilled person. The host cell has to be compatible with a propagation of the vector backbone chosen.

In yet a further aspect according to the present invention, there is provided a method for producing an immunogenic composition as disclosed herein, or for producing a recombinant virus-like particle (VLP) as disclosed herein, wherein the method may comprise the following steps: (i) inserting at least one nucleic acid sequence as defined in the context of the immunogenic compositions disclosed herein, or inserting at least one nucleic acid molecule as disclosed herein, into a vector backbone as defined in the context of the first aspect disclosed herein to operably link the nucleic acid sequence or molecule and the vector backbone to obtain a recombinant chimeric virus sequence, wherein chimeric implies that the vector backbone and the insert originate from different viral sequences and may additionally be modified or codon-optimized; (ii) infecting at least one host cell with at least one recombinant chimeric virus sequence obtained in step (i) to obtain a virus sample, wherein the virus sample may comprise a mixture of infectious virus particles and VLPs; (iii) optionally: clarifying the virus sample of step (ii); (iv) optionally: purifying the clarified virus sample of step (iii); (v) optionally: formulating the at least one recombinant chimeric virus with at least one pharmaceutically and/or veterinary acceptable carrier and/or excipient; (vi) obtaining an immunogenic composition comprising infectious virus particles and virus-like particles (VLPs); (vii) optionally: further purifying the immunogenic composition and thus obtaining, separated from each other, recombinant virus-like particles (VLPs) and/or infectious virus particles.

The term "clarifying" or "clarification" according to the present disclosure refers to a step for removing large impurities from a bulk product such as a cell culture supernatant to be clarified. The term removing also encompasses a reduction in the amount (e.g. concentration) of impurities. Clarification may rely on separation by size, molecular weight or density and the like. "Large impurities" is to be taken to preferably refer to a size bigger than, for example, sterically demanding MV particles. Clarification thus aims at removing cells and cell debris from the host cells infected with and producing a virus and/or VLPs. Clarification usually does not include separation of impurities that have a size corresponding to the virus particles to be purified or smaller. Common methods for clarification are centrifugation and microfiltration, including tangential flow filtration (TFF), ultracentrifugation, dead end filtering and the like. In the context of clarifying a bulk product comprising MV particles centrifugation is less desired, as centrifugation processes are not easily scalable under GMP conditions.

Preferably, chromatography, in particular flow-through chromatography may be used for purification. Ac chromatography unit at least comprises a stationary phase material that is preferably selected from the group consisting of at least a resin, at least a matrix, at least a gel and, preferably, beads. In a preferred embodiment, the stationary phase material is comprised in a container, e.g. for carrying out column chromatography. In preferred embodiments, the stationary phase material, preferably the beads, has/have at least size-exclusion functionality, hydrophobic interaction functionality or ion exchange functionality, or a combination thereof, preferably at least size-exclusion functionality, optionally combined with hydrophobic interaction functionality. The stationary phase material, preferably the beads, may comprise a ligand-activated core, and an inactive shell comprising pores. In another embodiment, the stationary phase may have a monolithic arrangement.

In certain embodiments according to the above method of the present invention, there is further provided a method additionally comprising a further purification step, comprising: further purifying the recombinant chimeric virus and/or VLPs as pharmaceutically active constituents by means of filtration, centrifugation, tangential flow filtration, membrane filtration, purification with grafted media, aqueous two phase extraction, precipitation, buffer exchange, dialysis or chromatography, including size exclusion chromatography for separating the purified recombinant infectious virus particles derived from a measles virus into a fraction containing virions and another fraction containing virus-like particles or recombinant subviral particles. Said embodiment is especially useful in case the antigenic region inserted as nucleic acid sequence into an attenuated vector backbone, preferably a measles virus vector backbone, has to be further separated into a fraction containing the replication competent virions of the measles virus containing genetic material, and the VLPs self-assembled from the antigens of the at least one Norovirus, preferably from at least one VP1 and at least one VP2 protein from a Norovirus, wherein the VLPs are devoid of any genetic material. Therefore, they possess relevant surface antigens to elicit an immune response, preferably a protective immune response, but cannot further be propagated in a host cell, which makes VLPs and other subviral particles an interesting target for several applications in immunology.

In view of the rather pronounce difference in the size and physico-chemical properties of infectious measles virus particles and VLPs derived from at least one Norovirus, specific purification strategies have to be applied as further detailed in Examples 4 and 5 below. In yet a further embodiment, there is provided an immunogenic composition according to the first aspect, or an immunogenic composition comprising at least two VP1 antigens from different Noroviruses, or a recombinant virus-like particle (VLP) to the third aspect disclosed herein for use in a method of preventing or treating a Norovirus virus disease in a subject.

As detailed above and as further detailed in the below Examples, the immunogenic compositions and the VLPs disclosed herein are suitable in triggering a protective immune response in a subject. The VLPs generated and comprised by the immunogenic compositions are stable and highly efficient in presenting the Norovirus antigens to elicit a protective immune response whilst having low reactogenicity.

In a further aspect, an immunogenic composition according to the first aspect, or an immunogenic composition comprising at least two VP1 antigens from different Noroviruses, or a nucleic acid molecule according to the second aspect, or a recombinant virus-like particle (VLP) according to the third aspect for use in a method of preventing or treating a Norovirus virus disease in a subject. A method for preventing or treating a Norovirus disease in a subject may thus be provided which allows the prevention or reduction of the symptoms of a Norovirus infection in a subject, i.e. a prevention or reduction of the symptoms of acute gastroenteritis in all age groups to reduce severe disease burden and thus complications in infected populations, in particular amongst children and elderly people, for which dehydration associated with Norovirus infection is a huge problem and associated with high rates of hospitalization and severe complications.

The present invention is further described with reference to the following non-limiting examples.

### Examples

### Example 1: Norovirus antigen design

For vaccine design, it was a main intention to design a versatile cloning strategy allowing the rapid provision of Norovirus vaccines in a modular manner. In view of the fact, on a global scale, different Norovirus genotypes and genogroups are predominating in different climate zones and populations, a vaccine should be created covers the most severe recently pandemic Norovirus genotypes and that can be adapted at short notice to keep up with Norovirus evolution.

Noroviruses are non-enveloped and belong to the Family of *Caliciviridae.* The genus is divided into 6 genogroups of which genogroup I, II and IV contain primarily human Noroviruses. The genome is organized in three open reading frames (ORF). Noroviruses produce two capsid proteins, the major capsid protein VP1 encoded by ORF2 and the minor capsid protein VP2 encoded by ORF3. These two proteins are translated from a subgenomic RNA. The outer capsid shell consists of the major capsid protein VP1. This protein has the ability to self-assemble into virus-like particles (VLPs), which morphologically and antigenically resemble the native virus. Therefore, VLPs are highly immunogenic and are the best candidate for vaccine development. No additional protein is needed for the VLP formation. Nevertheless, the minor capsid protein VP2 if expressed together with VP1 can also be incorporated into the virus-like particles. VP2 stays within the capsid, on the inner side of the capsid shell, and is thus not target of the immune response.

To take all this information into account, a Norovirus vaccine candidate based on the expression of either VP1 alone and VP1 and VP2 was developed. In a first instance, the coding sequence of VP1 and VP2 were chosen. As these proteins are not expressed as one polyprotein that is co- or post-translationally cleaved by host proteases, a transcriptional terminator-promotor region was introduced between the two coding sequences to ensure that both proteins are expressed and are thus available for VLP formation. The measles-specific terminator-promotor region located between P and M was taken and inserted in between (cf. SEQ ID NOs: 9 for VP1 and SEQ ID NO:10 for VP1 and VP2 both cloned into a specifically adapted ATU of the measles virus antigenomic sequence). Furthermore, it was crucial to identify a suitable backbone sequence in advance. The measles backbone as disclosed herein has several advantages. First, the vector backbone per se is safe and established. When properly taking into consideration the specific needs of this vector, it can be used as efficient shuttle platform to produce infectious virus particles based in the measles backbone as well as VLPs mainly as determined by the specific Norovirus insert to allow a rapid vaccine production.

For cloning, a Kozak sequence was also added in front of Norovirus insert sequence (cf. SEQ ID NOs:9 and 10) in order to obtain an optimal protein translation in target cells. This step, however, may be optional in view of the fact that Norovirus sequence usually express well in mammalian cell systems. In order to avoid read-through during protein translation, more than one stop codon was added at the end of the Norovirus antigens. Two or three stop codons were added to obey the rule of six as demanded by the measles backbone chosen as vector, respectively. This rule of six is especially important, if measles virus-derived RNA is used as a vector backbone, as otherwise no replication would be initiated. If working with other vector backbones, other design parameters have to be considered.

As a proof of principle of the general applicability of the cloning technique designed, two further constructs were established. First of all, it was the idea to create a Norovirus vaccine that also provides protection against severe non-GII.4 genotypes, for example, as those genotypes implicated in epidemics in China and Hong Kong, where a bimodal seasonality of Norovirus is observed, nowadays often also caused by non-GII.4 genotypes like GII.17 affecting all age groups GII.2 affecting older children an younger adults. Therefore, VP1 and VP2 genes of non-GII.4 genotypes, for example, as disclosed in Chi-Wai et al. (Bimodal Seasonality and Alternating Predominance of Norovirus GII.4 and Non-GII.4, Hong Kong, China, 2014-2017, CDC EID Journal, vol. 24, 2018) were cloned and codon-optimized. Notably, monovalent (GII.2 only and GII.17 only) and bivalent (GII.4 VP1 or VP2 and correspondingly a different VP1 or VP2 gene from a GII.2 or GII.17 genotype) were cloned. Another interesting candidate is a VP1 or VP2 from a GI genotype, i.e. that genotype the "prototypic" Noro-Norwalk virus belongs to. Fortunately, new Norovirus sequences are sequenced and published rapidly so that new potentially antigenic sequences can be retrieved ad hoc from various accessible databases so that the sequences can be further designed *in silico* and can be sent to synthesis afterwards without the need for a biological material of a Norovirus infected patent as template.

Another idea was to define a generally applicable consensus sequence derived from pandemic Norovirus. This strategy relies on the comparison of VP1 and VP2 sequences from at least two or three genotypes which have been identified as relevant cause of Norovirus outbreaks. An example of this strategy is presented in **Figure 1** and **2****.** First, the sequences are retrieved from the relevant databases and are then subjected to an alignment, preferably a sequence alignment optimized for high accuracy, e.g. MUSCLE. The date thus obtained can be immediately analyzed by MView (version 1.63). As evident from **Figure 1B** and **Figure 2B****,** this allows a graphical overview of highly conserved amino acid sequences within the different genotype sequences analyzed. Highly conserved sequences, i.e. sequences conserved in all compared sequences, should thus always be present in a consensus sequence. Other, non or less conserved sequences can be designed to obtain optimum protection against different genotypes by combining prominent amino acid residues of different characteristic, for example, in **Figure 1B****,** position 80 being a rather positively charged arginine in SEQ ID NO:12, and a likewise positively charged lysine in SEQ ID NOs:2, 14 and 16. Therefore, a positively charged residue (+) should be introduced into the consensus sequence. Interestingly, position 92 is a phenylalanine (F) in SEQ ID NO:2, but a serine in the other sequences compared in **Figure 1****.** S and F, however, have completely different properties. Therefore, a consensus sequence could be used relying on either the S or the F residue to test which sequence provides optimum protection. For example, a S could be integrated at the relevant position in an otherwise SEQ ID NO:2 based sequence to create chimeric sequences providing optimum protection against different Norovirus genotypes.

### Example 2: Codon optimization, mutagenesis and vector construction

The selected Norovirus antigens were subjected to codon optimization. Again, this might be an optional step in view of the fact that Norovirus is perfectly adapted to be expressed in a mammalian/human host cell. Codon optimization to adapt the codon usage of the desired nucleic acid sequences to a human codon usage was performed by Eurofins Genomic. MV-editing sequences were avoided. Cloning of synthesized Norovirus inserts (synthesis: Eurofins Genomics) into a recombinant vector backbone was performed using standard techniques of molecular biology. Preferably, inserts were cloned into the vector backbone derived from a measles virus Schwarz strain into the ATU2 region using the restriction enzymes *BsiW*I and *BssH*II*.*

### Example 3: Infection and master cell bank (MCB) and rescue

For the purpose of this example, a recombinant, Norovirus antigen expressing, live attenuated chimeric virus (see SEQ ID NOs:9 and 10) was used which was generated by transfecting helper cells with the vector, wherein said helper cells (the human embryonic kidney cell line 293 was chosen because it is highly permissive for MV. In addition, these cells can be transfected efficiently, e.g. by the calcium phosphate co-precipitation method) are capable of expressing helper functions to express an RNA polymerase, and to express the N, P and L proteins of a MV virus;) co-cultivating said transfected helper cells of step 1) with passaged cells suitable for the passage of the MV attenuated strain from which the cDNA originates; 3) recovering the recombinant infectious virus expressing at least one structural protein of a Norovirus to provide a master virus seed stock (MVSS). Vero 10-87 cells served as host cells and as master cell bank (MCB). The MVSS used for this exemplary purification scheme can be obtained from the sequence according to SEQ ID Nos:9 and 10 according to the present application.

Details on the rescue procedure for measles virus based MVSSs can be taken from Radecke et al. (Rescue of measles viruses from cloned DNA EMBO J. 1995 Dec 1; 14(23): 5773-5784).

### Example 4: MCB revival, expansion, harvest and purification under GMP conditions

For vaccine production, sterile and reproducible production strategies are needed, in particular when working with potentially Two cryovials each containing 1.0 mL MCB were removed from storage in vapour phase liquid nitrogen and are transported to the cleanroom in a sanitised container which is in turn transported on dry ice. Once within the cleanroom, the MCB cryovials were thawed in hands/at ambient while gently swirling the content until all ice within the vials has melted. When the vials had thawed they were transferred to a biosafety cabinet. The thawed cell suspensions from each vial were transferred aseptically into 50 mL centrifuge tubes. 9 mL of pre-warmed Dulbecco's Modified Eagle Medium (DMEM) +10% fetal bovine serum (FBS) medium were added drop-wise to each 50 mL tube containing the thawed cells while gently swirling the tubes. The MCB/WCB cryovials were each rinsed with the homogeneous cell suspension from the centrifuge tube and the rinse was transferred back to the respective 50 mL tubes.

The cell suspensions were then centrifuged at 300 x g ± 5% for 5 minutes at room temperature. The supernatants were discarded and the pellets were suspended in 10 mL DMEM+10% FBS medium. The resuspended pellets were removed from the biosafety cabinet and centrifuged for a second time using the same parameters as before. The supernatant was again discarded and the pellets were resuspended in 10 mL DMEM+10% FBS medium. 0.5 mL from the prepared cell suspensions was removed and used to perform a cell count determining viable cells and viability. The remaining cell suspensions were passaged to one T225 cell culture flasks/suspension and medium was made up to 50 mL using pre-warmed DMEM+10% FBS medium. Following growth in flask culture, the supernatant from the 2xT225 flasks was removed, discarded and the cell monolayer was washed with pre-warmed D-PBS. Pre-warmed TrypLE Select was added to each flask and distributed evenly over the monolayer. Flasks were incubated to detach cells and then observed for cell detachment under the microscope. If necessary, the flasks were taped gently for cell detachment. If detachment was below 90%, flasks were further incubated until detachment of greater than 90% was reached.

Pre-warmed DMEM+10% FBS medium was added to each flask and the cell suspension was removed to sterile centrifuge tubes. After centrifugation, the supernatant was removed and discarded while the cell pellets were resuspended in DMEM+10% FBS medium by pipetting up and down. The cultures were fully suspended if no cell clumps were visible. Resuspended cell-solutions from 2xT225 flasks were each transferred into sterile containers (50 mL centrifugation tube) and mixed to obtain a homogenous solution of cells. 0.5 mL from the prepared cell suspensions were removed and used to perform a cell count determining viable cells and viability of each cell solution. The remaining cell suspensions were passaged to 5 x T225 cell culture flasks/suspension and medium was made up to 50 mL using pre-warmed DMEM+10% FBS medium. Thus, in total 10xT225 flasks were prepared in this exemplary setting, which might naturally depend of the nature of the product to be produced.

Following growth in flask culture stage 2, the supernatant from the two T225 sets flasks was removed, discarded and the cell monolayer was washed with pre-warmed D-PBS. Pre-warmed TrypLE Select was then added to each flask and distributed evenly over the monolayer. Flasks were incubated to detach cells and then observed for cell detachment under the microscope. If necessary, the flasks were taped gently for cell detachment. If detachment was below 90%, flasks were further incubated until detachment of greater than 90% was reached. Parameters are described in Table 1 below.

Pre-warmed DMEM+10% FBS medium was added to each flask and the cell suspensions were removed to sterile centrifuge tubes. The cell suspensions were then centrifuged as described in Table 3 below. After centrifugation, the supernatant was removed and discarded while the cell pellets were resuspended in DMEM+10% FBS medium by pipetting up and down. The cultures were fully suspended if no cell clumps were visible. Resuspended cell-solutions from each set (5xT225 flasks) were transferred/pooled into one sterile container (50 mL centrifugation tube) and mixed to obtain a homogenous solution of cells. 0.5 mL from the prepared cell suspensions were removed and used to perform a cell count determining viable cells and viability of each cell solution. After analysis of viable cells and viability, the best performing set of T225 flasks was selected and passaged to 30 x T225 cell culture flasks and medium was made up to 50 mL using pre-warmed DMEM+10% FBS medium.

Details on the further cultivation, expansion, cell culture infection and processing until harvest and a subsequent purification was performed according to the protocols as detailed in the International patent applications WO 2017/109211 A9 and WO 2017/109222 A1 as well as disclosed in European patent application EP18178037.0 the content of which is incorporated herein by reference. A particular problem for chromatographic purification approaches may be pleomorphic viruses, i.e., the appearance and shape of one and the same virus can vary within a given preparation. MV is such as pleomorphic virus. Even though the Norovirus VLPs forming by self-assembly will be much smaller, purification of infectious chimeric MV particles, said infectious MV particles as vector backbone also encoding the genetic information for foreign Norovirus antigens to be expressed as VLPs according to the present disclosure, should thus follow special purification strategies as detailed herein below in Example 5.

Typically, following growth in flask culture, the supernatant from the 30xT225 flasks will be removed, discarded and the cell monolayer will be washed with pre-warmed D-PBS. Pre-warmed TrypLE Select can be added to each flask and distributed evenly over the monolayer. Flasks can be incubated to detach cells and observed for cell detachment under the microscope. If necessary, the flasks can be taped gently for cell detachment. If detachment is below 90%, flasks were further incubated until detachment of greater than 90% was reached.

Pre-warmed DMEM+10% FBS medium was added to each flask and the cell suspension can be removed to sterile centrifuge tubes. After centrifugation, the supernatant can be removed and discarded while the cell pellet is resuspended in DMEM+10% FBS medium by pipetting up and down. Resuspended cell-solutions from T225 flasks can be transferred into one sterile container and will be mixed to obtain a homogenous solution of cells. 0.5 mL from the prepared cell suspension should be removed and should be used to perform a cell count determining viable cells and viability. Spinner Flasks and Bioreactor microcarrier assisted culture can follow (WO 2017/109222 A1).

Cells should be ≥80% viable and microcarrier ≥80% confluent. The cell count can be used to determine the number of viral particles required to infect the culture at an MOI of 0.01 TCID₅₀/cell. This MOI may again vary depending on the host cell and the MVSS chosen, but can be easily determined after standard pre-testings with the respective host cell and the respective virus. MOIs between 0.0001 and 0.1 are preferable. Notably, the time point for harvest will change depending on the chosen MOI, which can be determined by the skilled person. After calculating the required amount of virus, an appropriate number of viral vials can be removed from -80°C storage and they can be transported to the cleanroom. The virus vials are then thawed at ambient/in hands until all ice has melted. The virus volume required to infect a 10 L suspension at an MOI of 0.01 TCID₅₀/cell is calculated and is diluted in 5000 mL VP-SFM w/o phenol red.

### Example 5: Further separation of VLPs from infectious MV particles

The resulting material of the above expression, harvest and downstream processing is an immunogenic composition comprising both, infectious MV particles as well as self-assembled VLPs of Norovirus even though it was observed that a huge proportion of the material already represented Norovirus VLPs expressing both VP1 and VP2.

For certain applications, it may be just the infectious and replicative virus, which is intended for use as vaccine. For other applications, e.g. in children, it may be preferred to further separate MV particles from VLPs. This can be achieved by performing a further round of column purification using a suitable column material. In case the VLPs are the product of interest, the huge and pleomorphic MV backbone may simply be adsorbed to a column material and the VLPs may be obtained as flow-through. In view of the specific physico-chemical properties of the MV and the Norovirus VLPs representing non-enveloped VLPs a suitable column material for a high yield recovery of VLPs, if desired, has to be determined. In particular an initial column chromatography purification as disclosed in WO 2017/109211 A9 relying on monolithic columns can already separate MVs from Norovirus VLPs during the initial chromatographic step. A process as disclosed EP18178037.0, depending on the specific process parameters, will likely not allow the VLP Norovirus particles to interact with the ligands using a CaptoCore material so that VLPs and MV will be obtained in the flow-through. Depending on the strongly varying size and surface characteristics of MV and Norovirus VLPs, an additional purification step can then be added to separate MVs from Norovirus VLPs to obtain both fractions in a highly purified form, if desired.

The diluted virus solution comprising a mixture of MV and Norovirus VLPs may thus be - in a GMP mode - connected to one of the bypass lines of inlet S1, using the SCD. The bypass on S1 is used to remove air from the line. It has to be ensured that no air will be introduced into inlet S1 later on. Otherwise only part of the virus material will be processed on the respective column. Depending on the resolution and the parameters chosen for column purification and depending on the column material, e.g. a size exclusion column, either one product peak will be obtained comprising the recombinant chimeric MV virus particles derived from a measles virus backbone as scaffold including nucleic acid material from a Norovirus packaged therein and optionally VP1/VP2 virus-like particles and VP1 VLP particles. Alternatively, two or three separate peaks can be obtained, one comprising the recombinant infectious virus particles and the other one comprising VLPs of VP1 and VP2 and one peak comprising VP1 VLPs (which may also form spontaneously). In the case of co-elution, a mixed population can optionally be further purified, polished or subjected to a buffer-exchange (for further details, see WO 2017/109211 A9, WO 2017/109222 A1 and EP18178037.0). At the end of the purification cycle, the exact volume of main peak fraction is noted and the bulk virus pool is snap frozen at -80°C ± 10°C directly or aliquot as required prior freezing. Alternatively, part of the virus pool can immediately be subjected to further analysis, including analysis for purity and infectivity and the like. The main peak fraction can additionally be subjected to a further round of purification, polishing or buffer-exchange to separate recombinant infectious virus particles containing genetic material from virus-like particles. Using the SCD, 20% EtOH was connected to buffer inlet B3. The system was flushed with 20% EtOH. Using the SCD, all buffers from the Äkta Pilot inlets were disconnected and discarded, the T-piece on inlet S1 was disconnected and discarded and a cleaning tubing was connected to inlets S1, S2, A1, A2, B1, B2 and B3. The cleaning inlet tubing was connected to 1 M NaOH. All inlets, outlets and the system were cleaned with 1 M NaOH. The 1 M NaOH connected to the cleaning tubing was replaced by WFI. Flush the system with WFI. Replace the WFI connected to the cleaning tubing by 20% EtOH. Store the Äkta Pilot in 20% EtOH.

### Example 6: Characterization and immunogenicity testing

MV Noro viruses were rescued and their biological properties were determined in cell culture. First, growth properties of MV Noro were analyzed and compared to the parent MV Schwarz. Growth curve analyses were performed on Vero 10-87 cells. Therefore, the cells were infected with a defined MOI (MOI of 1, 0.1 or 0.01) and samples were taken at different time points. MV-Noro exhibited slower growth kinetics than the MV Schwarz parent strain as determined by TCID50 and/or qPCR. Adaptation of different growth parameters increases the virus yield and allows the production of infectious virus particles similar to the MV Schwarz parent strain.

The antigen expression of the Noro VP1 protein was determined by immunostaining. Vero 10-87 cells were thus infected with a defined MOI (1, 0.1 or 0.01). Cells were stained with a commercially available Norovirus GII.4 antibody recognizing Norovirus virus-like particle (VLP) from GII.4 VLP 1987-2009 and 2012. Depending on the MOI used, cells exhibited a strong staining for Norovirus VP1 protein indicating that virus-like particles can be successfully expressed in functional form from the measles virus vector backbone. Expression of Norovirus VLPs was visualized and verified as shown in **Figure 3****.**

### Example 7: Animal model

To evaluate the immunogenicity of the MV Noro vaccine candidate in an animal model, groups of cotton rats were vaccinated intramuscularly with various doses of MV Noro (VP1+VP2, SEQ ID NO:10). All cotton rats were given a homologous booster immunization four weeks (28 days) after the first immunization. Sera were collected at various time points and analyzed for the presence of anti-Norovirus and anti-measles virus specific antibodies. Measles antibodies were detected by using a commercially available EUROIMMUN kit with slight modifications. A secondary antibody labelled with peroxidase specific for cotton rats was used instead of the human one. All cotton rats seroconverted to measles after the first immunization as shown in **Figure 4** indicating that cotton rats are a valid animal model to test recombinant measles-vectored vaccine candidates.

For the detection of Norovirus specific antibodies, an in-house ELISA was developed as follows. Plates were coated with recombinant GII.4 VLP overnight. On the next day, sera were pre-diluted and added to the coated plates. After a certain incubation period, the plates were washed and a secondary incubation step with the same secondary cotton rat antibody as used for the measles ELISA was initiated. Finally, substrate was added and after a certain time the reaction was stopped and the extinction values were measured and blotted on a graph (**Figure 5**). As evident from **Figure 5** showing four different measurement points (day zero before prime immunization, 28 days post infection before boost immunization, day 42 and day 56), a slight increase in Norovirus specific antibodies was already observed after the prime vaccination. The boost vaccination then resulted in a pronounced increase in antibody responses in sera from all tested animals.

### Example 8: Carbohydrate-Binding Blocking Assay

The blocking assay is used for identification of Norovirus specific antibodies that functionally block binding of Norovirus capsid proteins to carbohydrate ligands, HBGA. Sera that were tested positive for the presence of Norovirus-specific antibodies in the ELISA were further subjected to this analysis. These antibodies have been confirmed by the in-house ELISA to be able to recognize and bind GII.4 VLPs.

An antibody blocking assay was performed using synthetic carbohydrate HBGAs. Microtiter plates were coated overnight at 4°C with synthetic HBGAs. On the next day, GII.4 VLP and serially diluted sera were pre-incubated for a certain time and added to the coated plates. As a next step, VLPs bound to the HBGA ligand were detected by using first a polyclonal GII.4 VLP serum followed by a secondary HRP labelled antibody. The extinction values 450 nm were measured by an ELISA reader. An OD reading from the wells lacking cotton rat serum of the immunogenicity study was considered as a maximum signal for the binding of VLPs to the synthetic HBGAs further meaning that no antibody blocking could be observed. Blocking index was calculated as the percentage of the maximum blinding blocked by a certain serum dilution. As a result, the vaccine candidate was able to induce the production of functional Norovirus specific antibodies that block binding of VLPs to HBGA.

### Example 9: Sucrose gradient and Western Blotting

To further optimize Norovirus VLP purification, VLPs as obtainable from SEQ ID NO:10, i.e. VLPs comprising both VP1 and VP2 sequences, were produced as follows. Vero cells were infected with MV-Noro virus and 6 days post infection cell culture supernatants were harvested and clarified by 3,000×*g* for 30 min at 4°C. Then, recombinant measles virus and Norovirus VLPs in the supernatant were concentrated by ultracentrifugation and pellets were resuspended in Lysis Buffer, thereby destroying the cell membranes of the virus particles. The lysis buffer containing VLPs was then subjected to a sucrose cushion and put to high speed centrifugation. The difference in densities between sucrose and lysis buffer allowed the separation of different cell components. The sucrose layer contained the VLPs. A fraction was further analyzed by SDS Page and Western Blot.

## Claims

1. An immunogenic composition comprising at least one recombinant Norovirus antigen, and/or a sequence encoding the same, wherein the at least one recombinant Norovirus antigen is encoded by at least one nucleic acid sequence encoding at least one Norovirus VP1 sequence and at least one Norovirus VP2 sequence, wherein the at least one nucleic acid sequence is operably linked to a vector backbone, wherein the immunogenic composition optionally comprises at least one pharmaceutically acceptable carrier and/or excipient.

2. The immunogenic composition according to claim 1, wherein the Norovirus VP1 sequence and/or the Norovirus VP2 sequence is independently derived from a Norovirus of the GII genogroup, preferably from a Norovirus having a GII.4 genotype, preferably wherein the Norovirus VP1 sequence and the Norovirus VP2 sequence are encoded by the same nucleic acid sequence and are optionally separated by at least one linker sequence.

3. The immunogenic composition according to claims 1 or 2, wherein the vector backbone is selected from the group consisting of a measles virus backbone, a lentivirus backbone, a Vesicular stomatitis virus (VSV) backbone, or a vaccinia Ankara backbone, preferably wherein the vector backbone is from a recombinant live-attenuated measles virus strain, the measles virus strain preferably being selected from the group consisting of a Schwarz strain, a Zagreb strain, an AIK-C strain and a Moraten strain.

4. The immunogenic composition according to any of the preceding claims, wherein the VP1 and/or the VP2 sequence are independently selected from a Norovirus having a GII.4 genotype, preferably from a sequence from a Norovirus Hu/GII.4/Sydney/NSW0514/2012/AU, a Norovirus Hu/GII.4/Orange/NSW001P/2008/AU, a Norovirus Hu/GII.4/NIHIC17.5/2012/USA, a Norovirus Hu/GII.4/GII4-NG1242/2011/JP, or a Norovirus Hu/GII.4/Hiroshima4/2012/JP, wherein the VP1 and the VP2 sequence are derived from the same Norovirus, or wherein the VP1 and the VP2 sequence are derived from a different Norovirus, optionally wherein the VP1 and the VP2 sequence are modified in comparison to the reference sequence.

5. The immunogenic composition according to any of the preceding claims, wherein the at least one VP1 and/or the at least one VP2 sequence represent(s) a conserved consensus sequence.

6. The immunogenic composition according to any of the preceding claims, wherein the vector backbone comprises a nucleotide sequence of the full-length, infectious antigenomic (+) RNA strand of a measles virus, preferably wherein the immunogenic composition comprises a sequence of SEQ ID NO:10, or a sequence having at least 80%, 81%, 82%, 83%, 84%, 85%, 86%, 87%, 88%, 89%, 90%, 91%, 92%, 93%, 94%, 95%, 96%, 97%, 98% or 99% sequence identity thereto.

7. The immunogenic composition according to any of the preceding claims, wherein the at least one recombinant Norovirus VP1 antigen is selected from an amino acid sequence of SEQ ID NOs:1, 11, 13 or 15 or a nucleic acid sequence encoding the same, or an amino acid sequence having at least 90%, 91%, 92%, 93%, 94%, 95%, 96%, 97%, 98% or 99% sequence identity thereto, and wherein the at least one recombinant Norovirus VP2 antigen is selected from an amino acid sequence of SEQ ID NOs:2, 12, 14 or 16 or a nucleic acid sequence encoding the same, or an amino acid sequence having at least 90%, 91%, 92%, 93%, 94%, 95%, 96%, 97%, 98% or 99% sequence identity thereto.

8. The immunogenic composition according to any of the preceding claims, wherein the immunogenic composition is a bivalent or a multivalent immunogenic composition, wherein the at least one nucleic acid sequence encoding the at least one Norovirus VP1 sequence and/or the at least one nucleic acid sequence encoding at least one Norovirus VP2 sequence are selected from at least two different Norovirus genogroups, preferably a GII and a GI genogroups, and/or wherein the at least one nucleic acid sequence encoding the at least one Norovirus VP1 sequence and/or the at least one nucleic acid sequence encoding at least one Norovirus VP2 sequence are selected from different Norovirus GII genotypes, preferably a GII.4 and a GII.17 or GII.2 genotype.

9. A nucleic acid molecule comprising at least a first and at least a second nucleic acid sequence, wherein the first nucleic acid sequence encodes at least one VP1 protein of a Norovirus or a functional fragment thereof, and wherein the second nucleic acid sequence encodes at least one VP2 protein of a Norovirus or a functional fragment thereof, optionally wherein the at least one first and the at least one second nucleic acid sequence is/are separated by at least one linker.

10. The nucleic acid molecule according to claim 9, wherein the at least one first and/or the at least one second nucleic acid sequence is independently selected from a Norovirus having a GII.4 genotype, preferably from a sequence from a Norovirus Hu/GII.4/Sydney/NSW0514/2012/AU, a Norovirus Hu/GII.4/Orange/NSW001P/2008/AU, a Norovirus Hu/GII.4/NIHIC17.5/2012/USA, a Norovirus Hu/GII.4/GII4-NG1242/2011/JP, or a Norovirus Hu/GII.4/Hiroshima4/2012/JP, wherein the at least one nucleic acid sequence encoding the at least one Norovirus VP1 and the at least one nucleic acid sequence encoding the at least one Norovirus VP2 are derived from the same Norovirus, and/or wherein the at least one nucleic acid sequence encoding the at least one Norovirus VP1 and the at least one nucleic acid sequence encoding the at least one Norovirus VP2 are derived from a different Norovirus, preferably selected from a Norovirus of a different genogroups, including genogroups GI, GII and GIV, and/or wherein the at least one nucleic acid sequence encoding the at least one Norovirus VP1 and/or the at least one nucleic acid sequence encoding at least one Norovirus VP2 are selected from different Norovirus GII genotypes, preferably a GII.4 and a GII.17 or GII.2 genotype.

11. The nucleic acid molecule according to claim 9, wherein the nucleic acid molecule comprises a sequence selected from any one of SEQ ID NOs:3 to 8, or a sequence having at least 80%, 81%, 82%, 83%, 84%, 85%, 86%, 87%, 88%, 89%, 90%, 91%, 92%, 93%, 94%, 95%, 96%, 97%, 98% or 99% sequence identity thereto, or wherein the first nucleic acid sequence and/or the second nucleic acid sequence represent(s) a conserved consensus sequence.

12. A recombinant virus-like particle (VLP) comprising at least one recombinant Norovirus VP1 antigen and at least one recombinant Norovirus VP2 antigen as defined in any one of claims 1, 2 or 4 to 8.

13. A host cell comprising at least one nucleic acid sequence encoding at least one Norovirus VP1 sequence and at least one Norovirus VP2 sequence according to any one of claims 1 to 8, or comprising at least one nucleic acid molecule according to any one of claims 9 to 11, or comprising at least one nucleic acid sequence encoding a recombinant virus-like particle (VLP) according to claim 12, preferably wherein the at least one host cell is selected from the group consisting of Vero cells, chicken embryo fibroblast cells, HEK293 cells, HeLa cells, fetal rhesus lung cells or MRC5 cells.

14. A method for producing an immunogenic composition according to claims 1 to 8, or for producing a recombinant virus-like particle (VLP) according to claim 12, the method comprising the following steps:
(i) inserting at least one nucleic acid sequence as defined in any one of claims 1 to 8, or inserting at least one nucleic acid molecule as defined in any one of claims 9 to 11 into a vector backbone as defined in any one of claims 1, 2 or 6 to operably link the nucleic acid sequence or molecule and the vector backbone to obtain a recombinant chimeric virus sequence;
(ii) infecting at least one host cell with at least one recombinant chimeric virus sequence obtained in step (i) to obtain a virus sample;
(iii) optionally: clarifying the virus sample of step (ii);
(iv) optionally: purifying the clarified virus sample of step (iii);
(v) optionally: formulating the at least one recombinant chimeric virus with at least one pharmaceutically and/or veterinary acceptable carrier and/or excipient;
(vi) obtaining an immunogenic composition comprising infectious virus particles and virus-like particles (VLPs);
(vii) optionally: further purifying the immunogenic composition and thus obtaining, separated from each other, recombinant virus-like particles (VLPs) and/or infectious virus particles.

15. An immunogenic composition according to claims 1 to 8, or a recombinant virus-like particle (VLP) according to claim 12 for use in a method of preventing or treating a Norovirus virus disease in a subject.
